# EUROPEAN PATENT SPECIFICATION

(11) **EP 2 590 936 B1**
(45) Date of publication and mention of the grant of the patent: **12.12.2018**
(21) Application number: 11801178.2
(22) Date of filing: 01.07.2011
(51) Int. Cl.: C07C 271/14, C07C 31/42, A61K 31/27, A61P 21/02, C07C 33/46, C07C 271/12, C07C 271/24

(54) **PHENYLCARBAMATE COMPOUND AND MUSCLE RELAXANT CONTAINING THE SAME**
PHENYLCARBAMATVERBINDUNG UND MUSKELENTSPANNUNGSMITTEL DAMIT
COMPOSÉ DE PHÉNYLCARBAMATE ET RELAXANT MUSCULAIRE LE CONTENANT

(30) Priority: 02.07.2010 US 360952 P
(43) Date of publication of application: 15.05.2013
(73) Proprietor: Bio-Pharm Solutions Co., Ltd., Suwon-si, Gyeonggi-do 443-270 (KR)
(72) Inventor: CHOI, Yong Moon, Irvine, CA 926204 (US)
(74) Representative: Rees, Kerry
(86) International application number: PCT/KR2011/004862
(87) International publication number: WO 2012/002773

(56) References cited:
- WO-A1-2008/013213
- WO-A1-2008/048802
- WO-A1-2008/124848
- JP-A- S61 271 992
- US-A- 3 313 692
- US-A- 5 698 588
- US-A1- 2001 034 365
- JIAO, P. ET AL.: 'A Sequential 0-Nitrosoaldol and Grignard Addition Process: An Enantio- and Diastereoselective Entry to Chiral 1,2-Diols' ANGEWANDTE CHEMIE, INTERNATIONAL EDITION vol. 48, no. 18, 2009, pages 3333 - 3336, XP055073637
- GIRIJAVALLABHAN, V. M. ET AL.: 'Synthesis of the antifungal agent SCH 42427 (SM 9164)' BIOORGANIC & MEDICINAL CHEMISTRY LETTERS vol. 1, no. 7, 1991, pages 349 - 52, XP055108253

## Description

### BACKGROUND OF THE INVENTION

### (a) Field of the Invention

The present invention relates to novel phenyl carbamate compounds and pharmaceutical compositions containing the compound. More specifically, the present invention relates to phenyl carbamate compounds and a pharmaceutically acceptable salt thereof, which have a considerably high muscle relaxation effect and low toxicity, and compositions for muscle relaxation containing the compounds and/or pharmaceutically acceptable salt thereof as an active ingredient.

### (b) Description of the Related Art

Myotony or spasm is frequently observed as a sequel of cerebrovascular disorders such as stroke or sequel of head injuries, and is not easy to treat.

Myotony or spasm is one of skeletal muscle dysfunction diseases due to increase of muscle tone, and caused by central nervous system damage due to various causes such as external injury, cerebrovascular accidents, and the like. The muscle tension is caused by various causes, for example, cervicoomobrachial syndrome which is caused by abnormal posture, fatigue, age-related spine deformity, and the like, and causes spasticity or pain in skeletal muscles of the neck, shoulders, arms, waist, and back; spastic paralysis causing disability of voluntary movement due to muscle hypertonia of hands and feet by disorder of central nervous system such as cerebrovascular disorder; and a combination thereof, thereby resulting in serious hindrances to normal life.

In particular, spastic paralysis is a serious disorder with accompanying symptoms including muscle tension and/or muscular stiffness of hands and feet, difficulty in walking, and the like, thererby causing serious hindrances to normal life. Centrally acting muscle relaxants relieve muscle tension by blocking receptors associated with stimulating muscular function or stimulating receptors associated with inhibiting muscular function, or reducing excessively activated reflex function.

Such centrally acting muscle relaxants may include Methocarbaamol, Chlormezanon, Carisoprodol, Eperisone, Phenprobamide, and the like. However, these drugs act on interneuron of the spinal cord, thereby inhibiting monosynaptic and polysynaptic reflexes, and thus, may cause side effects, such as central nervous inhibition, muscle weakness, and the like.

US Patent No. 3,313,692 discloses a racemic carbamate compound useful as a therapeutic agent for the central nervous system with decreased side effects compared to cholinergic agent. US Patent Nos. 2,884,444, 2,937,119, and 3,265,727 suggest dicarbamate compounds useful as therapeutic agents for central nervous system disorders, and US Patent No. 2,937,119 discloses a N-isopropyl-2-methyl-2-propyl-1,3-propanediol dicarbamate, which was released with the trademark of Soma as a muscle relaxant.

Muscle relaxants are used for improving various symptoms, such as cervicoomobrachial syndrome (shoulder-arm-neck syndrome), lumbago(lower back pain), herniation of intervertebral disk, cerebrovascular disorders, vascular disorders of the spinal cord, spastic spinal paralysis, cervical spondylosis, cerebral palsy, sequelae of injuries (spinal cord injuries, head injuries), spinocerebellar degeneration, multiple sclerosis, amyotrophic lateral sclerosis, and the like, which are associated with muscle spasm involved in musculoskeletal diseases, and also used as an adjuvant to anesthestic agent.

Considering the various and valuable uses of muscle relaxants as aforementioned, development of more effective muscle relaxant is needed.

### SUMMARY OF THE INVENTION

To satisfy such requirement, an embodiment of the present invention provides specific phenyl carbamate compounds which are claimed in claim 1. Further, the disclosure relates to compounds represented by Chemical Formula 1; a racemate, an enantiomer, a diastereomer, a mixture of enantiomers, or a mixture of diastereomers thereof; or a pharmaceutically acceptable salt thereof: 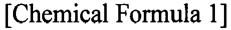 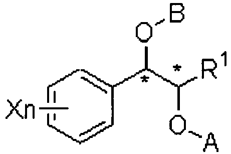 wherein,
X is a halogen, for example, chlorine, fluorine, iodine, or bromine,
N is an integer from 1 to 5, for example, 1 or 2,
R1 is a C1-C4 linear or branched alkyl group, for example, methyl group, ethyl group, isopropyl group, or butyl group,
A is hydrogen or 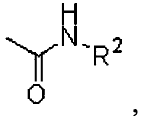
B is hydrogen or 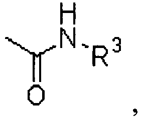
A and B are not carbamoyl derivatives at same time, and
R2 and R3 may be the same as or different from each other, and independently selected from the group consisting of hydrogen, a linear or branched alkyl group of C1-C4, for example C1-C3, a cycloalkyl group of C3-C8, for example C3-C7, and benzyl group, and more specifically, R2 and R3 may be the same as or different from each other, and independently selected from the group consisting of hydrogen, methyl group, propyl group, isopropyl group, cyclopropyl group, cyclohexyl group, bicycloheptane group, and benzyl group.

Another embodiment provides a pharmaceutical composition containing provides a phenyl carbamate compound claimed in claim 1; a racemate, an enantiomer, a diastereomer, a mixture of enantiomers, or a mixture of diastereomers thereof; or a pharmaceutically acceptable salt thereof, as an active ingredient.

Another embodiment provides a muscle relaxant containing provides a phenyl carbamate compound claimed in claim 1; a racemate, an enantiomer, a diastereomer, a mixture of enantiomers, or a mixture of diastereomers thereof; or a pharmaceutically acceptable salt thereof, as an active ingredient.

Another embodiment provides a pharmaceutical composition for use in treating and/or preventing a disease associated with muscle spasm involved in musculoskeletal diseases, containing a phenyl carbamate compound represented by Chemical Formula 1; a racemate, an enantiomer, a diastereomer, a mixture of enantiomers, or a mixture of diastereomers thereof; or a pharmaceutically acceptable salt thereof, as an active ingredient.

Another embodiment provides a phenyl carbamate compound claimed in claim 1; a racemate, an enantiomer, a diastereomer, a mixture of enantiomers, or a mixture of diastereomers thereof; or a pharmaceutically acceptable salt thereof, for use in the muscle relaxation or the manufacture of a muscle relaxan.

Another embodiment provides a phenyl carbamate compound claimed in claim 1; a racemate, an enantiomer, a diastereomer, a mixture of enantiomers, or a mixture of diastereomers thereof; or a pharmaceutically acceptable salt thereof, for use in the treatment and/or prevention of a disease associated with muscle spasm involved in musculoskeletal diseases, or in the manufacture of a composition for the treatment and/or prevention of a disease associated with muscle spasms related to musculoskeletal diseases.

### DETAILED DESCRIPTION OF THE EMBODIMENTS

Upon attempting to discover a muscle relaxant with more excellent efficacy and decreased side effects, the present inventors found that substituted phenyl carbamate compounds claimed in claim 1 exhibit a considerably excellent activity of muscle relaxation with very low toxicity, to complete the present invention.

Further, the disclosure relates to a phenyl carbamate compound represented by following Chemical Formula 1: 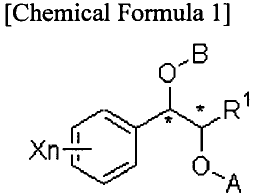 wherein,
X is a halogen, for example, chlorine, fluorine, iodine, or bromine,
N is an integer from 1 to 5, for example, 1 or 2,
R1 is a C1-C4 linear or branched alkyl group, for example, methyl group, ethyl group, isopropyl group, or butyl group,
A is hydrogen or a carbamoyl derivative represented by 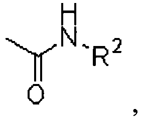
B is hydrogen or a carbamoyl derivative represented by 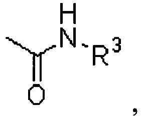
A and B are not carbamoyl derivatives at same time, and
R2 and R3 may be the same as or different from each other, and independently selected from the group consisting of hydrogen, a linear or branched alkyl group of Cl-C4, for example C1-C3, a cycloalkyl group of C3-C8, for example C3-C7, and benzyl group, and more specifically, R2 and R3 may be the same as or different from each other, and independently selected from the group consisting of hydrogen, methyl group, propyl group, isopropyl group, cyclopropyl group, cyclohexyl group, bicycloheptane group, and benzyl group.

In a concrete embodiment, when X is chlorine and n is 1, A and B may not be hydrogen at the same time, or R1 may not be methyl group, or R2 may not be hydrogen.

In another concrete embodiment, when X is chlorine and n is 1, A and B may not be hydrogen at the same time, or R1 may not be methyl group or not be ethyl group, or R2 may not be hydrogen, and when X is chlorine and n is 2, R1 may not be ethyl group, or R2 may not be hydrogen.

Since the compound has two chiral carbons at the 1^{st} and 2^{nd} positions from the X substituted phenyl group, they may be in the form of a racemate, an enantiomer, a diastereomer, a mixture of enantiomers, or a mixture of diastereomers.

Alternatively, the compound may be in the form of a pharmaceutically acceptable salt. The pharmaceutically acceptable salt may include an additional salt of acid or base, and its stereochemical isomer. For example, the compound may be in the form of an additional salt of an organic or inorganic acid. The salt may not be specially limited, and include any salts that maintain the activities of their parent compounds, with no undesirable effects, in the subject, when they are administered to the subject. Such salts may include inorganic and organic salts, such as salts of acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzene sulfonic acid, benzoic acid, stearic acid, lactic acid, bicarbonic acid, bisulfuric acid, bitartaric acid, oxalic acid, butyric acid, calcium edetate, carbonic acid, chlorobezoic acid, citric acid, edetic acid, toluenesulfonic acid, fumaric acid, gluceptic acid, esilic acid, pamoic acid, gluconic acid, methyl nitric acid, malonic acid, hydrochloric acid, hydroiodic, hydroxynaphtholic acid, isethionic acid, lactobionic acid, mandelic acid, mucic acid, naphthylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methane sulfonic acid, and the like. The additional salts of base may include salts of akali metal or alkaline earth metal, such as salts of ammonium, lithium, sodium, potassium, magnesium, calcium, and the like; salts having an organic base, such as benzathine, N-methyl-D- glucamine, hydrabamine, and the like; and salts having an amino acid such as arginine, lysine, and the like. In addition, these salts may be converted to a released form by treating with a proper base or acid.

As demonstrated in the following experimental examples, the compound of Chemical Formula 1; a racemate, an enantiomer, a diastereomer, a mixture of enantiomers, or a mixture of diastereomers thereof; or pharmaceutically acceptable salt thereof exhibits an excellent effect on muscle relaxation.

Therefore, another embodiment provides a pharmaceutical composition containing a compound of claim 1; a racemate, an enantiomer, a diastereomer, a mixture of enantiomers, or a mixture of diastereomers thereof; or a pharmaceutically acceptable salt thereof, as an active ingredient.

The pharmaceutical composition may be a muscle relaxant.

In addition, since a muscle relaxant can be used for improving and/or treating symptoms of diseases associated with muscle spasm, the pharmaceutical composition capable of acting as muscle relaxant may also be used as a pharmaceutical composition for preventing and/or treating a muscle spasm associated disease, for example, cervicoomobrachial syndrome (shoulder-arm-neck syndrome), lumbago (lower back pain), herniation of intervertebral disk, cerebrovascular disorders, vascular disorders of the spinal cord, spastic spinal paralysis, cervical spondylosis, cerebral palsy, sequelae of injuries (spinal cord injuries, head injuries), spinocerebellar degeneration, multiple sclerosis, amyotrophic lateral sclerosis, and the like.

Therefore, another embodiment provides a pharmaceutical composition for muscle relaxation, or a pharmaceutical composition for treating and/or preventing a disease associated with muscle spasm, containing a phenyl carbamate compound of claim 1; a racemate, an enantiomer, a diastereomer, a mixture of enantiomers, or a mixture of diastereomers thereof; or a pharmaceutically acceptable salt thereof, as an active ingredient.

The pharmaceutical composition may be formulated in various forms for oral or parenteral administration. For example, the pharmaceutical composition may be formulated in the oral administration form, such as a tablet, pill, soft or hard capsule, liquid, suspension, emulsion, syrup, granules, elixirs, and the like. In addition to the active ingredient, the oral administration form may further include pharmaceutically acceptable and conventional components, for example, a diluent such as lactose, dextrose, sucrose, mannitol, sorbitol, cellulose, glycine, and the like; a lubricant such as silica, talc, stearic acid, magnesium or calcium salt thereof, polyethyleneglycol, and the like.

In the case that the oral administration form is a tablet, it may further include a binder such as magnesium aluminium silicate, starch paste, gelatin, tragacanth, methylcellulose, sodium carboxymethylcellulose, polyvinylpirrolidine, and the like; and optionally include one or more additives selected from the group consisting of a disintegrant such as starch, agar, arginic acid or sodium salt thereof, an absorbent, a colorant, a flavoring, a sweetener, and the like.

Alternatively, the pharmaceutical composition may also be formulated in a parenteral administration form, which can be administered by subcutaneous injection, intravenous injection, intramuscular injection, injection into thoracic cavity, and the like. In order to formulate the parenteral administration form, the pharmaceutical composition may be prepared as a solution or suspension wherein the active ingredient is dissolved in water together with a stabilizer and/or a buffering agent, and such solution or suspension formulation may be prepared as a dosage form in ample or vial.

The pharmaceutical composition may be sterilized, and/or include further additives such as a preservative, a stabilizer, a hydrating agent, an emulsification accelerator, a salt and/or buffering agent for osmoregulation, and the like, and/or further therapeutically effective ingredients. The pharmaceutical composition may be formulated by any conventional method for mixing, granulating, coating, and the like.

The pharmaceutical composition may be administered to a mammal including human, in the therapeutically effective amount of 0.01 to 750 mg/kg(body weight), preferably 0.1 to 500 mg/kg(body weight) per one day, based on the active ingredient. The therapeutically effective amount may be administered through oral or parenteral pathway, one or two or more times per one day.

The therapeutically effective amount and the administration pathway of the present pharmaceutical composition may be properly adjusted by a person skilled in the relevant field considering the conditions of the subject (patient), desired effects, and the like.

Another embodiment provides a phenyl carbamate compound of claim 1; a racemate, an enantiomer, a diastereomer, a mixture of enantiomers, or a mixture of diastereomers thereof; or a pharmaceutically acceptable salt thereof, for the use of muscle relaxation, or treatment and/or prevention of a disease associated with muscle spasm; and a use of a phenyl carbamate compound of claim 1; and for the use of preparing a muscle relaxant or a pharmaceutical composition for treating and/or preventing a disease associated with muscle spasms.

The subject to be treated with the compound may be any mammal, preferably human.

The carbamate compound of the present invention may prepared by the following reaction formula. Reaction Formula I: Syn-addition Diol

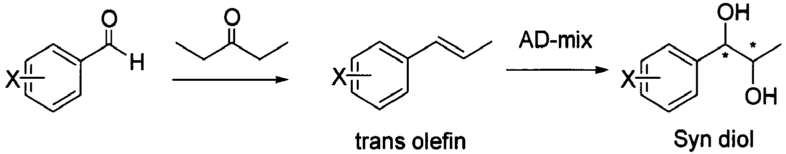

A Syn diol compound used in the synthesis of the carbamate compound may be synthesized by dihydroxylation of a trans-olefin compound. A diol compound having optical activity may be synthesized using a sharpless asymmetric dihydroxylation catalyst.

### Reaction Formula II: Anti-addition Diol

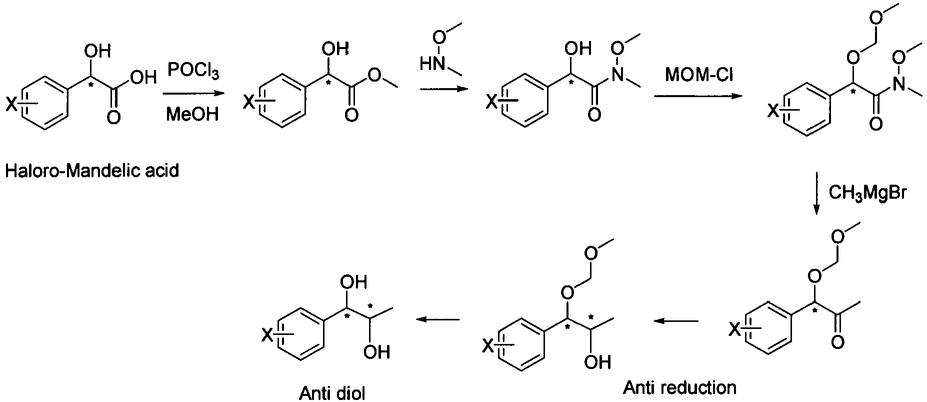

An optically active substance of anti-diol may be synthesized using an anti-reduction reagent after synthesizing a hydroxy-ketone compound using Haloro-Mandelic acid.

### Reaction Formula III: Carbamation reaction

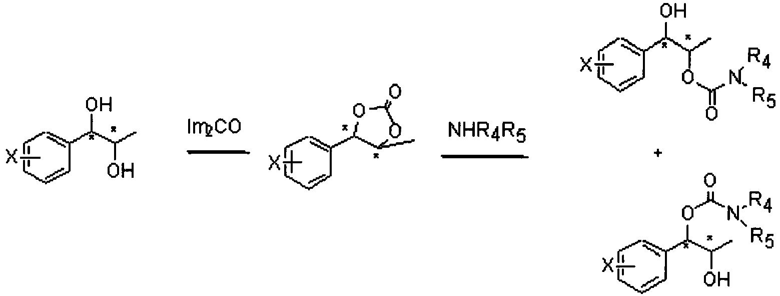

In the Reaction Formula III, R4 and R5 may be the same as or different from each other, and independently selected from the group consisting of hydrogen, a linear or branched alkyl group of C1-C4, for example C1-C3, a cycloalkyl group of C3-C8, for example C3-C7, and benzyl group, and more specifically, R4 and R5 may be the same as or different from each other, and independently selected from the group consisting of hydrogen, methyl group, propyl group, isopropyl group, cyclopropyl group, cyclohexyl group, bicycloheptane group, and benzyl group.

Two substances in the form of regioisomers of a single carbamate of diol having halogen substituent at phenyl ring may be separated by flash column chromatography to obtain two kinds of single carbamate compounds.

### EXAMPLE

The present invention is further explained in more detail with reference to the following examples. These examples, however, should not be interpreted as limiting the scope of the present invention in any manner.

### Preparation Example 1: Synthesis of 1-(2-chlorophenyl)-trans-1-propene

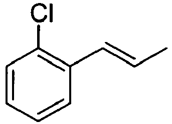

48ml of 2-chlorobenzenaldehyde (0.42mol) and 49.7ml of 3-pentanone (0.47mol) were dissolved in 600mL of hexane in flask, and then stirred with raising the temperature. 53.6ml of Boron trifluoride etherate (BF₃OEt₂, 0.42mol) was added to the resultant under reflux conditions. When the reaction was completed, water was added thereto. After layer separation, the obtained organic layer was washed twice with 1M sodium hydroxide solution (1M NaOH), and then the separated organic layer was washed with water. The separated organic layer was dehydrated with anhydrous magnesium sulfate (MgSO₄) and concentrated. The concentrated residue was purified by a silica gel column chromatography to produce the title compound (38g, yield 58%).
¹H NMR(400MHz, CDCl₃) δ1.94(d, *J*=4.8Hz, 3H), 6.24(m, 1H), 6.78(d, *J*=14Hz, 1H), 7.11∼7.5 1(m, 4H)

### Preparation Example 2: Synthesis of 1-(2-chlorophenyl)-trans-1-butene

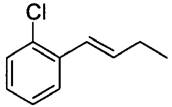

The substantially same method as described in Preparation Example 1 was conducted, except that 3-heptanone was used instead of 3-pentanone, to obtain the title compound (2.9g, yield 83%).
¹H NMR(400MHz, CDCl₃) δ1.14(d, *J*=7.6Hz, 3H), 2.29∼2.33(m, 2H), 6.28(dt, *J*=16Hz, 6.4Hz, 1H), 6.78(d, *J*=15.6Hz, 1H), 7.13∼7.54(m, 4H)

### Preparation Example 3: Synthesis of 1-(2-chlorophenyl)-3-methyl-trans-1-butene

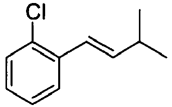

The substantially same method as described in Preparation Example 1 was conducted, except that 2,6-dimethyl-heptan-4-one was used instead of 3-pentanone, to obtain the title compound (8.0g, yield 50∼90%).
¹H NMR(400MHz, CDCl₃) δ1.14(d, *J*=6.8Hz, 6H), 2.25∼2.57(m, 1H), 6.20(dd, *J*=16Hz, 7.2Hz, 1H), 7.64(d, *J*=16Hz, 1H), 7.12∼7.54(m, 4H)

### Preparation Example 4: Synthesis of 1-(2-chlorophenyl)-trans-1-hexene

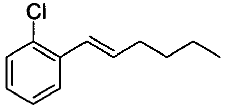

The substantially same method as described in Preparation Example 1 was conducted, except that 6-undecanone was used instead of 3-pentanone, to obtain the title compound (10g, yield 85%).
¹H NMR(400MHz, CDCl₃) δ0.96(t, *J*=7.2Hz, 3H), 1.33∼1.56(m, 4H), 2.26∼2.32(m, 4H), 6.24(dt, *J*=15.6Hz, 7Hz, 1H), 6.78(d, *J*=16Hz, 1H), 7.13∼*7.*54(m, 4H)

### Preparation Example 5: Synthesis of 1-(2,4-dichlorophenyl)-trans-1-propene

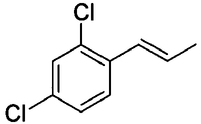

The substantially same method as described in Preparation Example 1 was conducted, except that 2,4-dichlorobenzenaldehyde was used instead of 2-chlorobenzenaldehyde, to obtain the title compound (2.4g, yield 57%).
¹H NMR(400MHz, CDCl₃) δ1.95(dd, *J*=6.8Hz, 1.6Hz, 3H), 6.24(m, 1H), 6.72(d, *J*=15.6Hz, 1H), 7.18∼7.44(m, 3H)

### Preparation Example 6: Synthesis of 1-(2,4-dichlorophenyl)-trans-1-butene

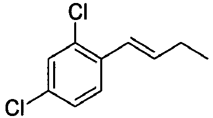

The substantially same method as described in Preparation Example 5 was conducted, except that 3-heptanone was used instead of 3-pentanone, to obtain the title compound (2.1g, yield 90%).
¹H NMR(400MHz, CDCl₃) δ1.14(d, *J*=7.6Hz, 3H), 2.20∼2.33(m, 2H), 6.26(dt, *J*=16Hz, 6.8Hz, 1H), 6.70(d, *J*=15.6Hz, 1H), 7.18∼7.46(m, 3H)

### Preparation Example 7: Synthesis of 1-(2,6-dichlorophenyl)-3-methyl-trans-1-butene

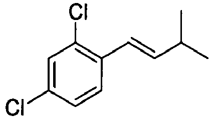

The substantially same method as described in Preparation Example 5 was conducted, except that 2,6-dimethyl-heptan-4-one was used instead of 3-pentanone, to obtain the title compound (0.23g, yield 10∼40%).
¹H NMR(400MHz, CDCl₃) δ1.15(d, *J*=6.8Hz, 6H), 2.53∼2.58(m, 1H), 6.19(dd, *J*=16.4Hz, 6.8Hz, 1H), 6.31(d, *J*=16.4Hz, 1H), 7.18∼7.46(m, 3H)

### Preparation Example 8: Synthesis of 1-(2,4-dichlorophenyl)-trans-1-hexene

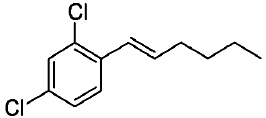

The substantially same method as described in Preparation Example 5 was conducted, except that 6-undecanone was used instead of 3-pentanone, to obtain the title compound (3.2g, yield 40∼80%).
¹H NMR(400MHz, CDCl₃) δ0.96(t, *J*=7.2Hz, 3H), 1.38∼1.52(m, 4H), 2.25∼2.31(m, 2H), 6.22(dt, *J*=15.6Hz, 6.8Hz, 1H), 6.70(d, *J*=15.6Hz, 1H), 7.18∼7.46(m, 3H)

### Preparation Example 9: Synthesis of 1-(2,6-dichlorophenyl)-trans-1-propene

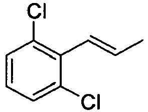

The substantially same method as described in Preparation Example 1 was conducted, except that 2,6-dichlorobenzenaldehyde was used instead of 2-chlorobenzenaldehyde, to obtain the title compound (0.4g, yield 10∼40%).
¹H NMR(400MHz, CDCl₃) δ1.98(d, *J*=8Hz, 3H), 6.23∼6.31(m, 1H), 6.40(d, *J*=16Hz, 1H), 7.05∼7.32(m, 3H)

### Preparation Example 10: Synthesis of 1-(2,6-dichlorophenyl)-trans-1-butene

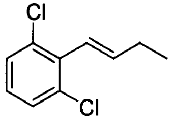

The substantially same method as described in Preparation Example 9 was conducted, except that 3-heptanone was used instead of 3-pentanone, to obtain the title compound (1.2g, yield 10∼40%).
¹H NMR(400MHz, CDCl₃) δ1.17(t, *J*=7.6Hz, 3H), 2.30∼2.37(m, 2H), 6.29(dt, *J*=16.4Hz, 6Hz, 1H), 6.37(d, *J*=16.4Hz, 1H), 7.05∼7.32(m, 3H)

### Preparation Example 11: Synthesis of 1-(2,6-dichlorophenyl)-3-methyl-trans-1-butene

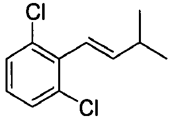

The substantially same method as described in Preparation Example 9 was conducted, except that 2,6-dimethyl-heptan-4-one was used instead of 3-pentanone, to obtain the title compound (0.23g, yield 10∼40%).
¹H NMR(400MHz, CDCl₃) δ1.15(d, *J*=6.8Hz, 6H), 2.53∼2.58(m, 1H), 6.19(dd, *J*=16.4Hz, 6.8Hz, 1H), 6.31(d,*J*=16.4Hz, 1H), 7.05∼7.32(m, 3H)

### Preparation Example 12: Synthesis of 1-(2,6-dichlorophenyl)-trans-1-hexene

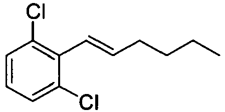

The substantially same method as described in Preparation Example 9 was conducted, except that 6-undecanone was used instead of 3-pentanone, to obtain the title compound (0.2g, yield 10∼40%).
¹H NMR(400MHz, CDCl₃) δ0.99(t, *J*=7.2Hz, 3H),1.14∼1.59(m, 4H), 2.30∼2.36(m, 2H), 6.24(dt, *J*=16Hz, 6.6Hz, 1H), 6.38(d, *J*=16.4Hz, 1H), 7.05∼7.33(m, 3H)

### Preparation Example 13: Synthesis of 1-(2,3-dichlorophenyl)-trans-1-propene

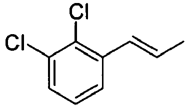

The substantially same method as described in Preparation Example 1 was conducted, except that 2,3-dichlorobenzenaldehyde was used instead of 2-chlorobenzenaldehyde, to obtain the title compound (0.2g, yield 10∼40%).
¹H NMR(400MHz, CDCl₃) δ1.94(d, *J*=4.8Hz, 3H), 6.24(m, 1H), 6.78(d, J=14Hz, 1H), 7.11∼7.51(m, 3H)

### Preparation Example 14: Synthesis of 1-(2-chlorophenyl)-(S,S)-1,2-propanediol

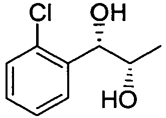

1-(2-chlorophenyl)-trans-1-propene(1.5g, Preparation Example 1) was dissolved in 30mL of the mixture of t-BuOH/H₂O (1:1(V/V)). At 0°C, AD-mix-α (Aldrich, U.S.A.) (13.7g) and methane sulfone amide (CH₃SO₂NH₂, 0.76g, 0.0080mol) were added thereto and stirred for overnight. When the reaction was completed, the obtained product was washed with an aqueous solution of sodium sulfite (Na₂SO₃) and ethylacetate (EA). Then, the organic layer was dehydrated with anhydrous magnesium sulfate (MgSO₄), filtrated, and concented under reduced pressure. The concentrated residue was purified by a silica gel column chromatography to produce the title compound (1.65g, yield 90%).
¹H NMR(400MHz, CDCl₃) δ1.20(d, *J*=6.4Hz, 3H), 2.48(d, *J*=4.0Hz 1H), 2.92(d, *J*=4.4Hz, 1H), 3.93∼3.97(m, 1H), 4.97(t, *J*=4.8Hz, 1H), 7.22∼7.51(m, 4H)
¹³CNMR(100MHz,CDCl₃) δ18.8, 71.5, 74.4, 127.1, 128.1, 128.9, 129.5, 132.6, 138.9

### Preparation Example 15: Synthesis of 1-(2-chlorophenyl)-(R,R)-1,2-propanediol

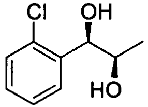

1-(2-chlorophenyl)-trans-1-propene (2.5g, Preparation Example 1) was dissolved in 50mL of the mixture of t-BuOH/H₂O (1:1(V/V)). At 0°C, AD-mix-α (Aldrich, U.S.A.) (23.5g) and methane sulfone amide (CH₃SO₂NH₂, 1.27g, 0.013mol) were added thereto and stirred for overnight. When the reaction was completed, the obtained product was washed with an aqueous solution of sodium sulfite (Na₂SO₃) and ethylacetate (EA). Then, the organic layer was dehydrated with anhydrous magnesium sulfate (MgSO₄), filtrated, and concented under reduced pressure. The concentrated residue was purified by a silica gel column chromatography to produce the title compound (2.96g, yield 90%).
¹H NMR(400MHz, CDCl₃) δ1.20(d, *J*=6.4Hz, 3H), 2.48(d, *J*=4.0Hz, 1H), 2.92(d, *J*=4.4Hz, 1H), 3.93∼3.97(m, 1H), 4.97(t, *J*=4.8Hz, 1H), 7.22∼7.5 1(m, 4H)

### Preparation Example 16: Synthesis of the mixture of 1-(2-chlorophenyl)-(S,S)-1,2-propanediol and 1-(2-chlorophenyl)-(R,R)-1,2-propanediol

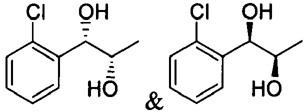

1-(2-chlorophenyl)-trans-1-propene(6.53g, Preparation Example 1) was dissolved in 45mL of the mixture of acetone/t-BuOH/H₂O(5:1:1 V/V). At the room temperature, N-methylmorpholine-N-oxide (7.51g) and OsO₄ (0.54g) were added thereto and stirred for 2-3 hours. When the reaction was completed, the obtained product was washed with water and methylenechloride (MC). Then, the organic layer was dehydrated with anhydrous magnesium sulfate (MgSO₄), filtrated, and concented under reduced pressure. The concentrated residue was purified by a silica gel column chromatography to produce the title compound (6.42g, yield 80%).
¹H NMR(400MHz, CDCl₃) δ1.20(d, *J*=6.4Hz, 3H), 2.48(d, *J*=4.0Hz, 1H), 2.92(d, *J*=4.4Hz, 1H), 3.93∼3.97(m, 1H), 4.97(t, *J*=4.8Hz, 1H), 7.22∼7.51(m, 4H)

### Preparation Example 17: Synthesis of 1-(2-chlorophenyl)-(S,S)-1,2-butanediol

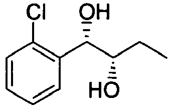

The substantially same method as described in Preparation Example 14 was conducted, except that 1-(2-chlorophenyl)-trans-1-butene(Preparation Example 2) was used instead of 1-(2-chlorophenyl)-trans-1-propene(Preparation Example 1), to obtain the title compound (0.36g, yield 95%).
¹H NMR(400MHz, CDCl₃) δ1.01(t, *J*=7.4Hz, 3H), 1.52∼1.65(m, 2H), 2.01(d, *J*=4.4Hz, 1H), 2.74(d, *J*=5.2Hz, 1H), 3.69∼3.75(m, 1H), 5.05(t, *J*=5.0Hz, 1H), 7.23∼7.54(m, 4H)

### Preparation Example 18: Synthesis of 1-(2-chlorophenyl)-(R,R)-1,2-butanediol

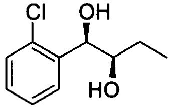

The substantially same method as described in Preparation Example 15 was conducted, except that 1-(2-chlorophenyl)-trans-1-butene(Preparation Example 2) was used instead of 1-(2-chlorophenyl)-trans-1-propene(Preparation Example 1), to obtain the title compound (0.84g, yield 60∼95%).
¹H NMR(400MHz, CDCl₃) δ1.01(t, *J*=7.4Hz, 3H), 1.52∼1.65(m, 2H), 2.01(d, *J*=4.4Hz, 1H), 2.74(d, *J*=5.2Hz, 1H), 3.69∼3.75(m, 1H), 5.05(t, *J*=5.0Hz, 1H), 7.23∼7.54(m, 4H)

### Preparation Example 19: Synthesis of the mixture of 1-(2-chlorophenyl)-(S,S)-1,2-butanediol and 1-(2-chlorophenyl)-(R,R)-1,2-butanediol

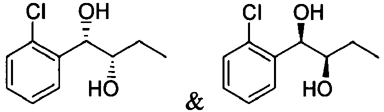

The substantially same method as described in Preparation Example 16 was conducted, except that 1-(2-chlorophenyl)-trans-1-butene(Preparation Example 2) was used instead of 1-(2-chlorophenyl)-trans-1-propene(Preparation Example 1), to obtain the title compound (5.1g, yield 60∼90%).
¹H NMR(400MHz, CDCl₃) δ1.01(t, *J*=7.4Hz, 3H), 1.52∼1.65(m, 2H), 2.01(d, *J*=4.4Hz, 1H), 2.74(d, *J*=5.2Hz, 1H), 3.69∼3.75(m, 1H), 5.05(t, *J*=5.0Hz, 1H), 7.23∼7.54(m, 4H)

### Preparation Example 20: Synthesis of 1-(2-chlorophenyl)-3-methyl-(S,S)-1,2-butanediol

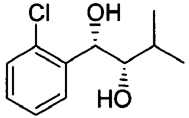

The substantially same method as described in Preparation Example 14 was conducted, except that 1-(2-chlorophenyl)-3-methyl-trans-1-butene(Preparation Example 3) was used instead of 1-(2-chlorophenyl)-trans-1-propene(Preparation Example 1), to obtain the title compound (0.96g, yield 60∼90%).
¹H NMR(400MHz, CDCl₃) δ1.07(t, *J*=7.2Hz, 6H), 1.83∼1.89(m, 1H), 1.92(d, *J=5.6Hz,* 1H), 2.69(d, *J*=6.4Hz, 1H), 3.53∼3.56(m, 1H), 5.22∼5.25(m, 1H), 7.23∼7.55(m, 4H)

### Preparation Example 21: Synthesis of 1-(2-chlorophenyl)-3-methyl-(R,R)-1,2-butanediol

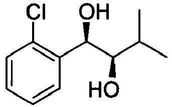

The substantially same method as described in Preparation Example 15 was conducted, except that 1-(2-chlorophenyl)-3-methyl-trans-1-butene(Preparation Example 3) was used instead of 1-(2-chlorophenyl)-trans-1-propene(Preparation Example 1), to obtain the title compound (4.2g, yield 60∼90%).
¹H NMR(400MHz, CDCl₃) δ1.07(t, *J*=7.2Hz, 6H), 1.82∼1.90(m, 1H), 1.93(d, *J*=5.6Hz, 1H), 2.79(d, *J*=6Hz, 1H), 3.53∼3.57(m, 1H), 5.23∼5.25(m, 1H), 7.23∼7.54(m, 4H)

### Preparation Example 22: Synthesis of the mixture of 1-(2-chlorophenyl)-3-methyl-(S,S)-1,2-butanediol and 1-(2-chlorophenyl)-3-methyl-(R,R)-1,2-butanediol

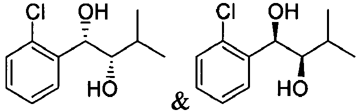

The substantially same method as described in Preparation Example 16 was conducted, except that 1-(2-chlorophenyl)-3-methyl-trans-1-butene(Preparation Example 3) was used instead of 1-(2-chlorophenyl)-trans-1-propene(Preparation Example 1), to obtain the title compound (0.8g, yield 60∼90%).
¹H NMR(400MHz, CDCl₃) δ1.07(t, *J*=7.2Hz, 6H), 1.83∼1.90(m, 1H), 1.92(d, J=5.6Hz, 1H), 2.69(d, *J*=6.4Hz, 1H), 3.53∼3.56(m, 1H), 5.22∼5.25(m, 1H), 7.23∼7.55(m, 4H)

### Preparation Example 23: Synthesis of 1-(2-chlorophenyl)-(S,S)-1,2-hexanediol

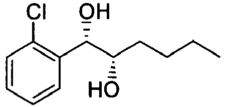

The substantially same method as described in Preparation Example 14 was conducted, except that 1-(2-chlorophenyl)-trans-1-hexene(Preparation Example 4) was used instead of 1-(2-chlorophenyl)-trans-1-propene(Preparation Example 1), to obtain the title compound (0.37g, yield 90%).
¹H NMR(400MHz, CDCl₃) δ0.90(t, *J*=7.2Hz, 3H), 1.35∼1.65(m, 6H), 2.08(d, *J*=4.4Hz, 1H), 2.71(d, *J*=5.2Hz, 1H), 3.78∼3.83(m, 1H), 5.04(t, *J*=5.OHz, 1H), 7.23∼7.53(m, 4H)

### Preparation Example 24: Synthesis of 1-(2-chlorophenyl)-(R,R)-1,2-hexanediol

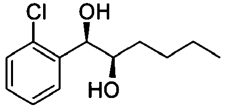

The substantially same method as described in Preparation Example 15 was conducted, except that 1-(2-chlorophenyl)-trans-1-hexene(Preparation Example 4) was used instead of 1-(2-chlorophenyl)-trans-1-propene(Preparation Example 1), to obtain the title compound (4.2g, yield 60∼90%).
¹H NMR(400MHz, CDCl₃) δ0.91(t, *J*=6.6Hz, 3H), 1.35∼1.65(m, 6H), 2.08(d, *J*=4.8Hz, 1H), 2.70(d, *J*=5.2Hz, 1H), 3.80∼3.83(m, 1H), 5.05(t, *J*=5.0Hz, 1H), 7.24∼7.56(m, 4H)

### Preparation Example 25: Synthesis of the mixture of 1-(2-chlorophenyl)-(S,S)-1,2-hexanediol and 1-(2-chlorophenyl)-(R,R)-1,2-hexanediol

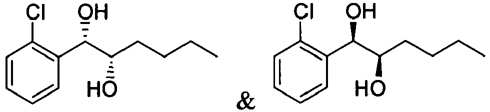

The substantially same method as described in Preparation Example 16 was conducted, except that 1-(2-chlorophenyl)-trans-1-hexene(Preparation Example 4) was used instead of 1-(2-chlorophenyl)-trans-1-propene(Preparation Example 1), to obtain the title compound (7.9g, yield 60∼90%).
¹H NMR(400MHz, CDCl₃) δ0.90(t, *J*=7.2Hz, 3H), 1.26∼1.55(m, 6H), 2.08(d, *J*=4.4Hz, 1H), 2.71(d, *J*=5.6Hz, 1H), 3.78∼3.84(m, 1H), 5.04(t, *J*=3.2Hz, 1H), 7.24∼7.55(m, 4H)

### Preparation Example 26: Synthesis of 1-(2,4-dichlorophenyl)-(S,S)-1,2-propanediol

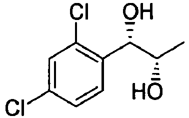

The substantially same method as described in Preparation Example 14 was conducted, except that 1-(2,4-ichlorophenyl)-trans-1-propene(Preparation Example 5) was used instead of 1-(2-chlorophenyl)-trans-1-propene(Preparation Example 1), to obtain the title compound (0.33g, yield 60∼95%).
¹H NMR(400MHz, CDCl₃) δ1.22(d, *J*=6.4Hz, 3H), 2.10(d, *J*=4.4Hz, 1H), 2.71(d, *J*=4.8Hz, 1H), 3.90∼3.95(m, 1H), 4.94(t, *J*=5.0Hz, 1H), 7.31(dd, *J*=2.0Hz, *J*=8.0Hz, 1H), 7.40(d, *J*=2.0Hz, 1H), 7.49(d, *J*=8.4Hz, 1H)

### Preparation Example 27: Synthesis of 1-(2,4-dichlorophenyl)-(R,R)-1,2-propanediol

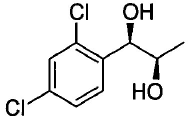

The substantially same method as described in Preparation Example 15 was conducted, except that 1-(2,4-ichlorophenyl)-trans-1-propene(Preparation Example 5) was used instead of 1-(2-chlorophenyl)-trans-1-propene(Preparation Example 1), to obtain the title compound (0.45g, yield 60∼95%).
¹H NMR(400MHz, CDCl₃) δ1.22(d, *J*=6.4Hz, 3H), 2.10(d, *J*=4.4Hz, 1H), 2.71(d, *J*=4.8Hz, 1H), 3.90∼3.95(m, 1H), 4.94(t, *J*=5.0Hz, 1H), 7.31∼7.49(m, 3H)

### Preparation Example 28: Synthesis of the mixture of 1-(2,4-dichlorophenyl)-(S,S)-1,2-propanediol and 1-(2,4-dichlorophenyl)-(R,R)-1,2-propanediol

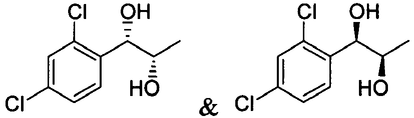

The substantially same method as described in Preparation Example 16 was conducted, except that 1-(2,4-ichlorophenyl)-trans-1-propene(Preparation Example 5) was used instead of 1-(2-chlorophenyl)-trans-1-propene(Preparation Example 1), to obtain the title compound (0.45g, yield 60∼95%).
¹H NMR(400MHz, CDCl₃) δ1.22(d, *J*=6.4Hz, 3H), 2.10(d, *J*=4.4Hz, 1H), 2.71(d, *J*=4.8Hz, 1H), 3.90∼3.95(m, 1H), 4.94(t, *J*=5.0Hz, 1H), 7.31∼7.49(m, 3H)

### Preparation Example 29: Synthesis of 1-(2,4-dichlorophenyl)-(S,S)-1,2-butanediol

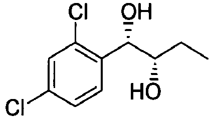

The substantially same method as described in Preparation Example 14 was conducted, except that 1-(2,4-dichlorophenyl)-trans-1-butene(Preparation Example 6) was used instead of 1-(2-chlorophenyl)-trans-1-propene(Preparation Example 1), to obtain the title compound (0.32g, yield 90%).
¹H NMR(400MHz, CDCl₃) δ1.02(t, *J*=7.4Hz, 3H), 1.54∼1.61(m, 2H), 2.07(d, *J*=4.8Hz, 1H), 2.74(d, *J*=4.8Hz, 1H), 3.65∼3.68(m, 1H), 5.01(t, *J*=5.0Hz, 1H), 7.31∼7.49(m, 3H)

### Preparation Example 30: Synthesis of 1-(2,4-dichlorophenyl)-(R,R)-1,2-butanediol

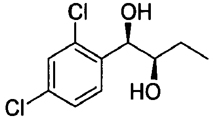

The substantially same method as described in Preparation Example 15 was conducted, except that 1-(2,4-dichlorophenyl)-trans-1-butene(Preparation Example 6) was used instead of 1-(2-chlorophenyl)-trans-1-propene(Preparation Example 1), to obtain the title compound (0.43g, yield 60∼90%).
¹H NMR(400MHz, CDCl₃) δ1.02(t, *J*=7.4Hz, 3H), 1.54∼1.61(m, 2H), 2.07(d, *J*=4.8Hz, 1H), 2.74(d, *J*=4.8Hz, 1H), 3.65∼3.68(m, 1H), 5.01(t, *J*=5.0Hz, 1H), 7.31∼7.49(m, 3H)

### Preparation Example 31: Synthesis of the mixture of 1-(2,4-dichlorophenyl)-(S,S)-1,2-butanediol and 1-(2,4-dichlorophenyl)-(R,R)-1,2-butanediol

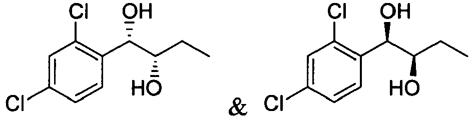

The substantially same method as described in Preparation Example 16 was conducted, except that 1-(2,4-dichlorophenyl)-trans-1-butene(Preparation Example 6) was used instead of 1-(2-chlorophenyl)-trans-1-propene(Preparation Example 1), to obtain the title compound (0.33g, yield 60∼90%).
¹H NMR(400MHz, CDCl₃) δ1.02(t, *J*=7.4Hz, 3H), 1.54∼1.61(m, 2H), 2.07(d, *J*=4.8Hz, 1H), 2.74(d, *J*=4.8Hz, 1H), 3.65∼3.68(m, 1H), 5.01(t, *J*=5.0Hz, 1H), 77.31∼7.49(m, 3H)

### Preparation Example 32: Synthesis of 1-(2,4-dichlorophenyl)-3-methyl-(S,S)-1,2-butanediol

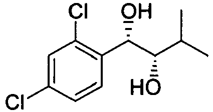

The substantially same method as described in Preparation Example 14 was conducted, except that 1-(2,4-dichlorophenyl)-3-methyl-trans-1-butene(Preparation Example 7) was used instead of 1-(2-chlorophenyl)-trans-1-propene(Preparation Example 1), to obtain the title compound (0.25g, yield 60∼95%).
¹H NMR(400MHz, CDCl₃) δ1.00(d, *J*=6.8Hz, 6H), 1.60∼1.65(m, 1H), 2.35(d, *J*=4.0Hz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 33: Synthesis of 1-(2,4-dichlorophenyl)-3-methyl-(R,R)-1,2-butanediol

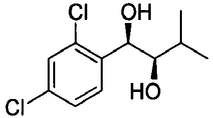

The substantially same method as described in Preparation Example 15 was conducted, except that 1-(2,4-dichlorophenyl)-3-methyl-trans-1-butene(Preparation Example 7) was used instead of 1-(2-chlorophenyl)-trans-1-propene(Preparation Example 1), to obtain the title compound (0.36g, yield 60∼95%).
¹H NMR(400MHz, CDCl₃) δ1.00(d, *J*=6.8Hz, 6H), 1.60∼1.65(m, 1H), 2.35(d, *J*=4.0Hz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 34: Synthesis of the mixture of 1-(2,4-dichlorophenyl)-3-methyl-(S,S)-1,2-butanediol and 1-(2,4-dichlorophenyl)-3-methyl-(R,R)-1,2-butanediol

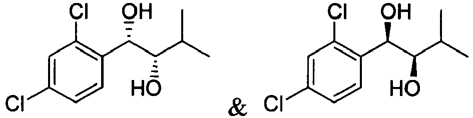

The substantially same method as described in Preparation Example 16 was conducted, except that 1-(2,4-dichlorophenyl)-3-methyl-trans-1-butene(Preparation Example 7) was used instead of 1-(2-chlorophenyl)-trans-1-propene(Preparation Example 1), to obtain the title compound (0.26g, yield 60∼95%).
¹H NMR(400MHz, CDCl₃) δ1.00(d, *J*=6.8Hz, 6H), 1.60∼1.65(m, 1H), 2.35(d, *J*=4.0Hz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 35: Synthesis of 1-(2,4-dichlorophenyl)-(S,S)-1,2-hexanediol

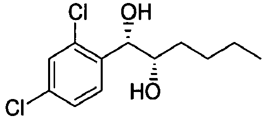

The substantially same method as described in Preparation Example 14 was conducted, except that 1-(2,4-dichlorophenyl)-trans-1-propene(Preparation Example 8) was used instead of 1-(2-chlorophenyl)-trans-1-propene(Preparation Example 1), to obtain the title compound (1.1g, yield 60∼90%).
¹H NMR(400MHz, CDCl₃) δ0.89∼0.93(m, 3H), 1.30∼1.39(m, 2H), 1.49∼1.52(m, 2H), 1.56∼1.62(m, 2H), 2.05(d, *J*=5.2Hz, 1H), 2.74(d, *J*=5.2Hz, 1H), 3.72∼3.77(m, 1H), 4.98(t, *J*=4.8Hz, 1H), 7.28∼7.50(m, 3H)

### Preparation Example 36: Synthesis of 1-(2,4-dichlorophenyl)-(R,R)-1,2-hexanediol

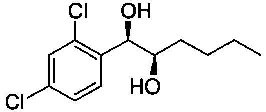

The substantially same method as described in Preparation Example 15 was conducted, except that 1-(2,4-dichlorophenyl)-trans-1-propene(Preparation Example 8) was used instead of 1-(2-chlorophenyl)-trans-1-propene(Preparation Example 1), to obtain the title compound (1.2g, yield 60∼95%).
¹H NMR(400MHz, CDCl₃) δ0.89∼0.93(m, 3H), 1.30∼1.39(m, 2H), 1.49∼1.52(m, 2H), 1.56∼1.62(m, 2H), 2.05(d, *J*=5.2Hz, 1H), 2.74(d, *J*=5.2Hz, 1H), 3.72∼3.77(m, 1H), 4.98(t, *J*=4.8Hz, 1H), 7.28∼7.50(m, 3H)

### Preparation Example 37: Synthesis of the mixture of 1-(2,4-dichlorophenyl)-(S,S)-1,2-hexanediol and 1-(2,4-dichlorophenyl)-(R,R)-1,2-hexanediol

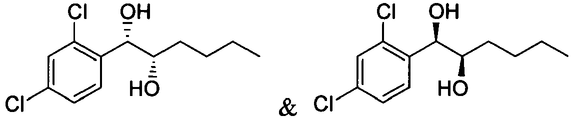

The substantially same method as described in Preparation Example 16 was conducted, except that 1-(2,4-dichlorophenyl)-trans-1-propene(Preparation Example 8) was used instead of 1-(2-chlorophenyl)-trans-1-propene(Preparation Example 1), to obtain the title compound (0.67g, yield 60∼95%).
¹H NMR(400MHz, CDCl₃) δ0.89∼0.93(m, 3H), 1.30∼1.39(m, 2H), 1.49∼1.52(m, 2H), 1.56∼1.62(m, 2H), 2.05(d, *J*=5.2Hz, 1H), 2.74(d, *J*=5.2Hz, 1H), 3.72∼3.77(m, 1H), 4.98(t, *J*=4.8Hz, 1H), 7.28∼7.50(m, 3H)

### Preparation Example 38: Synthesis of 1-(2,6-dichlorophenyl)-(S,S)-1,2-propanediol

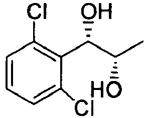

The substantially same method as described in Preparation Example 14 was conducted, except that 1-(2,6-dichlorophenyl)-trans-1-propene(Preparation Example 9) was used instead of 1-(2-chlorophenyl)-trans-1-propene(Preparation Example 1), to obtain the title compound (0.9g, yield 60∼90%).
¹H NMR(400MHz, CDCl₃) δ1.10(d, *J*=6.4Hz, 3H), 2.72(d, J=2.4Hz, 1H), 3.10(d, *J*=8.4Hz, 1H), 4.47∼4.54(m, 1H), 5.24(t, *J*=8.8Hz, 1H), 7.18∼7.36(m, 3H)

### Preparation Example 39: Synthesis of 1-(2,6-dichlorophenyl)-(R,R)-1,2-propanediol

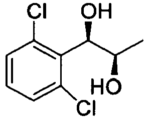

The substantially same method as described in Preparation Example 15 was conducted, except that 1-(2,6-dichlorophenyl)-trans-1-propene(Preparation Example 9) was used instead of 1-(2-chlorophenyl)-trans-1-propene(Preparation Example 1), to obtain the title compound (0.84g, yield 60∼90%).
¹H NMR(400MHz, CDCl₃) δ1.10(d, *J*=6.4Hz, 3H), 2.72(d, *J*=2.4Hz, 1H), 3.10(d, *J*=8.4Hz, 1H), 4.47∼4.54(m, 1H), 5.24(t, *J*=8.8Hz, 1H), 7.18∼7.36(m, 3H)

### Preparation Example 40: Synthesis of the mixture of 1-(2,6-dichlorophenyl)-(S,S)-1,2-propanediol and 1-(2,6-dichlorophenyl)-(R,R)-1,2-propanediol

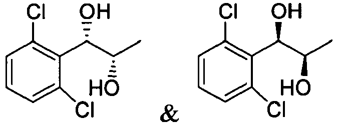

The substantially same method as described in Preparation Example 16 was conducted, except that 1-(2,6-dichlorophenyl)-trans-1-propene(Preparation Example 9) was used instead of 1-(2-chlorophenyl)-trans-1-propene(Preparation Example 1), to obtain the title compound (0.91g, yield 60∼90%).
¹H NMR(400MHz, CDCl₃) δ1.10(d, *J*=6.4Hz, 3H), 2.72(d, *J*=2.4Hz, 1H), 3.10(d, *J*=8.4Hz, 1H), 4.47∼4.54(m, 1H), 5.24(t, *J*=8.8Hz, 1H), 7.18∼7.3 6(m, 3H)

### Preparation Example 41: Synthesis of 1-(2,6-dichlorophenyl)-(S,S)-1,2-butanediol

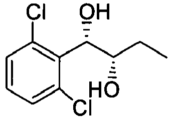

The substantially same method as described in Preparation Example 14 was conducted, except that 1-(2,6-dichlorophenyl)-trans-l-butene(Preparation Example 10) was used instead of 1-(2-chlorophenyl)-trans-1-propene(Preparation Example 1), to obtain the title compound (1.23g, yield 60∼95%).
¹H NMR(400MHz, CDCl₃) δ0.97(t, *J*=7.6Hz, 3H), 1.26∼1.53(m, 2H), 2.64(dd, *J*=0.8Hz, *J*=4.OHz, 1H), 3.14(d, *J*=8.4Hz, 1H), 4.22∼4.26(m, 1H), 5.26(t, *J*=8.4Hz, 1H), 7.17∼7.35(m, 3H)

### Preparation Example 42: Synthesis of 1-(2,6-dichlorophenyl)-(R,R)-1,2-butanediol

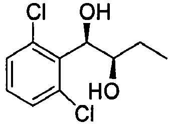

The substantially same method as described in Preparation Example 15 was conducted, except that 1-(2,6-dichlorophenyl)-trans-1-butene(Preparation Example 10) was used instead of 1-(2-chlorophenyl)-trans-1-propene(Preparation Example 1), to obtain the title compound (0.96g, yield 60∼95%).
¹H NMR(400MHz, CDCl₃) δ0.97(t, *J*=7.6Hz, 3H), 1.26∼1.53(m, 2H), 2.64(dd, *J*=0.8Hz, *J*=4.0Hz, 1H), 3.14(d, *J*=8.4Hz, 1H), 4.22∼4.26(m, 1H), 5.26(t, *J*=8.4Hz, 1H), 7.17∼7.35(m, 3H)

### Preparation Example 43: Synthesis of the mixture of 1-(2,6-dichlorophenyl)-(S,S)-1,2-butanediol and 1-(2,6-dichlorophenyl)-(R,R)-1,2-butanediol

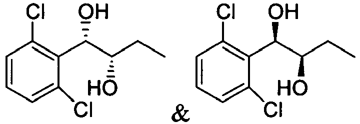

The substantially same method as described in Preparation Example 16 was conducted, except that 1-(2,6-dichlorophenyl)-trans-1-butene(Preparation Example 10) was used instead of 1-(2-chlorophenyl)-trans-1-propene(Preparation Example 1), to obtain the title compound (0.86g, yield 60∼95%).
¹H NMR(400MHz, CDCl₃) δ0.97(t, *J*=7.6Hz, 3H), 1.26∼1.53(m, 2H), 2.64(dd, *J*=0.8Hz, *J*=4.OHz, 1H), 3.14(d, *J*=8.4Hz, 1H), 4.22∼4.26(m, 1H), 5.26(t, *J*=8.4Hz, 1H), 7.17∼7.35(m, 3H)

### Preparation Example 44: Synthesis of 1-(2,6-dichlorophenyl)-3-methyl-(S,S)-1,2-butanediol

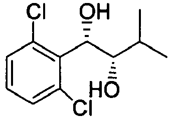

The substantially same method as described in Preparation Example 14 was conducted, except that 1-(2,6-dichlorophenyl)-3-methyl-trans-1-butene(Preparation Example 11) was used instead of 1-(2-chlorophenyl)-trans-1-propene(Preparation Example 1), to obtain the title compound (0.25g, yield 60∼95%).
¹H NMR(400MHz, CDCl₃) δ1.00(d, *J*=6.8Hz, 6H), 1.60∼1.65(m, 1H), 2.35(d, *J*=4.0Hz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 45: Synthesis of 1-(2,6-dichlorophenyl)-3-methyl-(R,R)-1,2-butanediol

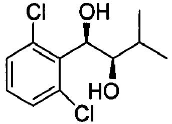

The substantially same method as described in Preparation Example 15 was conducted, except that 1-(2,6-dichlorophenyl)-3-methyl-trans-1-butene(Preparation Example 11) was used instead of 1-(2-chlorophenyl)-trans-1-propene(Preparation Example 1), to obtain the title compound (0.37g, yield 60∼95%).
¹H NMR(400MHz, CDCl₃) δ1.00(d, *J*=6.8Hz, 6H), 1.60∼1.65(m, 1H), 2.35(d, *J*=4.0Hz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 46: Synthesis of the mixture of 1-(2,6-dichlorophenyl)-3-methyl-(S,S)-1,2-butanediol and 1-(2,6-dichlorophenyl)-3-methyl-(R,R)-1,2-butanediol

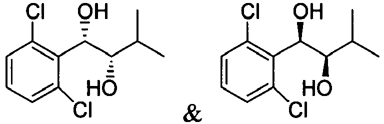

The substantially same method as described in Preparation Example 16 was conducted, except that 1-(2,6-dichlorophenyl)-3-methyl-trans-1-butene(Preparation Example 11) was used instead of 1-(2-chlorophenyl)-trans-1-propene(Preparation Example 1), to obtain the title compound (0.47g, yield 60∼95%).
¹H NMR(400MHz, CDCl₃) δ1.00(d, *J*=6.8Hz, 6H), 1.60∼1.65(m, 1H), 2.35(d, *J*=4.OHz, 1H), 3.12(d, *J*=8.4Hz, 1H), 4.13∼4.18(m, 1H), 5.36(t, *J*=7.6Hz, 1H), 7.17∼7.35(m, 3H)

### Preparation Example 47: Synthesis of 1-(2,6-dichlorophenyl)-(S,S)-1,2-hexanediol

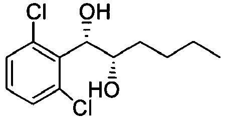

The substantially same method as described in Preparation Example 14 was conducted, except that 1-(2,6-dichlorophenyl)-trans-1-hexene(Preparation Example 12) was used instead of 1-(2-chlorophenyl)-trans-1-propene(Preparation Example 1), to obtain the title compound (0.36g, yield 60∼90%).
¹H NMR(400MHz, CDCl₃) δ0.85(t, *J*=6.8Hz, 3H), 1.20∼1.31(m, 4H), 1.45∼1.53(m, 2H), 2.61∼2.62(m, 1H), 3.12(d, *J*=8.4Hz, 1H), 4.28∼4.33(m, 1H), 5.25(t, *J*=8.4Hz, 1H), 7.18∼7.35(m, 3H)

### Preparation Example 48: Synthesis of 1-(2,6-dichlorophenyl)-(R,R)-1,2-hexanediol

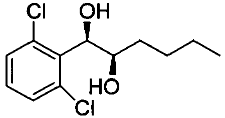

The substantially same method as described in Preparation Example 15 was conducted, except that 1-(2,6-dichlorophenyl)-trans-1-hexene(Preparation Example 12) was used instead of 1-(2-chlorophenyl)-trans-1-propene(Preparation Example 1), to obtain the title compound (0.58g, yield 60∼90%).
¹H NMR(400MHz, CDCl₃) δ0.85(t, *J*=6.8Hz, 3H), 1.20∼1.31(m, 4H), 1.45∼1.53(m, 2H), 2.61∼2.62(m, 1H), 3.12(d, *J*=8.4Hz, 1H), 4.28∼4.33(m, 1H), 5.25(t, *J*=8.4Hz, 1H), 7.18∼7.35(m, 3H)

### Preparation Example 49: Synthesis of the mixture of 1-(2,6-dichlorophenyl)-(S,S)-1,2-hexanediol and 1-(2,6-dichlorophenyl)-(R,R)-1,2-hexanediol

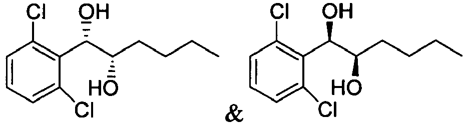

The substantially same method as described in Preparation Example 16 was conducted, except that 1-(2,6-dichlorophenyl)-trans-1-hexene(Preparation Example 12) was used instead of 1-(2-chlorophenyl)-trans-1-propene(Preparation Example 1), to obtain the title compound (0.62g, yield 60∼90%).
¹H NMR(400MHz, CDCl₃) δ0.85(t, *J*=6.8Hz, 3H), 1.20∼1.31(m, 4H), 1.45∼1.53(m, 2H), 2.61∼2.62(m, 1H), 3.12(d, *J*=8.4Hz, 1H), 4.28∼4.33(m, 1H), 5.25(t, *J*=8.4Hz, 1H), 7.18∼7.35(m, 3H)

### Preparation Example 50: Synthesis of methyl 2-(2-chlorophenyl)-(R)-2-hydroxyacetate

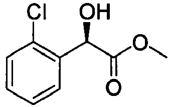

15g of (R)-2-chloromandelic acid was mixed with methanol (CH₃OH, 150ml) and phosphorus chloride oxide (POCl₃, 0.76ml) in a flask by stirring using a magnetic stirrer at the room temperature for 6 hours. When the reaction was completed, the obtained product was washed with an aqueous solution of sodium sulfite (Na₂SO₃) and ethylacetate (EA). Then, the organic layer was dehydrated with anhydrous magnesium sulfate (MgSO₄), filtrated, and concented under reduced pressure. The concentrated residue was purified by a silica gel column chromatography to produce the title compound (15.64g, yield 95%).
¹H NMR(400MHz, CDCl₃) δ 3.59(d, *J*=5.2, 1H), 3.79(t, *J*=6.0, 3H), 5.59(d, *J*=5.2, 1H), 7.28∼7.43(m, 4H)

### Preparation Example 51: Synthesis of 2-(2-chlorophenyl)-(R)-2-hydroxy-N-methoxy-N-methylacetamide

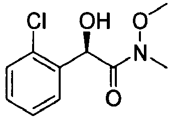

N,O-dimethylhydroxylamine hydrochloride (N,O-dimethylhydroxylamine.HCl, 15.2g) was dissolved in dichloromethane (DCM, 150ml), and cooled to 0°C using an ice-bath. Then, 77.7ml of 2.0M trimethylaluminium in hexane was slowly added thereto in drop-wise manner for 30 minutes. Thereafter, the ice-bath was removed, and the obtained product was stirred at the room temperature for 2 hours. Methyl-2-(2-chlorophenyl)-(R)-2-hydroxyacetate(15.64g) dissolved in dichloromethane(DCM, 150ml) was added in drop-wise manner thereto at the room temperature for 30 minutes, and subjected to reflux for 12 hours. When the reaction was completed, the obtained product was cooled to 0°C, and washed by a slow drop-wise addition of hydrochloric acid (HCl, 200ml). The obtained organic layer was washed with distilled water and brine, dehydrated with anhydrous magnesium sulfate (MgSO₄), filtrated, and concented under reduced pressure. The concentrated residue was purified by a silica gel column chromatography to produce the title compound (14.68g, yield 82%).
¹H NMR(400MHz, CDCl₃) δ3.23(s, 3H), 3.28(s, 3H), 4.33(d, *J*=6.0Hz, 1H), 5.81(d, *J*=5.6Hz, 1H), 7.23∼7.42(m, 4H)

### Preparation Example 52: Synthesis of 2-(2-chlorophenyl)-N-methoxy-(R)-2-(methoxymethoxy)-N-methylacetamide

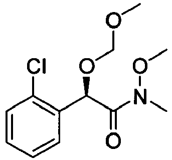

2-(2-chlorophenyl)-(R)-2-hydroxy-N-methoxy-N-methylacetamide (14.68g) obtained in Preparation Example 51 was dissolved in dichloromethane (DCM, 140ml), and cooled to 0°C. Diisopropylethylamine (55.67ml) was slowly added thereto in drop-wise manner, and stirred for 10 minutes. Chloro methyl methyl ether (25.25ml) was slowly added thereto in drop-wise manner for 30 minutes. After 30 minutes, the ice-bath was removed and the obtained product was stirred for 30 at room temperature. When the reaction was completed, the obtained product was cooled to 0°C. And then, to the obtained product, 1M sodium hydroxide solution (1M NaOH, 20ml) was added in drop-wise manner, and dichloromethane(DMC) was injected. Then the obtained product was washed with water. The obtained organic layer was dehydrated with anhydrous magnesium sulfate (MgSO₄), filtrated, and concented under reduced pressure. The concentrated residue was purified by a silica gel column chromatography to produce the title compound (15.57g, yield 89%).
¹H NMR(400MHz, CDCl₃) δ3.19(s, 3H), 3.42(s, 3H), 3.47(s, 3H), 4.75(d, *J*=6.8, 1H), 4.81(d, *J*=6.8, 1H), 6.07(s, 1H), 7.27∼7.58(m, 4H)

### Preparation Example 53: Synthesis of 1-(2-chlorophenyl)-(R)-1-(methoxymethoxy)propane-2-on

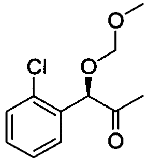

2-(2-chlorophenyl)-N-methoxy-(R)-2-(methoxymethoxy)-N-methylacetamide(15.57g) obtained in Preparation Example 52 was dissolved in tetrahydrofuran(THF, 150ml), and cooled to 0°C. 3.0M methyl magnesium bromide (MeMgBr) solution in ether was added thereto in drop-wise manner for 30 minutes, and the obtained product was stirred for 1 hour at 0°C. When the reaction was completed, diethylether(100ml) was added thereto. The obtained product was washed with 10%(w/v) potassium hydrogen sulfate (KHSO₄, 100ml) and then, washed again with brine. The obtained organic layer was dehydrated with anhydrous magnesium sulfate (MgSO₄), filtrated, and concented under reduced pressure. The concentrated residue was purified by a silica gel column chromatography to produce the title compound (11.83g, yield 90%).
¹H NMR(400MHz, CDCl₃) δ2.18(s, 3H), 3.39(s, 3H), 4.65(d, *J*=6.8, 1H), 4.74(d, *J*=6.8, 1H), 5.63(s, 1H), 7.30∼7.45(m, 4H)

### Preparation Example 54: Synthesis of 1-(2-chlorophenyl)-(R)-1-(methoxymethoxy)-(S)-2-propanol

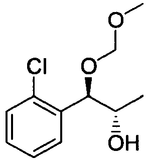

1-(2-chlorophenyl)-(R)-1-(methoxymethoxy)propane-2-on(11.83g) obtained in Preparation Example 53 was dissolved in toluene(110m1), and cooled to -40°C. Sodium bis(2-methoxyethoxy)aluminumhydride solution (15.7ml) in toluene was slowly added thereto for 30 minutes, and then, the obtained product was stirred for 1 hour. When the reaction was completed, the obtained product was washed by slow drop-wise addition of sodium potassium tartrate (100ml). The obtained organic layer was dehydrated with anhydrous magnesium sulfate (MgSO₄), filtrated, and concented under reduced pressure. The concentrated residue was purified by a silica gel column chromatography to produce the title compound (10.38g, yield 87%).
¹H NMR(400MHz, CDCl₃) δ1.13(d, *J*=6.4, 3H), 2.33(d, *J*=7.2, 1H), 3.44(s, 3H), 4.10∼4.18(m, 1H), 4.61(d, *J*=6.4, 1H), 4.69(d, *J*=6.8, 1H), 5.14(d, *J*=3.6, 1H), 7.22∼7.55(m, 4H)

### Preparation Example 55: Synthesis of 1-(2-chlorophenyl)-(R,S)-1,2-propanediol

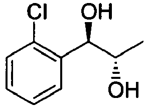

1-(2-chlorophenyl)-(R)-1-(methoxymethoxy)-(S)-2-propanol(10.38g) obtained in Preparation Example 54 was dissolved in methanol (CH₃OH, 100ml), and then, cooled to 0°C. 8M hydrochloric acid(HCl, 56.2ml) was slowly added in drop-wise manner to the obtained product, and then, the obtained product was warmed to the room temperature, and stirred for 15 hours. When the reaction was completed, the obtained product was cooled to 0°C. 5N sodium hydroxide (NaOH, 30ml) was slowly added thereto, and the obtained product was subjected to vacuum concentration. The obtained product was diluted with ethylacetate. The obtained organic layer was washed with distilled water, dehydrated with anhydrous magnesium sulfate (MgSO₄), filtrated, and concented under reduced pressure. The concentrated residue was purified by a silica gel column chromatography to produce the title compound (7.05g, yield60∼90%).
¹H NMR(400MHz, CDCl₃) δ1.07(d, *J*=6.8, 3H), 2.01(d, *J*=5.6, 1H), 2.61(s, 1H), 4.21∼4.27(m, 1H), 5.24(d, *J*=3.6, 1H), 7.22∼7.64(m, 4H)

### Preparation Example 56: Synthesis of 1-(2-chlorophenyl)-(S,R)-1,2-propanediol

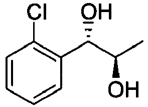

The substantially same method as described in Preparation Examples 50-55 was conducted, except that (S)-2-chloromandelic acid was used instead of (R)-2-chloromandelic acid, to obtain the title compound (5.04g, yield 84%).
¹H NMR(400MHz, CDCl₃) δ1.07(d, *J*=6.8, 3H), 2.00(d, *J*=5.6, 1H), 2.54(d, *J*=3.6, 1H), 4.22∼4.26(m, 1H), 5.25(t, *J*=3.2, 1H), 7.22∼7.65(m, 4H)

### Preparation Example 57: Synthesis of 1-(2,3-dichlorophenyl)-(S,S)-1,2-propanediol

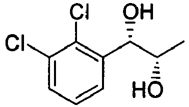

The substantially same method as described in Preparation Example 14 was conducted, except that 1-(2,3-dichlorophenyl)-trans-1-propene(Preparation Example 13) was used instead of 1-(2-chlorophenyl)-trans-1-propene(Preparation Example 1), to obtain the title compound (0.9g, yield 60∼90%).
¹H NMR(400MHz, CDCl₃) δ1.10(d, *J*=6.4Hz, 3H), 2.72(d, *J*=2.4Hz, 1H), 3.10(d, *J*=8.4Hz, 1H), 4.47∼4.54(m, 1H), 5.24(t, *J*=8.8Hz, 1H), 7.18∼(m, 3H)

### Preparation Example 58: Synthesis of 1-(2,3-dichlorophenyl)-(R,R)-1,2-propanediol

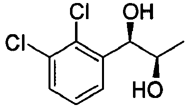

The substantially same method as described in Preparation Example 15 was conducted, except that 1-(2,3-dichlorophenyl)-trans-1-propene(Preparation Example 13) was used instead of 1-(2-chlorophenyl)-trans-1-propene(Preparation Example 1), to obtain the title compound (0.84g, yield 60∼90%).
¹H NMR(400MHz, CDCl₃) δ1.10(d, *J*=6.4Hz, 3H), 2.72(d, *J*=2.4Hz, 1H), 3.10(d, *J*=8.4Hz, 1H), 4.47∼4.54(m, 1H), 5.24(t, *J*=8.8Hz, 1H), 7.18∼ (m, 3H)

### Preparation Example 59: Synthesis of the mixture of 1-(2,3-dichlorophenyl)-(S,S)-1,2-propanediol and 1-(2,3-dichlorophenyl)-(R,R)-1,2-propanediol

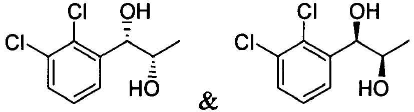

The substantially same method as described in Preparation Example 16 was conducted, except that 1-(2,3-dichlorophenyl)-trans-1-propene(Preparation Example 13) was used instead of 1-(2-chlorophenyl)-trans-1-propene(Preparation Example 1), to obtain the title compound (0.91g, yield 60∼90%).
¹H NMR(400MHz, CDCl₃) δ1.10(d, *J*=6.4Hz, 3H), 2.72(d, *J*=2.4Hz, 1H), 3.10(d, *J*=8.4Hz, 1H), 4.47∼4.54(m, 1H), 5.24(t, *J*=8.8Hz, 1H), 7.18∼(m, 3H)

### Preparation Example 60: Synthesis of 1-(2-fluorophenyl)-trans-1-propene

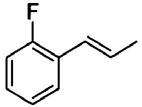

The substantially same method as described in Preparation Example 1 was conducted, except that 2-fluorobenzenaldehyde was used instead of 2-chlorobenzenealdehyde, to obtain the title compound (6.67g, yield 61%).)
¹H NMR(400MHz, CDCl₃) δ1.94(d, *J*=6.8Hz, 3H), 6.30∼6.38(m, 1H), 6.57(d, *J*=16Hz, 1H), 7.00∼7.41(m, 4H)

### Preparation Example 61: Synthesis of 1-(2-fluorophenyl)-(S,S)-1,2-propanediol

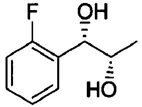

The substantially same method as described in Preparation Example 14 was conducted, except that 1-(2-fluorophenyl)-trans-1-propene (Preparation Example 60) was used instead of 1-(2-chlorophenyl)-trans-1-propene (Preparation Example 1), to obtain the title compound (6.46g, yield 78%).
¹H NMR(400MHz, CDCl₃) δ1.15(d, *J*=6.4Hz, 3H), 2.43(d, *J*=3.6Hz, 1H), 2.69(d, *J*=4.8Hz, 1H), 3.90∼3.98(m, 1H), 4.78(dd, *J*=4.4, 7.2Hz, 1H), 7.04∼7.50(m, 4H)

### Preparation Example 62: Synthesis of 1-(2-fluorophenyl)-(R,R)-1,2-propanediol

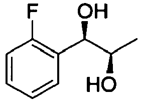

The substantially same method as described in Preparation Example 15 was conducted, except that 1-(2-fluorophenyl)-trans-1-propene (Preparation Example 60) was used instead of 1-(2-chlorophenyl)-trans-1-propene (Preparation Example 1), to obtain the title compound (3.29g, yield 79%).
¹H NMR(400MHz, CDCl₃) δ1.15(d, *J*=6.4Hz, 3H), 2.43(d, *J*=3.6Hz, 1H), 2.69(d, *J*=4.8Hz, 1H), 3.90∼3.98(m, 1H), 4.78(dd, *J*=4.4, 7.2Hz, 1H), 7.04∼7.50(m, 4H)

### Preparation Example 63: Synthesis of 2-iodobenzenealdehyde

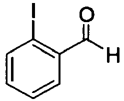

In a flask, 2-iodobenzyl alcohol (4g, 17.09mmol) was dissolved in dichloromethane (MC, 85ml), and then, manganese oxide (MnO₂, 14.86g, 170.92mmol) was added thereto. The obtained reaction product was stirred under the reflux condition. When the reaction was completed, the obtained reaction product was cooled to the room temperature, and then, fiteated and concentrated using celite, to obtain the title compound (3.6g, yield 91%).
¹H NMR(400MHz, CDCl₃)δ7.30∼7.99(m, 4H), 10.10(s, 1H)

### Preparation Example 64: Synthesis of 1-(2-iodophenyl)-trans-1-propene

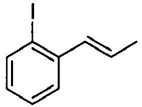

The substantially same method as described in Preparation Example 1 was conducted, except that 2-iodobenzenealdehyde (Preparation Example 63) was used instead of 2-chlorobenzenealdehyde, to obtain the title compound (3.4g, yield 65%).
¹H NMR(400MHz, CDCl₃)δ1.95(dd, *J*=6.8Hz, 1.6Hz, 3H), 6.09∼6.18(m, 1H), 6.60(dd, *J*=15.66Hz, 1.8Hz, 1H), 6.89∼7.84(m, 4H)

### Preparation Example 65: Synthesis of 1-(2-iodophenyl)-trans-1-butene

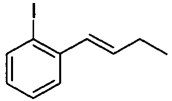

The substantially same method as described in Preparation Example 64 was conducted, except that 3-heptanone was used instead of 3-pentanone, to obtain the title compound (8.5g, yield 75%).
¹H NMR(400MHz, CDCl₃)δ1.46(t, *J*=7.6Hz, 3H), 2.26∼2.34(m, 2H), 6.17(dt, *J*=15.6Hz, 6.6Hz 1H), 6.57(d, *J*=15.6Hz, 1H), 6.89∼7.85(m, 4H)

### Preparation Example 66: Synthesis of 1-(2-iodophenyl)-(S,S)-1,2-propanediol

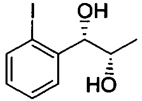

The substantially same method as described in Preparation Example 14 was conducted, except that 1-(2-iodophenyl)-trans-1-propene (Preparation Example 64) was used instead of 1-(2-chlorophenyl)-trans-1-propene (Preparation Example 1), to obtain the title compound (3.4g, yield 88%).
¹H NMR(400MHz, CDCl₃)δ1.27(d, *J*=6.4Hz, 3H), 2.26(br s, 1H), 2.74(br s, 1H), 3.99(t, *J*=6.0Hz, 1H), 4.81(d, *J*=4.0Hz, 1H), 7.01∼7.87(m, 4H)

### Preparation Example 67: Synthesis of 1-(2-iodorophenyl)-(R,R)-1,2-propanediol

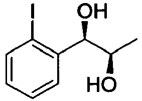

The substantially same method as described in Preparation Example 15 was conducted was conducted, except that **1-(2-iodophenyl)-trans-1-propene** (Preparation Example 64) was used instead of 1-(2-chlorophenyl)-trans-1-propene (Preparation Example 1), to obtain the title compound (7.4g, yield 84%).
¹H NMR(400MHz, CDCl₃)δ1.26(d, *J*=6.4Hz, 3H), 2.35(br s, 1H), 2.85(br d, *J*=4.0Hz, 1H), 3.98(t, *J*=6.2Hz, 1H), 4.80(dd, *J*=5.0, 4.4Hz, 1H), 7.00∼7.87(m, 4H)

### Preparation Example 68: Synthesis of 1-(2-iodophenyl)-(S,S)-1,2-butanediol

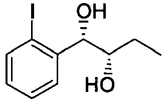

The substantially same method as described in Preparation Example 14 was conducted was conducted, except that 1-(2-iodophenyl)-trans-1-butene (Preparation Example 65) was used instead of 1-(2-chlorophenyl)-trans-1-propene (Preparation Example 1), to obtain the title compound (9.5g, yield 84%).
¹H NMR(400MHz, CDCl₃)δ1.04(t, *J*=7.6Hz, 3H), 1.60∼1.71(m, 2H), 2.07(br s, 1H), 2.74(br s, 1H), 3.71∼3.76(m, 1H), 4.87(d, *J*=4.8Hz, 1H), 7.01∼7.87(m, 4H)

### Example 1: Synthesis of 1-(2-chlorophenyl)-(S)-1-hydroxypropyl-(S)-2-carbamate(1)

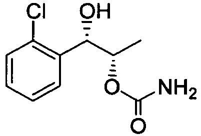

1-(2-chlorophenyl)-(S,S)-1,2-propanediol(2.33g) obtained in Preparation Example 14, tetrahydrofuran (THF, 12ml), and carbonyldiimidazole (CDI, 3.04g) were put into a flask and stirred at the room temperature. After approximately 3 hours, ammonia solution (NH₄OH, 4ml) was added thereto. When the reaction was completed, the obtained product was washed with 1M HCl solution and ethylacetate (EA). The separated organic layer was dehydrated with anhydrous magnesium sulfate (MgSO₄), filtrated, and concented under reduced pressure. The concentrated residue was purified by a silica gel column chromatography, to obtain the title compound (1.40g, yield 49%).
M.P. 83∼84°C
¹H NMR(400MHz, CDCl₃) δ1.24(d, *J*=6.4Hz, 3H), 2.91(d, *J*=4.8Hz, 1H), 4.68(br s, 2H), 5.06∼5.09(m, 1H), 5.18∼5.21(m, 1H), 7.23∼7.55(m, 4H)
¹³C NMR(100MHz, CDCl₃) δ16.4, 73.1, 75.0, 127.0, 128.4, 129.1, 129.5, 132.7, 138.0, 156.6

### Example 2: Synthesis of 1-(2-chlorophenyl)-(R)-1-hydroxypropyl-(R)-2-carbamate(2)

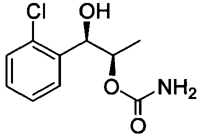

The substantially same method as described in Example 1 was conducted, except that 1-(2-chlorophenyl)-(R,R)-1,2-propanediol obtained in Preparation Example 15 was used instead of 1-(2-chlorophenyl)-(S,S)-1,2-propanediol, to obtain the title compound (1.74g, yield 50%).
M.P. 85∼86°C
¹H NMR(400MHz, CDCl₃) δ1.24(d, *J*=6.4Hz, 3H), 2.98(d, *J*=4.0Hz, 1H), 4.73(br s, 2H), 5.04∼5.10(m, 1H), 5.18∼5.20(m, 1H), 7.24∼7.55(m, 4H)

### Example 3: Synthesis of 1-(2-chlorophenyl)-1-hydroxypropyl-2-carbamate(3)

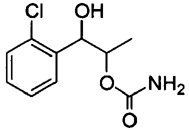

The substantially same method as described in Example 1 was conducted, except that the mixture of 1-(2-chlorophenyl)-(S,S)-1,2-propanediol and 1-(2-chlorophenyl)-(R,R)-1,2-propanediol obtained in Preparation Example 16 was used instead of 1-(2-chlorophenyl)-(S,S)-1,2-propanediol, to obtain the title compound (0.41g, yield 38%).
¹H NMR(400MHz, CDCl₃) δ1.14(d, *J* = 6.8Hz, 3H), 3.34(d, *J* = 3.2Hz, 1H), 5.06(brs, 2H), 5.09∼5.15(m, 1H), 5.31(br t, *J* = 2.4Hz, 1H), 7.18∼7.59(m, 4H)

### Example 4: Synthesis of 1-(2-chlorophenyl)-(R)-1-hydroxypropyl-(S)-2-carbamate(4)

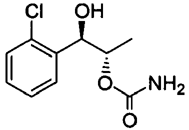

The substantially same method as described in Example 1 was conducted, except that 1-(2-chlorophenyl)-(R,S)-1,2-propanediol obtained in Preparation Example 55 was used instead of 1-(2-chlorophenyl)-(S,S)-1,2-propanediol, to obtain the title compound (1.7g, yield 50%).
¹H NMR(400MHz, CDC13) δ1.20(d, J=6.8, 3H), 2.68(s, 1H), 4.67(s, 2H), 5.16∼5.22(m, 1H), 5.36(t, J=3.2, 1H), 7.23∼7.61(m, 4H)

### Example 5: Synthesis of 1-(2-chlorophenyl)-(S)-1-hydroxypropyl-(R)-2-carbamate(5)

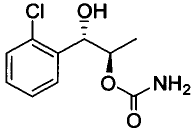

The substantially same method as described in Example 1 was conducted, except that 1-(2-chlorophenyl)-(S,R)-1,2-propanediol obtained in Preparation Example 56 was used instead of 1-(2-chlorophenyl)-(S,S)-1,2-propanediol, to obtain the title compound (1.74g, yield 50%).
¹H NMR(400MHz, CDC13) δ1.20(d, *J*=6.4, 3H), 2.83(d, *J*=3.6, 1H), 4.78(s, 2H), 5.15∼5.21(m, 1H), 5.36(t, *J*=3.2, 1H), 7.23∼7.63(m, 4H)

### Example 6: Synthesis of 1-(2-chlorophenyl)-(S)-1-hydroxybutyl-(S)-2-carbamate(6)

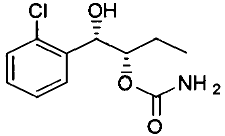

The substantially same method as described in Example 1 was conducted, except that 1-(2-chlorophenyl)-(S,S)-1,2-butanediol obtained in Preparation Example 17 was used instead of 1-(2-chlorophenyl)-(S,S)-1,2-propanediol, to obtain the title compound (1.0g, yield 45%).
¹H NMR(400MHz, CDCl₃) δ0.96(t, *J*= 7.4Hz, 3H), 1.57∼1.73(m, 2H), 3.01(d, *J* = 5.6Hz, 1H), 4.74(br s, 2H), 4.95(dt, *J* = 7.2, 8.8Hz, 1H), 5.23(t, *J* = 5.6Hz, 1H), 7.22∼7.54(m, 4H)

### Example 7: Synthesis of 1-(2-chlorophenyl)-(R)-1-hydroxybtyl-(R)-2-carbamate(7)

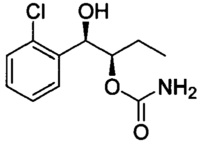

The substantially same method as described in Example 1 was conducted, except that 1-(2-chlorophenyl)-(R,R)-1,2-butanediol obtained in Preparation Example 18 was used instead of 1-(2-chlorophenyl)-(S,S)-1,2-propanediol, to obtain the title compound (1.5g, yield 25%).
¹H NMR(400MHz, CDCl₃) δ 0.94(t, *J*=7.4Hz, 3H), 1.53∼1.73(m, 2H), 2.92(s, 1H), 4.78(br s, 2H), 4.91∼4.96(m, 1H), 5.22(d, *J*=5.5Hz, 1H), 7.20∼7.54(m, 4H)

### Example 8: Synthesis of 1-(2-chlorophenyl)-1-hydroxybutyl-2-carbamate(8)

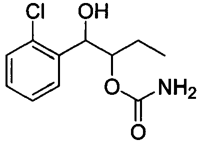

The substantially same method as described in Example 1 was conducted, except that 1-(2-chlorophenyl)-1,2-butanediol obtained in Preparation Example 19 was used instead of 1-(2-chlorophenyl)-(S,S)-1,2-propanediol, to obtain the title compound (1.8g, yield 30%).
¹H NMR(400MHz, CDCl₃) δ 0.97(t, *J*=7Hz, 3H), 1.58∼1.74(m, 2H), 2.94(d, *J*=6Hz, 1H), 4.69(br s, 2H), 4.94∼4.99(m, 1H), 5.24(t, *J*=6Hz, 1H), 7.23∼7.56(m, 4H)

### Example 9: Synthesis of 1-(2-chlorophenyl)-(S)-1-hydroxy-3-methyl-butyl-(S)-2-carbamate(9)

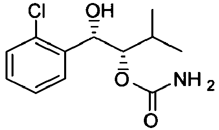

The substantially same method as described in Example 1 was conducted, except that 1-(2-chlorophenyl)-3-methyl-(S,S)-1,2-butanediol obtained in Preparation Example 20 was used instead of 1-(2-chlorophenyl)-(S,S)-1,2-propanediol, to obtain the title compound (0.72g, yield 48%).
¹H NMR(400MHz, CDCl₃) δ1.01(d, J = 6.4Hz, 3H), 1.09(d, *J* = 6.8Hz, 3H), 2.06(m, 1H), 2.75(d, *J* = 6.8Hz, 1H), 4.58(br s, 2H), 4.85∼4.88(m, 1H), 5.34∼5.37(m, 1H), 7.22∼7.33(m, 2H), 7.35∼7.37(m, 1H), 7.51∼7.53(m, 1H)

### Example 10: Synthesis of 1-(2-chlorophenyl)-(R)-1-hydroxy-3-methylbutyl-(R)-2-carbamate(10)

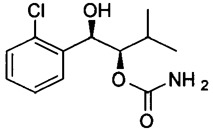

The substantially same method as described in Example 1 was conducted, except that 1-(2-chlorophenyl)-3-methyl-(R,R)-1,2-butanediol obtained in Preparation Example 21 was used instead of 1-(2-chlorophenyl)-(S,S)-1,2-propanediol, to obtain the title compound (0.56g, yield 43%).
¹H NMR(400MHz, CDCl₃) δ1.01(d, *J* = 6.8Hz, 3H), 1.09(d, *J* = 6.8Hz, 3H), 2.06(m, 1H), 2.73(d, *J* = 6.8Hz, 1H), 4.57(br s, 2H), 4.85∼4.88(m, 1H), 5.34∼5.37(m, 1H), 7.24∼7.30(m, 2H), 7.35∼7.37(m, 1H), 7.51∼7.53(m, 1H)

### Example 11: Synthesis of 1-(2-chlorophenyl)-1-hydroxy-3-methyl-butyl-2-carbamate(11)

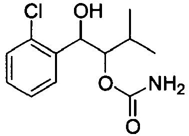

The substantially same method as described in Example 1 was conducted, except that 1-(2-chlorophenyl)-3-methyl-1,2-butanediol obtained in Preparation Example 22 was used instead of 1-(2-chlorophenyl)-(S,S)-1,2-propanediol, to obtain the title compound (1.5g, yield 23%).
¹H NMR(400MHz, CDCl₃) δ1.00(d, *J*=6.4Hz, 3H), 1.09(d, *J*=6.4Hz, 3H), 2.08(m, 1H), 2.76(d, *J*=6.0Hz, 1H), 4.59(br s, 2H), 4.87(dd, *J*=7.2Hz, 4.4Hz, 1H), 5.36(t, *J*=4.6, 1H), 7.23∼7.54(m, 4H)

### Example 12: Synthesis of 1-(2-chlorophenyl)-(S)-1-hydroxyhexyl-(S)-2-carbamate(12)

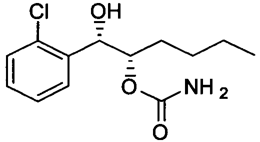

The substantially same method as described in Example 1 was conducted, except that 1-(2-chlorophenyl)-(S,S)-1,2-hexanediol obtained in Preparation Example 23 was used instead of 1-(2-chlorophenyl)-(S,S)-1,2-propanediol, to obtain the title compound (0.24g, yield 49%).
¹H NMR(400MHz, CDCl₃) δ0.88(t, *J* = 7Hz, 3H), 1.33∼1.42(m, 4H), 1.53∼1.71(m, 2H), 2.89(d, *J* = 5.6Hz, 1H) 4.64(br s, 2H), 5.04(dt, *J* = 5.0, 9.0Hz, 1H), 5.20(t, *J* = 5.6Hz, 1H), 7.23∼7.55(m, 4H)

### Example 13: Synthesis of 1-(2-chlorophenyl)-(R)-1-hydroxyhexyl-(R)-2-carbamate(13)

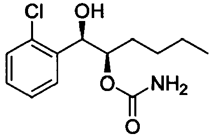

The substantially same method as described in Example 1 was conducted, except that 1-(2-chlorophenyl)-(R,R)-1,2-hexanediol obtained in Preparation Example 24 was used instead of 1-(2-chlorophenyl)-(S,S)-1,2-propanediol, to obtain the title compound (2.2g, yield 44%).
¹H NMR(400MHz, CDCl₃) δ 0.89(dd, *J*=5Hz, 3H), 1.28∼1.43(m, 4H), 1.52∼1.58(m, 1H), 1.65∼1.72(m, 1H), 2.90(d, *J*=6Hz, 1H), 4.64(br s, 2H), 5.01∼5.06(m, 1H), 5.22(t, *J*=6Hz, 1H), 7.22∼7.56(m, 4H)

### Example 14: Synthesis of 1-(2-chlorophenyl)-1-hydroxyhexyl-2-carbamate(14)

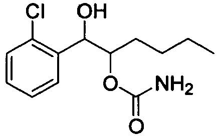

The substantially same method as described in Example 1 was conducted, except that 1-(2-chlorophenyl)-1,2-hexanediol obtained in Preparation Example 25 was used instead of 1-(2-chlorophenyl)-(S,S)-1,2-propanediol, to obtain the title compound (1.6g, yield 34%).
¹H NMR(400MHz, CDCl₃) δ 0.88(dd, *J*=5Hz, 3H), 1.31∼1.43(m, 4H), 1.63∼1.70(m, 1H), 1.52∼1.60(m, 1H), 3.06(d, *J*=6Hz, 1H), 4.75(br s, 2H), 5.00∼5.05(m, 1H), 5.21(t, *J*=6Hz, 1H), 7.22∼7.55(m, 4H)

### Example 15: Synthesis of 1-(2-chlorophenyl)-(S)-1-hydroxypropyl-(S)-2-N-methylcarbamate(15)

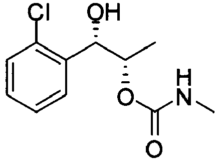

The substantially same method as described in Example 1 was conducted, except that methylamine was used instead of ammonia solution(NH₄OH), to obtain the title compound (1.6g, yield 51%).
¹H NMR(400MHz, CDCl₃) δ1.03∼1.25(m, 3H), 2.76(s, 3H), 3.34(s, 1H), 4.80(br s 1H), 5.04(t, *J*=12.5Hz, 1H), 5.14(s, 1H), 7.20∼7.53(m, 4H)

### Example 16: Synthesis of 1-(2-chlorophenyl)-(S)-1-hydroxypropyl-(S)-2-N-propylcarbamate(16)

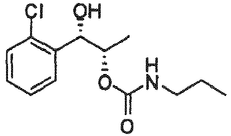

The substantially same method as described in Example 1 was conducted, except that propylamine was used instead of ammonia solution(NH₄OH), to obtain the title compound (0.79g, yield 25%).
¹H NMR(400MHz, CDCl₃) δ0.90(t, *J*=6.8Hz, 3H), 1.20(d, *J*=5.96Hz, 3H), 1.49(dd, *J*=14.2Hz, 2H), 3.11(d, *J*=6.28Hz, 2H), 3.34(s, 1H), 4.84(br s, 1H), 5.05(t, *J*=5.88Hz, 1H), 5.14(s, 1H), 7.22∼7.53(m, 4H)

### Example 17: Synthesis of 1-(2-chlorophenyl)-(S)-1-hydroxypropyl-(S)-2-N-isopropylcarbamate(17)

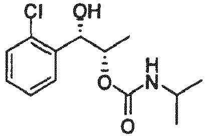

The substantially same method as described in Example 1 was conducted, except that isopropylamine was used instead of ammonia solution(NH₄OH), to obtain the title compound (1.5g, yield 41%).
¹H NMR(400MHz, CDCl₃) δ1.14(dd, *J*=6.5Hz, 6H), 1.19(d, *J*=6.4Hz, 3H), 3.21(s, 1H), 3.73∼3.82(m, 1H), 4.59(br s, 1H), 5.01∼5.07(m, 1H), 5.14(t, *J*=5.8Hz, 1H), 7.20∼7.53(m, 4H)

### Example 18: Synthesis of 1-(2-chlorophenyl)-(S)-1-hydroxypropyl-(S)-2-N-cyclopropylcarbamate(18)

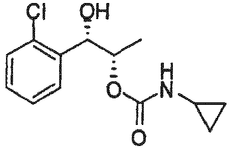

The substantially same method as described in Example 1 was conducted, except that cyclopropylamine was used instead of ammonia solution(NH₄OH), to obtain the title compound (2.2g, yield 43%).
¹H NMR(400MHz, CDCl₃) δ0.50∼0.56(m, 2H), 0.74(d, J=7.21Hz, 2H), 1.25(s, 3H), 2.56∼2.61(m, 1H), 3.72(s, 1H), 4.98(br s, 1H), 5.05∼5.11(m, 1H), 7.16(s, 1H), 7.23∼7.54(m, 4H)

### Example 19: Synthesis of 1-(2-chlorophenyl)-(S)-1-hydroxypropyl-(S)-2-N-cyclohexyl carbamate(19)

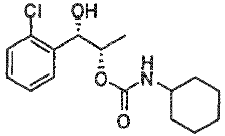

The substantially same method as described in Example 1 was conducted, except that cyclohexylamine was used instead of ammonia solution(NH₄OH), to obtain the title compound (1.1g, yield 26%).
¹H NMR(400MHz, CDCl₃) δ1.06∼1.40(m, 7H), 1.56∼1.61(m, 2H), 1.69∼1.71(m, 2H), 1.87∼1.94(m, 2H), 3.19(d, *J*=4.32Hz, 1H), 3.45(s, 1H), 4.64(br s 1H), 5.02∼5.07(m, 1H), 5.14(t, *J*=6.08Hz, 1H) 7.20∼7.53(m, 4H)

### Example 20: Synthesis of 1-(2-chlorophenyl)-(S)-1-hydroxypropyl-(S)-2-N-benzyl carbamate(20)

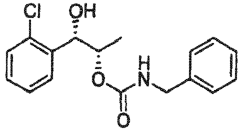

The substantially same method as described in Example 1 was conducted, except that benzylamine was used instead of ammonia solution(NH₄OH), to obtain the title compound (1.2g, yield 18%).
¹H NMR(400MHz, CDCl₃) δ 1.27(d, *J*=10Hz, 3H), 3.12(d, *J*=5Hz, 1H), 4.37(d, *J*=6Hz, 2H), 5.12∼5.19(m, 3H), 7.15∼7.56(m, 9H)

### Example 21: Synthesis of 1-(2-chlorophenyl)-(S)-1-hydroxypropyl-(S)-2-N-bicyclo[2,2,1]heptanescarbamate(21)

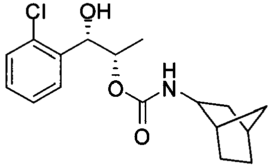

The substantially same method as described in Example 1 was conducted, except that 2-aminonorbornane was used instead of ammonia solution(NH₄OH), to obtain the title compound (1.7g, yield 32%).
¹H NMR(400MHz, CDCl₃) δ1.08∼1.35(m, 9H), 1.65(br s, 1H), 1.75∼1.71(m, 1H), 2.14∼2.24(m, 1H), 2.27∼2.30(m, 1H), 3.23∼3.29(m, 1H), 3.47∼3.52(m, 1H), 4.67(br s, 1H), 5.01∼5.09(m, 1H), 5.12∼5.18(m, 1H), 7.22∼7.55(m, 4H)

### Example 22: Synthesis of 1-(2-chlorophenyl)-(R)-1-hydroxypropyl-(R)-2-N-methylcarbamate(22)

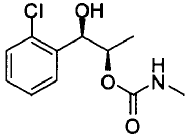

The substantially same method as described in Example 2 was conducted, except that methylamine was used instead of ammonia solution(NH₄OH), to obtain the title compound (3.36g, yield 60%).
¹H NMR(400MHz, CDCl₃) δ 1.20(d, *J*=6.8Hz, 3H), 2.80(d, *J*=4.8Hz, 3H), 3.20(d, *J*=4.4Hz, 1H), 4.75(br s, 1H), 5.03∼5.09(m, 1H), 5.14∼5.17(m, 1H), 7.22∼7.55(m, 4H)

### Example 23: Synthesis of 1-(2-chlorophenyl)-(R)-1-hydroxypropyl-(R)-2-N-propylcarbamate(21)

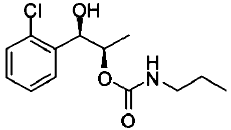

The substantially same method as described in Example 2 was conducted, except that propylamine was used instead of ammonia solution(NH₄OH), to obtain the title compound (3.1g, yield 53%).
¹H NMR(400MHz, CDCl₃) δ0.92(t, *J*=7.6Hz, 3H), 1.21(d, *J*=6.4Hz, 3H), 1.51(m, 2H), 3.09∼3.14(m, 2H), 3.28(d, *J*=4.4Hz, 1H), 4.82(br s, 1H), 5.03∼5.09(m, 1H), 5.14∼5.17(m, 1H), 7.22∼7.55(m. 4H)

### Example 24: Synthesis of 1-(2-chlorophenyl)-(R)-1-hydroxypropyl-(R)-2-N-isopropylcarbamate(24)

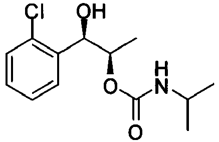

The substantially same method as described in Example 2 was conducted, except that isopropylamine was used instead of ammonia solution(NH₄OH), to obtain the title compound (0.16g, yield 27%).
¹H NMR(400MHz, CDCl₃) δ0.88∼1.16(m, 6H), 1.19∼1.26(m, 3H), 3.34(s, 1H), 3.71∼3.78(m, 1H), 4.62(br s, 1H), 5.03(t, *J*=5.8Hz, 1H), 5.13(d, *J*=4.9Hz, 1H), 7.20∼7.53(m, 4H)

### Example 25: Synthesis of 1-(2-chlorophenyl)-(R)-1-hydroxypropyl-(R)-2-N-cyclopropylcarbamate(25)

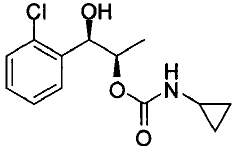

The substantially same method as described in Example 2 was conducted, except that cyclopropylamine was used instead of ammonia solution(NH₄OH), to obtain the title compound (3.7g, yield 60%).
¹H NMR(400MHz, CDCl₃) 80.49∼0.54(m, 2H), 0.74(d, J=7.2Hz, 2H), 1.22(s, 3H), 2.55∼2.60(m, 1H), 3.16(s, 1H), 5.00(s, 1H), 5.04∼5.11(m, 1H), 5.16(s, 1H), 7.23∼7.54(m, 4H)

### Example 26: Synthesis of 1-(2-chlorophenyl)-(R)-1-hydroxypropyl-(R)-2-N-cyclohexyl carbamate(26)

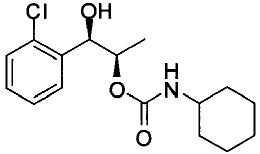

The substantially same method as described in Example 2 was conducted, except that cyclohexylamine was used instead of ammonia solution(NH₄OH), to obtain the title compound (1.9g, yield 28%).
¹H NMR(400MHz, CDCl₃) δ1.05∼1.38(m, 8H), 1.58∼1.70(m, 3H), 1.85∼1.95(m, 2H), 3.39∼3.47(m, 1H), 3.56(s, 1H), 4.79(br s, 1H), 5.01∼5.07(m, 1H), 5.14(t, *J*=5.2Hz, 1H), 7.20-7.54(m, 4H)

### Example 27: Synthesis of 1-(2-chlorophenyl)-(R)-1-hydroxypropyl-(R)-2-N-benzylcarbamate(27)

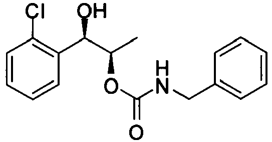

The substantially same method as described in Example 2 was conducted, except that benzylamine was used instead of ammonia solution(NH₄OH), to obtain the title compound (0.52g, yield 19%).
¹H NMR(400MHz, CDCl₃) δ1.25(d, *J*=6Hz, 3H), 1.64(s, 1H), 3.13(d, *J*=4.4Hz, 1H), 4.37(d, *J*=5.6Hz, 2H), 5.12∼5.19(m, 2H), 7.23∼7.55(m, 9H)

### Example 28: Synthesis of 1-(2-chlorophenyl)-(R)-1-hydroxypropyl-(R)-2-N-bicyclo[2,2,1]heptanecarbamate(28)

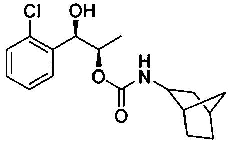

The substantially same method as described in Example 2 was conducted, except that 2-aminonorbornane was used instead of ammonia solution(NH₄OH), to obtain the title compound (1.7g, yield 20∼50%).
¹H NMR(400MHz, CDCl₃) δ1.08∼1.35(m, 9H), 1.65(br s, 1H), 1.75∼1.71(m, 1H), 2.14∼2.24(m, 1H), 2.27∼2.30(m, 1H), 3.23∼3.29(m, 1H), 3.47∼3.52(m, 1H), 4.67(br s, 1H), 5.01∼5.09(m, 1H), 5.12∼5.18(m, 1H), 7.22∼7.55(m, 4H)

### Example 29: Synthesis of 1-(2-chlorophenyl)-1-hydroxypropyl-2-N-methylcarbamate(29)

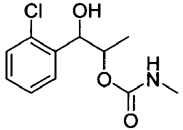

The substantially same method as described in Example 3 was conducted, except that methylamine was used instead of ammonia solution(NH₄OH), to obtain the title compound (2.6g, yield 45%).
¹H NMR(400MHz, CDCl₃) δ 1.21(d, *J*=6Hz, 3H), 2.81(d, *J*=5Hz, 3H), 3.14(d, J=4Hz, 1H), 4.72(br s, 1H), 5.07(dd, *J*=6Hz, 1H), 5.16(t, *J*=6Hz, 1H), 7.22∼7.56(m, 4H)

### Example 30: Synthesis of 1-(2-chlorophenyl)-1-hydroxypropyl-2-N-propylcarbamate(30)

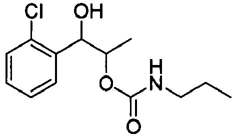

The substantially same method as described in Example 3 was conducted, except that propylamine was used instead of ammonia solution(NH₄OH), to obtain the title compound (1.0g, yield 17%).
¹H NMR(400MHz, CDCl₃) δ 0.92(t, *J*=7Hz, 3H), 1.21(d, *J*=6Hz, 3H), 1.53(dd, *J*=7Hz, 2H), 3.13(dd, *J*=7Hz, 2H), 3.28(d, 1H), 4.82(S, 1H), 5.06(dd, *J*=7Hz, 1H), 5.16(t, *J*=5Hz, 1H), 7.21∼7.56(m, 4H)

### Example 31: Synthesis of 1-(2-chlorophenyl)-1-hydroxypropyl-2-N-isopropylcarbamate(31)

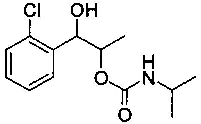

The substantially same method as described in Example 3 was conducted, except that isopropylamine was used instead of ammonia solution(NH₄OH), to obtain the title compound (0.54g, yield 16%).
¹H NMR(400MHz, CDCl₃) δ 1.16(dd, *J*=6Hz, 6H), 1.21(d, *J*=6Hz, 3H), 3.23(d, *J*=6Hz, 1H), 3.75∼3.84(m, 1H), 4.61(br s, 1H), 5.06(t, *J*=6Hz, 1H), 5.16(t, *J*=6Hz, 1H), 7.22∼7.56(m, 4H)

### Example 32: Synthesis of 1-(2-chlorophenyl)-1-hydroxypropyl-2-N-cyclopropylcarbamate(32)

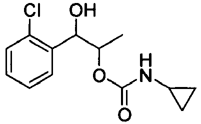

The substantially same method as described in Example 3 was conducted, except that cyclopropylamine was used instead of ammonia solution(NH₄OH), to obtain the title compound (1.0g, yield 17%).
¹H NMR(400MHz, CDCl₃) δ 0.50(t, *J*=6Hz, 2H), 0.77(t, *J*=3Hz, 2H), 1.12(d, *J*=7Hz, 3H), 2.53∼2.59(m, 1H), 3.22(d, *J*=4Hz, 1H), 5.08(dd, *J*=6Hz, 1H), 5.15(S, 1H), 7.22∼7.55(m, 4H)

### Example 33: Synthesis of 1-(2-chlorophenyl)-1-hydroxypropyl-2-N-cyclohexylcarbamate(33)

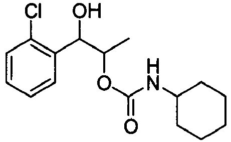

The substantially same method as described in Example 3 was conducted, except that cyclohexylamine was used instead of ammonia solution(NH₄OH), to obtain the title compound (2.2g, yield 33%).
¹H NMR(400MHz, CDCl₃) δ 1.07∼1.17(m, 3H), 1.21(d, *J*=6Hz, 3H), 1.29∼1.42(m, 3H), 1.72(dd, *J*=6Hz, 2H), 1.92(dd, *J*=6Hz, 2H), 3.26(d, *J*=4Hz, 1H), 3.46(t, *J*=4Hz, 1H), 4.68(d, *J*=6Hz, 1H), 5.07(dd, *J*=6Hz, 1H), 5.16(t, *J*=6Hz, 1H), 7.22∼7.55(m, 4H)

### Example 34: Synthesis of 1-(2-chlorophenyl)-1-hydroxypropyl-2-N-benzylcarbamate(34)

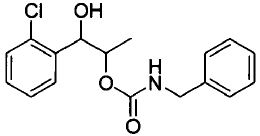

The substantially same method as described in Example 3 was conducted, except that benzylamine was used instead of ammonia solution(NH₄OH), to obtain the title compound (1.3g, yield 19%).
¹H NMR(400MHz, CDCl₃) δ 1.25(d, *J*=6Hz, 3H), 3.16(d, *J*=4Hz, 1H), 4.36(d, *J*=6Hz, 2H), 5.14(dd, *J*=6Hz, 3H), 7.23∼7.56(m, 9H), yield: 19%(1.3g)

### Example 35: Synthesis of 1-(2-chlorophenyl)-1-hydroxypropyl-2-N-bicyclo[2,2,1]heptanecarbamate(35)

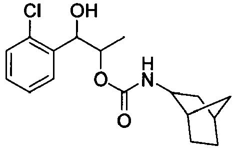

The substantially same method as described in Example 3 was conducted, except that 2-aminonorbornane was used instead of ammonia solution(NH₄OH), to obtain the title compound (1.7g, yield 20-50%).
¹H NMR(400MHz, CDCl₃) δ1.08∼1.35(m, 9H), 1.65(br s, 1H), 1.75∼1.71(m, 1H), 2.14∼2.24(m, 1H), 2.27∼2.30(m, 1H), 3.23∼3.29(m, 1H), 3.47∼3.52(m, 1H), 4.67(br s, 1H), 5.01∼5.09(m, 1H), 5.12∼5.18(m, 1H), 7.22∼7.55(m, 4H)

### Example 36: Synthesis of 1-(2,4-dichlorophenyl)-(S)-1-hydroxypropyl-(S)-2-carbamate(36)

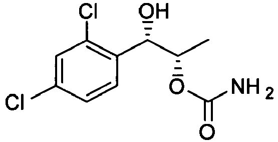

The substantially same method as described in Example 1 was conducted, except that 1-(2,4-dichlorophenyl)-(S,S)-1,2-propanediol obtained in Preparation Example 26 was used instead of 1-(2-chlorophenyl)-(S,S)-1,2-propanediol, to obtain the title compound (0.14g, yield 34%).
¹H NMR(400MHz, CDCl₃) δ1.22(d, *J* = 6.4Hz, 3H), 4.16(br t, 1H) 4.96(br t, 3H), 5.07(t, *J*= 4.8Hz, 1H), 7.23∼7.52(m, 3H)

### Example 37: Synthesis of 1-(2,6-dichlorophenyl)-(S)-1-hydroxypropyl-(S)-2-carbamate(37)

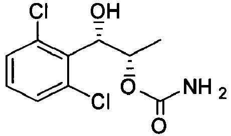

The substantially same method as described in Example 1 was conducted, except that 1-(2,6-dichlorophenyl)-(S,S)-1,2-propanediol obtained in Preparation Example 38 was used instead of 1-(2-chlorophenyl)-(S,S)-1,2-propanediol, to obtain the title compound (0.22g, yield 49%).
¹H NMR(400MHz, CDCl₃) δ1.15(d, *J* = 6.4Hz, 3H), 3.66(d, *J* = 9.2Hz, 1H), 4.73(br s, 2H), 5.43(t, *J*= 9.0Hz, 1H), 5.62∼5.69(m, 1H), 7.18∼7.22(m, 3H),

### Example 38: Synthesis of 1-(2,3-dichlorophenyl)-(S)-1-hydroxypropyl-(S)-2-carbamate(38)

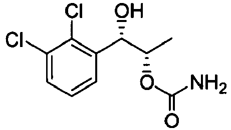

The substantially same method as described in Example 1 was conducted, except that 1-(2,3-dichlorophenyl)-(S,S)-1,2-propanediol obtained in Preparation Example 57 was used instead of 1-(2-chlorophenyl)-(S,S)-1,2-propanediol, to obtain the title compound (0.21g, yield 20∼60%).
¹H NMR(400MHz, CDCl₃) δ1.15(d, *J* = 6.4Hz, 3H), 3.66(d, *J* = 9.2Hz, 1H), 4.73(br s, 2H), 5.43(t, *J*= 9.0Hz, 1H), 5.62∼5.69(m, 1H), 7.18∼7.22(m, 3H),

### Example 39: Synthesis of 1-(2,4-dichlorophenyl)-(S)-1-hydroxybutyl-(S)-2-carbamate(39)

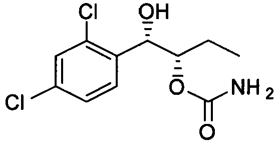

The substantially same method as described in Example 1 was conducted, except that 1-(2,4-dichlorophenyl)-(S,S)-1,2-butanediol obtained in Preparation Example 29 was used instead of 1-(2-chlorophenyl)-(S,S)-1,2-propanediol, to obtain the title compound (0.23g, yield 52%).
¹H NMR(400MHz, CDCl₃) δ0.96(t, *J* = 7.4Hz, 3H), 1.58∼1.74(m, 2H), 2.98(d, *J* = 5.6Hz, 1H) 4.68(br s, 2H), 5.59(dt, *J* = 5.2, 8.8Hz, 1H), 5.19(t, *J* = 5.4Hz, 1H), 7.30∼7.50(m, 3H)

### Example 40: Synthesis of 1-(2,6-dichlorophenyl)-(S)-1-hydroxybutyl-(S)-2-carbamate(40)

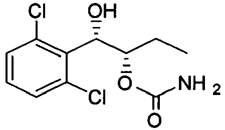

The substantially same method as described in Example 1 was conducted, except that 1-(2,6-dichlorophenyl)-(S,S)-1,2-butanediol obtained in Preparation Example 41 was used instead of 1-(2-chlorophenyl)-(S,S)-1,2-propanediol, to obtain the title compound (0.49g, yield 34%).
¹H NMR(400MHz, CDCl₃) δ0.92(t, *J* = 7.4Hz, 3H), 1.30∼1.38(m, 1H), 1.57∼1.64(m, 1H), 3.74(d, *J* = 9.2Hz, 1H), 4.80(br s, 2H), 5.40∼5.50(m, 2H), 7.17∼7.34(m, 3H)

### Example 41: Synthesis of 1-(2,4-dichlorophenyl)-(S)-1-hydroxy-3-methylbutyl-(S)-2-carbamate(41)

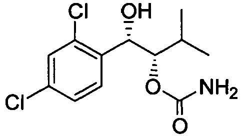

The substantially same method as described in Example 1 was conducted, except that 1-(2,4-dichlorophenyl)-3-methyl-(S,S)-1,2-butanediol obtained in Preparation Example 32 was used instead of 1-(2-chlorophenyl)-(S,S)-1,2-propanediol, to obtain the title compound (0.13g, yield 20∼60%).
¹H NMR(400MHz, CDCl₃) δ1.00(t, *J* = 7.2Hz, 6H), 1.73∼1.79(m, 1H), 3.673∼3.69(m, 1H), 4.85(br s, 2H), 5.40∼5.43(m, 1H), 5.49∼5.54(m, 1H), 7.30∼7.50(m, 3H)

### Example 42: Synthesis of 1-(2,6-dichlorophenyl)-(S)-1-hydroxy-3-methylbutyl-(S)-2-carbamate(42)

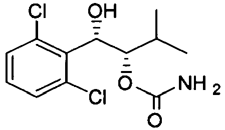

The substantially same method as described in Example 1 was conducted, except that 1-(2,6-dichlorophenyl)-3-methyl-(S,S)-1,2-butanediol obtained in Preparation Example 44 was used instead of 1-(2-chlorophenyl)-(S,S)-1,2-propanediol, to obtain the title compound (0.12g, yield 20%).
¹H NMR(400MHz, CDCl₃) δ1.00(t, *J* = 7.2Hz, 6H), 1.73∼1.79(m, 1H), 3.673∼3.69(m, 1H), 4.85(br s, 2H), 5.40∼5.43(m, 1H), 5.49∼5.54(m, 1H), 7.16∼7.33(m, 3H)

### Example 43: Synthesis of 1-(2,4-dichlorophenyl)-(S)-1-hydroxyhexyl-(S)-2-carbamate(43)

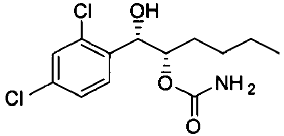

The substantially same method as described in Example 1 was conducted, except that 1-(2,4-dichlorophenyl)-(S,S)-1,2-hexanediol obtained in Preparation Example 35 was used instead of 1-(2-chlorophenyl)-(S,S)-1,2-propanediol, to obtain the title compound (0.94g, yield 81%).
¹H NMR(400MHz, CDCl₃) δ0.89(t, *J* = 3.6Hz, 3H), 1.28∼1.42(m, 4H), 1.52∼1.59(m, 1H), 1.64∼1.71(m, 1H), 2.98(d, *J* = 5.6Hz, 1H), 4.67(br s, 2H), 4.96∼5.00(m, 1H), 5.17(t, *J* = 5.6Hz, 1H), 7.30∼7.49(m 3H)

### Example 44: Synthesis of 1-(2,6-dichlorophenyl)-(S)-1-hydroxyhexyl-(S)-2-carbamate(44)

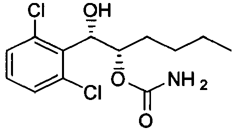

The substantially same method as described in Example 1 was conducted, except that 1-(2,6-dichlorophenyl)-(S,S)-1,2-hexanediol obtained in Preparation Example 47 was used instead of 1-(2-chlorophenyl)-(S,S)-1,2-propanediol, to obtain the title compound (0.15g, yield 31%).
¹H NMR(400MHz, CDCl₃) δ0.84(t, *J* = 7.0Hz, 3H), 1.20∼1.35(m, 4H), 1.36∼1.41(m, 1H), 1.59∼1.63(m, 1H), 3.71(d, *J* = 10.0Hz, 1H), 4.74(br s, 2H), 5.40∼5.44(m, 1H), 5.52∼5.57(m, 1H), 7.17∼7.35(m, 3H)

### Example 45: Synthesis of 1-(2,4-dichlorophenyl)-(R)-1-hydroxypropyl-(R)-2-carbamate(45)

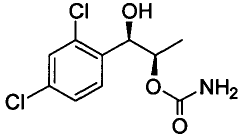

The substantially same method as described in Example 1 was conducted, except that 1-(2,4-dichlorophenyl)-(R,R)-1,2-propanediol obtained in Preparation Example 27 was used instead of 1-(2-chlorophenyl)-(S,S)-1,2-propanediol, to obtain the title compound (0.14g, yield 20∼60%).
¹H NMR(400MHz, CDCl₃) δ1.22(d, *J* = 6.4Hz, 3H), 4.16(br t, 1H) 4.96(br t, 3H), 5.07(t, *J* = 4.8Hz, 1H), 7.23∼7.52(m, 3H)

### Example 46: Synthesis of 1-(2,6-dichlorophenyl)-(R)-1-hydroxypropyl-(R)-2-carbamate(46)

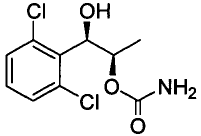

The substantially same method as described in Example 1 was conducted, except that 1-(2,6-dichlorophenyl)-(R,R)-1,2-propanediol obtained in Preparation Example 39 was used instead of 1-(2-chlorophenyl)-(S,S)-1,2-propanediol, to obtain the title compound (0.21g, yield 20∼60%).
¹H NMR(400MHz, CDCl₃) δ1.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 47: Synthesis of 1-(2,3-dichlorophenyl)-(R)-1-hydroxypropyl-(R)-2-carbamate(47)

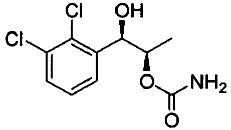

The substantially same method as described in Example 1 was conducted, except that 1-(2,3-dichlorophenyl)-(R,R)-1,2-propanediol obtained in Preparation Example 58 was used instead of 1-(2-chlorophenyl)-(S,S)-1,2-propanediol, to obtain the title compound (0.08g, yield 20∼60%).
¹H NMR(400MHz, CDCl₃) δ1.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 48: Synthesis of 1-(2,4-dichlorophenyl)-(R)-1-hydroxybutyl-(R)-2-carbamate(48)

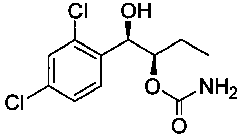

The substantially same method as described in Example 1 was conducted, except that 1-(2,4-dichlorophenyl)-(R,R)-1,2-butanediol obtained in Preparation Example 30 was used instead of 1-(2-chlorophenyl)-(S,S)-1,2-propanediol, to obtain the title compound (0.23g, yield 20-60%).
¹H NMR(400MHz, CDCl₃) δ0.96(t, *J* = 7.4Hz, 3H), 1.58∼1.74(m, 2H), 2.98(d, *J* = 5.6Hz, 1H) 4.68(br s, 2H), 5.59(dt, *J* = 5.2, 8.8Hz, 1H), 5.19(t, *J* = 5.4Hz, 1H), 7.30∼7.50(m, 3H)

### Example 49: Synthesis of 1-(2,6-dichlorophenyl)-(R)-1-hydroxybutyl-(R)-2-carbamate(49)

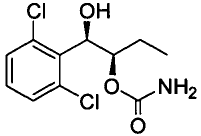

The substantially same method as described in Example 1 was conducted, except that 1-(2,6-dichlorophenyl)-(R,R)-1,2-butanediol obtained in Preparation Example 42 was used instead of 1-(2-chlorophenyl)-(S,S)-1,2-propanediol, to obtain the title compound (0.49g, yield 20∼60%).
¹H NMR(400MHz, CDCl₃) δ0.92(t, *J* = 7.4Hz, 3H), 1.30∼1.38(m, 1H), 1.57∼1.64(m, 1H), 3.74(d, *J* = 9.2Hz, 1H), 4.80(br s, 2H), 5.40∼5.50(m, 2H), 7.17∼7.34(m, 3H)

### Example 50: Synthesis of 1-(2,4-dichlorophenyl)-(R)-1-hydroxy-3-methylbutyl-(R)-2-carbamate(50)

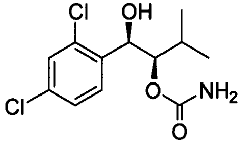

The substantially same method as described in Example 1 was conducted, except that 1-(2,4-dichlorophenyl)-3-methyl-(R,R)-1,2-butanediol obtained in Preparation Example 33 was used instead of 1-(2-chlorophenyl)-(S,S)-1,2-propanediol, to obtain the title compound (0.23g, yield 20∼60%).
¹H NMR(400MHz, CDCl₃) δ1.00(t, *J* = 7.2Hz, 6H), 1.73∼1.79(m, 1H), 3.673∼3.69(m, 1H), 4.85(br s, 2H), 5.40∼5.43(m, 1H), 5.49∼5.54(m, 1H), 7.30∼7.50(m, 3H)

### Example 51: Synthesis of 1-(2,6-dichlorophenyl)-(R)-1-hydroxy-3-methylbutyl-(R)-2-carbamate(51)

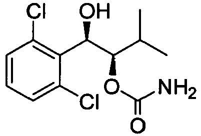

The substantially same method as described in Example 1 was conducted, except that 1-(2,6-dichlorophenyl)-3-methyl-(R,R)-1,2-butanediol obtained in Preparation Example 45 was used instead of 1-(2-chlorophenyl)-(S,S)-1,2-propanediol, to obtain the title compound (0.14g, yield 20∼60%).
¹H NMR(400MHz, CDCl₃) δ1.00(t, *J* = 7.2Hz, 6H), 1.73∼1.79(m, 1H), 3.673∼3.69(m, 1H), 4.85(br s, 2H), 5.40∼5.43(m, 1H), 5.49∼5.54(m, 1H), 7.16∼7.33(m, 3H)

### Example 52: Synthesis of 1-(2,4-dichlorophenyl)-(R)-1-hydroxyhexyl-(R)-2-carbamate(52)

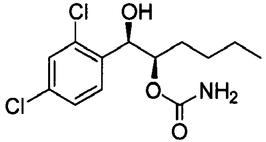

The substantially same method as described in Example 1 was conducted, except that 1-(2,4-dichlorophenyl)-(R,R)-1,2-hexanediol obtained in Preparation Example 36 was used instead of 1-(2-chlorophenyl)-(S,S)-1,2-propanediol, to obtain the title compound (0.84g, yield 20∼60%).
¹H NMR(400MHz, CDCl₃) δ0.89(t, *J* = 3.6Hz, 3H), 1.28∼1.42(m, 4H), 1.52∼1.59(m, 1H), 1.64∼1.71(m, 1H), 2.98(d, *J* = 5.6Hz, 1H), 4.67(br s, 2H), 4.96∼5.00(m, 1H), 5.17(t, *J* = 5.6Hz, 1H), 7.30∼7.49(m, 3H)

### Example 53: Synthesis of 1-(2,6-dichlorophenyl)-(R)-1-hydroxyhexyl-(R)-2-carbamate(53)

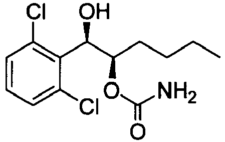

The substantially same method as described in Example 1 was conducted, except that 1-(2,6-dichlorophenyl)-(R,R)-1,2-hexanediol obtained in Preparation Example 48 was used instead of 1-(2-chlorophenyl)-(S,S)-1,2-propanediol, to obtain the title compound (0.15g, yield 20∼60%).
¹H NMR(400MHz, CDCl₃) δ0.84(t, *J* = 7.0Hz, 3H), 1.20∼1.35(m, 4H), 1.36∼1.41(m, 1H), 1.59∼1.63(m, 1H), 3.71(d, *J* = 10.0Hz, 1H), 4.74(br s, 2H), 5.40∼5.44(m, 1H), 5.52∼5.57(m, 1H), 7.17∼7.35(m, 3H)

### Example 54: Synthesis of 1-(2,4-dichlorophenyl)-1-hydroxypropyl-2-carbamate(54)

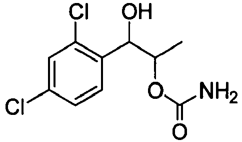

The substantially same method as described in Example 1 was conducted, except that 1-(2,4-dichlorophenyl)-1,2-propanediol obtained in Preparation Example 28 was used instead of 1-(2-chlorophenyl)-(S,S)-1,2-propanediol, to obtain the title compound (0.14g, yield 20∼60%).
¹H NMR(400MHz, CDCl₃) δ1.22(d, *J* = 6.4Hz, 3H), 4.16(br t, 1H) 4.96(br t, 3H), 5.07(t, *J* = 4.8Hz, 1H), 7.23∼7.52(m, 3H)

### Example 55: Synthesis of 1-(2,6-dichlorophenyl)-1-hydroxypropyl-2-carbamate(55)

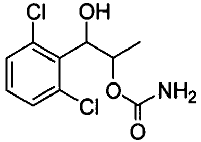

The substantially same method as described in Example 1 was conducted, except that 1-(2,6-dichlorophenyl)-1,2-propanediol obtained in Preparation Example 40 was used instead of 1-(2-chlorophenyl)-(S,S)-1,2-propanediol, to obtain the title compound (0.19g, yield 20∼60%).
¹H NMR(400MHz, CDCl₃) 81.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 56: Synthesis of 1-(2,3-dichlorophenyl)-1-hydroxypropyl-2-carbamate(56)

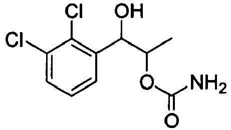

The substantially same method as described in Example 1 was conducted, except that 1-(2,3-dichlorophenyl)-1,2-propanediol obtained in Preparation Example 59 was used instead of 1-(2-chlorophenyl)-(S,S)-1,2-propanediol, to obtain the title compound (0.21g, yield 20∼60%).
¹H NMR(400MHz, CDCl₃) 81.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 57: Synthesis of 1-(2,4-dichlorophenyl)-1-hydroxybutyl-2-carbamate(57)

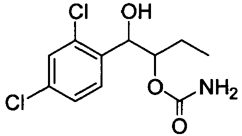

The substantially same method as described in Example 1 was conducted, except that 1-(2,4-dichlorophenyl)-1,2-butanediol obtained in Preparation Example 31 was used instead of 1-(2-chlorophenyl)-(S,S)-1,2-propanediol, to obtain the title compound (0.23g, yield 20∼60%).
¹H NMR(400MHz, CDCl₃) 80.96(t, *J* = 7.4Hz, 3H), 1.58∼1.74(m, 2H), 2.98(d, *J* = 5.6Hz, 1H) 4.68(br s, 2H), 5.59(dt, *J* = 5.2, 8.8Hz, 1H), 5.19(t, *J* = 5.4Hz, 1H), 7.30∼7.50(m, 3H)

### Example 58: Synthesis of 1-(2,6-dichlorophenyl)-1-hydroxybutyl-2-carbamate(58)

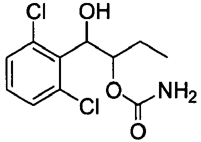

The substantially same method as described in Example 1 was conducted, except that 1-(2,6-dichlorophenyl)-1,2-butanediol obtained in Preparation Example 43 was used instead of 1-(2-chlorophenyl)-(S,S)-1,2-propanediol, to obtain the title compound (0.49g, yield 20∼60%).
¹H NMR(400MHz, CDCl₃) δ0.92(t, *J* = 7.4Hz, 3H), 1.30∼1.38(m, 1H), 1.57∼1.64(m, 1H), 3.74(d, *J* = 9.2Hz, 1H), 4.80(br s, 2H), 5.40∼5.50(m, 2H), 7.17∼7.34(m, 3H)

### Example 59: Synthesis of 1-(2,4-dichlorophenyl)-1-hydroxy-3-methylbutyl-2-carbamate(59)

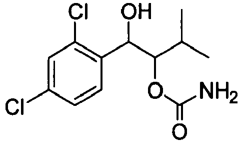

The substantially same method as described in Example 1 was conducted, except that 1-(2,4-dichlorophenyl)-3-methyl-1,2-butanediol obtained in Preparation Example 34 was used instead of 1-(2-chlorophenyl)-(S,S)-1,2-propanediol, to obtain the title compound (0.13g, yield 20∼60%).
¹H NMR(400MHz, CDCl₃) δ1.00(t, *J* = 7.2Hz, 6H), 1.73∼1.79(m, 1H), 3.67∼3.69(m, 1H), 4.85(br s, 2H), 5.40∼5.43(m, 1H), 5.49∼5.54(m, 1H), 7.30∼7.50(m, 3H)

### Example 60: Synthesis of 1-(2,6-dichlorophenyl)-1-hydroxy-3-methylbutyl-2-carbamate(60)

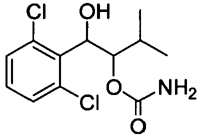

The substantially same method as described in Example 1 was conducted, except that 1-(2,6-dichlorophenyl)-3-methyl-1,2-butanediol obtained in Preparation Example 46 was used instead of 1-(2-chlorophenyl)-(S,S)-1,2-propanediol, to obtain the title compound (0.13g, yield 20∼60%).
¹H NMR(400MHz, CDCl₃) δ1.00(t, *J* = 7.2Hz, 6H), 1.73∼1.79(m, 1H), 3.67∼3.69(m, 1H), 4.85(br s, 2H), 5.40∼5.43(m, 1H), 5.49∼5.54(m, 1H), 7.16∼7.33(m, 3H)

### Example 61: Synthesis of 1-(2,4-dichlorophenyl)-1-hydroxyhexyl-2-carbamate(61)

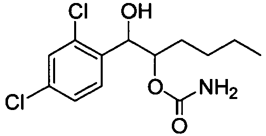

The substantially same method as described in Example 1 was conducted, except that 1-(2,4-dichlorophenyl)-1,2-hexanediol obtained in Preparation Example 37 was used instead of 1-(2-chlorophenyl)-(S,S)-1,2-propanediol, to obtain the title compound (0.94g, yield 20∼60%).
¹H NMR(400MHz, CDCl₃) δ0.89(t, *J* = 3.6Hz, 3H), 1.28∼1.42(m, 4H), 1.52∼1.59(m, 1H), 1.64∼1.71(m, 1H), 2.98(d, *J* = 5.6Hz, 1H), 4.67(br s, 2H), 4.96∼5.00(m, 1H), 5.17(t, *J* = 5.6Hz, 1H), 7.30∼7.49(m, 3H)

### Example 62: Synthesis of 1-(2,6-dichlorophenyl)-1-hydroxyhexyl-2-carbamate(62)

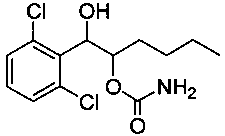

The substantially same method as described in Example 1 was conducted, except that 1-(2,6-dichlorophenyl)-1,2-hexanediol obtained in Preparation Example 49 was used instead of 1-(2-chlorophenyl)-(S,S)-1,2-propanediol, to obtain the title compound (0.15g, yield 20∼60%).
¹H NMR(400MHz, CDCl₃) δ0.84(t, *J* = 7.0Hz, 3H), 1.20∼1.35(m, 4H), 1.36∼1.41(m, 1H), 1.59∼1.63(m, 1H), 3.71(d, *J* = 10.0Hz, 1H), 4.74(br s, 2H), 5.40∼5.44(m, 1H), 5.52∼5.57(m, 1H), 7.17∼7.35(m, 3H)

### Example 63: Synthesis of 1-(2-fluorophenyl)-(S)-1-hydroxypropyl-(S)-2-carbamate(63)

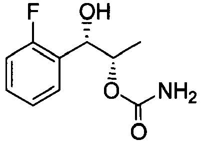

The substantially same method as described in Example 1 was conducted, except that 1-(2-fluorophenyl)-(S,S)-1,2-propanediol(12.23g) obtained in Preparation Example 61 was used instead of 1-(2-chlorophenyl)-(S,S)-1,2-propanediol, to obtain the title compound (6.11g, yield 40%).
¹H NMR(400MHz, CDCl₃) δ1.19(d, *J*=5.2Hz, 3H), 2.93(d, *J*=4.4Hz, 1H), 4.71(br s, 2H), 4.99∼5.06(m, H), 7.04∼7.48(m, 4H)

### Example 64: Synthesis of 1-(2-fluorophenyl)-(R)-1-hydroxypropyl-(R)-2-carbamate(64)

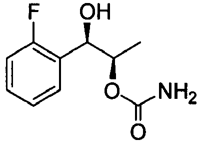

The substantially same method as described in Example 1 was conducted, except that 1-(2-fluorophenyl)-(R,R)-1,2-propanediol(6.26g) obtained in Preparation Example 62 was used instead of 1-(2-chlorophenyl)-(S,S)-1,2-propanediol, to obtain the title compound (3.13g, yield 40%).
¹H NMR(400MHz, CDCl₃) δ1.19(d, *J*=5.2Hz, 3H), 2.93(d, *J*=4.4Hz, 1H), 4.71(br s, 2H), 4.99∼5.06(m, H), 7.04∼7.48(m, 4H)

### Example 65: Synthesis of 1-(2-iodophenyl)-(S)-1-hydroxypropyl-(S)-2-carbamate(65)

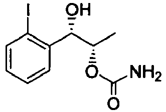

The substantially same method as described in Example 1 was conducted, except that 1-(2-iodophenyl)-(S,S)-1,2-propanediol obtained in Preparation Example 66 was used instead of 1-(2-chlorophenyl)-(S,S)-1,2-propanediol, to obtain the title compound (2.2g, yield 30∼60%).
¹H NMR(400MHz, CDCl₃) δ1.27(d, *J*=6.4Hz, 3H), 3.09(br s, 1H), 4.83(br s, 2H), 5.00∼5.10(M, 2H), 7.00∼7.76(m, 4H)

### Example 66: Synthesis of 1-(2-iodophenyl)-(R)-1-hydroxypropyl-(R)-2-carbamate(66)

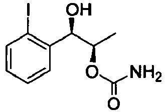

The substantially same method as described in Example 1 was conducted, except that 1-(2-iodophenyl)-(R,R)-1,2-propanediol obtained in Preparation Example 67 was used instead of 1-(2-chlorophenyl)-(S,S)-1,2-propanediol, to obtain the title compound (3.13g, yield 30∼60%).
¹H NMR(400MHz, CDCl₃) δ1.27(d, *J*=6.4Hz, 3H), 2.95(d, *J*=3.6Hz, 1H), 4.73(br s, 2H), 5.01∼5.11(m, 2H), 7.01∼7.86(m, 4H)

### Example 67: Synthesis of 1-(2-iodophenyl)-(S)-1-hydroxybutyl-(S)-2-carbamate(67)

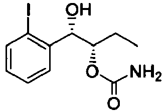

The substantially same method as described in Example 1 was conducted, except that 1-(2-iodophenyl)-(S,S)-1,2-butanediol obtained in Preparation Example 68 was used instead of 1-(2-chlorophenyl)-(S,S)-1,2-propanediol, to obtain the title compound (3.6g, yield 30∼60%).
¹H NMR(400MHz, CDCl₃) δ1.27(d, *J*=6.4Hz, 3H), 3.09(br s, 1H), 4.83(br s, 2H), 5.00∼5.10(m, 2H), 7.00∼7.76(m, 4H)

### Example 68: Synthesis of 1-(2-chlorophenyl)-(S)-2-hydroxypropyl-(S)-1-carbamate(68)

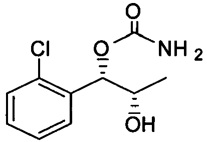

A regioisomer of monocarbamate was separated and purified by conducting the silica gel column chromatography as described in Example 1, to obtain the title compound (0.34g, yield 10%).
¹H NMR(400MHz, CDCl₃) δ1.24(d, *J* = 6.8Hz, 3H), 2.13(d, *J* = 4.4Hz, 1H), 4.12∼4.16(m, 1H), 4.85(br s, 2H), 5.98(d, *J* = 5.6Hz, 1H), 7.24∼7.43(m, 4H)

### Example 69: Synthesis of 1-(2-chlorophenyl)-(R)-2-hydroxypropyl-(R)-1-carbamate(69)

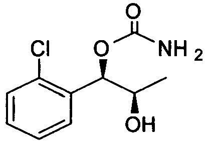

A regioisomer of monocarbamate was separated and purified by conducting the silica gel column chromatography as described in Example 2, to obtain the title compound (0.77g, yield 16%).
¹H NMR(400MHz, CDCl₃) δ1.24(d, *J* = 6.4Hz, 3H), 2.04(d, *J* = 4.8Hz, 1H), 4.11∼4.18(m, 1H), 4.74(br s, 2H), 6.00(d, *J* = 5.6Hz, 1H), 7.24∼7.43(m, 4H)

### Example 70: Synthesis of 1-(2-chlorophenyl)-2-hydroxypropyl-1-carbamate(70)

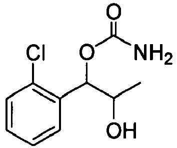

A regioisomer of monocarbamate was separated and purified by conducting the silica gel column chromatography as described in Example 3, to obtain the title compound (0.16g, yield 10∼30%).
¹H NMR(400MHz, CDCl₃) δ1.24(d, *J* = 6.4Hz, 3H), 2.04(d, *J* = 4.8Hz, 1H), 4.11∼4.18(m, 1H), 4.74(br s, 2H), 6.00(d, *J* = 5.6Hz, 1H), 7.24∼7.43(m, 4H)

### Example 71: Synthesis of 1-(2-chlorophenyl)-(S)-2-hydroxypropyl-(S)-1-N-methylcarbamate(71)

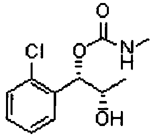

A regioisomer of monocarbamate was separated and purified by conducting the silica gel column chromatography as described in Example 15, to obtain the title compound (0.70g, yield 10∼30%).
¹H NMR(400MHz, CDCl₃) δ1.21(d, *J*=6.4Hz, 3H), 2.80(d, *J*=4.8Hz, 3H), 3.12(s, 1H), 4.09-4.16(m, 1H), 4.86(br s, 1H), 5.99(d, *J*= 6.0Hz, 1H), 7.23∼7.40(m, 4H)

### Example 72: Synthesis of 1-(2-chlorophenyl)-(R)-2-hydroxypropyl-(R)-1-N-methylcarbamate(72)

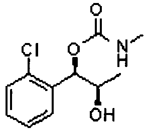

A regioisomer of monocarbamate was separated and purified by conducting the silica gel column chromatography as described in Example 22, to obtain the title compound (0.69g, yield 10∼30%).
¹H NMR(400MHz, CDCl₃) δ1.21(d, *J*=6.4Hz, 3H), 2.80(d, *J*=4.8Hz, 3H), 3.12(s, 1H), 4.09∼4.16(m, 1H), 4.86(br s, 1H), 5.99(d, *J*= 6.0Hz, 1H), 7.23∼7.40(m, 4H)

### Example 73: Synthesis of 1-(2-chlorophenyl)-2-hydroxypropyl-1-N-methylcarbamate(73)

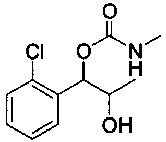

A regioisomer of monocarbamate was separated and purified by conducting the silica gel column chromatography as described in Example 29, to obtain the title compound (0.73g, yield 10%).
¹H NMR(400MHz, CDCl₃) δ 1.22(d, *J*=6Hz, 3H), 2.15(d, *J*=4Hz, 1H), 2.81(d, *J*=5Hz, 3H), 4.12(dd, *J*=6Hz, 1H), 4.83(br s, 1H), 6.00(d, *J*=6Hz, 1H), 7.23∼7.4 1(m, 4H)

### Example 74: Synthesis of 1-(2-chlorophenyl)-(S)-2-hydroxypropyl-(S)-1-N-propylcarbamate(74)

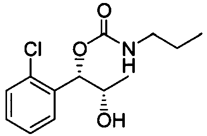

A regioisomer of monocarbamate was separated and purified by conducting the silica gel column chromatography as described in Example 16, to obtain the title compound (0.15g, yield 10∼30%).
¹H NMR(400MHz, CDCl₃) δ 0.91(t, *J*=7Hz, 3H), 1.22(d, *J*=6Hz, 3H), 1.52(dd, *J*=7Hz, 2H), 2.23(d, *J*=4Hz, 1H), 3.09∼3.21(m, 2H), 4.09∼4.17(m, 1H), 4.93(s, 1H), 5.99(d, *J*=6Hz, 1H), 7.23∼7.47(m, 4H)

### Example 75: Synthesis of 1-(2-chlorophenyl)-(R)-2-hydroxypropyl-(R)-1-N-propylcarbamate(75)

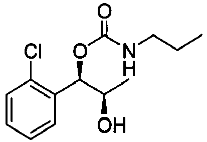

A regioisomer of monocarbamate was separated and purified by conducting the silica gel column chromatography as described in Example 23, to obtain the title compound (0.04g, yield 10∼30%).
¹H NMR(400MHz, CDCl₃) δ 0.91(t, *J*=7Hz, 3H), 1.22(d, *J*=6Hz, 3H), 1.52(dd, *J*=7Hz, 2H), 2.23(d, *J*=4Hz, 1H), 3.09∼3.21(m, 2H), 4.09∼4.17(m, 1H), 4.93(s, 1H), 5.99(d, *J*=6Hz, 1H), 7.23∼7.47(m, 4H)

### Example 76: Synthesis of 1-(2-chlorophenyl)-2-hydroxypropyl-1-N-propylcarbamate(76)

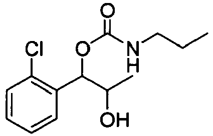

A regioisomer of monocarbamate was separated and purified by conducting the silica gel column chromatography as described in Example 30, to obtain the title compound (0.15g, yield 10%).
¹H NMR(400MHz, CDCl₃) δ 0.91(t, *J*=7Hz, 3H), 1.22(d, *J*=6Hz, 3H), 1.52(dd, *J*=7Hz, 2H), 2.23(d, *J*=4Hz, 1H), 3.09∼3.21(m, 2H), 4.09∼4.17(m, 1H), 4.93(s, 1H), 5.99(d, *J*=6Hz, 1H), 7.23∼7.47(m, 4H)

### Example 77: Synthesis of 1-(2-chlorophenyl)-(S)-2-hydroxypropyl-(S)-1-N-isopropylcarbamate(77)

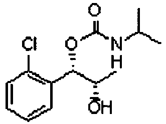

A regioisomer of monocarbamate was separated and purified by conducting the silica gel column chromatography as described in Example 17, to obtain the title compound (0.42g, yield 28%).
¹H NMR(400MHz, CDCl₃) δ1.10(d, *J*=6.0Hz, 3H), 1.15∼1.19(m, 6H), 2.41(s, 1H), 3.76∼4.08(m, 1H), 4.34(s, 1H), 4.83(br s 1H), 5.95(d, *J*=5.3Hz, 1H), 7.19∼7.39(m, 4H)

### Example 78: Synthesis of 1-(2-chlorophenyl)-(R)-2-hydroxypropyl-(R)-1-N-isopropylcarbamate(78)

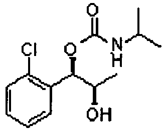

A regioisomer of monocarbamate was separated and purified by conducting the silica gel column chromatography as described in Example 24, to obtain the title compound (0.5g, yield 10%).
¹H NMR(400MHz, CDCl₃) δ1.13(d, *J*=6Hz, 3H), 1.20(dd, *J*=9.2Hz, 6H), 2.23(s, 1H), 3.77∼3.82(m, 1H), 4.10(s, 1H), 4.76(br s, 1H), 5.98(d, *J*=5.6Hz, 1H), 7.23∼7.41(m, 4H)

### Example 79: Synthesis of 1-(2-chlorophenyl)-2-hydroxypropyl-1-N-isopropylcarbamate(79)

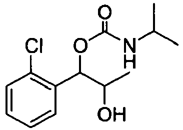

A regioisomer of monocarbamate was separated and purified by conducting the silica gel column chromatography as described in Example 31, to obtain the title compound (0.09g, yield 40%).
¹H NMR(400MHz, CDCl₃) δ 1.14(d, *J*=6Hz, 3H), 1.21(dd, *J*=6Hz, 6H), 2.16(d, *J*=5Hz, 1H), 3.81(t, *J*=6Hz, 1H), 4.11(d, *J*=5Hz, 1H), 4.73(br s, 1H), 5.98(d, *J*=5Hz, 1H), 7.24∼741(m, 4H)

### Example 80: Synthesis of 1-(2-chlorophenyl)-(S)-2-hydroxypropyl-(S)-1-N-cyclopropylcarbamate(80)

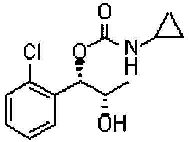

A regioisomer of monocarbamate was separated and purified by conducting the silica gel column chromatography as described in Example 18, to obtain the title compound (0.53g, yield 10∼30%).
¹H NMR(400MHz, CDCl₃) δ0.53∼0.60(m, 2H), 0.74(s, 2H), 1.21(d, J=6.0Hz, 3H), 2.19(s, 1H), 2.59(s, 1H), 4.11∼4.15(m, 1H), 5.13(br s, 1H), 5.99(d, J=5.20Hz, 1H), 7.23∼7.40(m, 4H)

### Example 81: Synthesis of 1-(2-chlorophenyl)-(R)-2-hydroxypropyl-(R)-1-N-cyclopropylcarbamate(81)

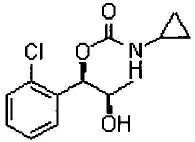

A regioisomer of monocarbamate was separated and purified by conducting the silica gel column chromatography as described in Example 25, to obtain the title compound (0.58g, yield 10%).
¹H NMR(400MHz, CDCl₃) δ0.53∼0.60(m, 2H), 0.74(s, 2H), 1.21(d, J=6.0Hz, 3H), 2.19(s, 1H), 2.59(s, 1H), 4.11∼4.15(m, 1H), 5.13(br s, 1H), 5.99(d, J=5.20Hz, 1H), 7.23∼7.40(m, 4H)

### Example 82: Synthesis of 1-(2-chlorophenyl)-2-hydroxypropyl-1-N-cyclopropylcarbamate(82)

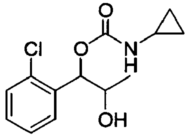

A regioisomer of monocarbamate was separated and purified by conducting the silica gel column chromatography as described in Example 32, to obtain the title compound (0.38g, yield 14%).
¹H NMR(400MHz, CDCl₃) δ 0.71(s, 2H), 1.19(d, *J*=6Hz, 3H), 2.45(S, 1H), 2.57(S, 1H), 4.08∼4.12(m, 1H), 5.26(s, 1H), 5.97(d, *J*=4Hz, 1H), 7.22∼7.54(m, 4H)

### Example 83: Synthesis of 1-(2-chlorophenyl)-(S)-2-hydroxypropyl-(S)-1-N-cyclohexylcarbamate(83)

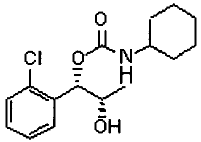

A regioisomer of monocarbamate was separated and purified by conducting the silica gel column chromatography as described in Example 19, to obtain the title compound (0.24g, yield 10∼30%).
¹H NMR(400MHz, CDCl₃) δ1.10∼1.39(m, 7H), 1.61(s, 3H), 1.71∼1.74(m, 2H), 1.87(d, *J*=11.2Hz, 1H), 2.48(d, *J*=10.8Hz, 1H), 3.46(t, *J*=4Hz, 1H), 4.10∼4.11(m, 1H), 4.80(br s 1H), 5.97(d, *J*=5.6Hz, 1H), 7.23∼7.41(m, 4H)

### Example 84: Synthesis of 1-(2-chlorophenyl)-(R)-2-hydroxypropyl-(R)-1-N-cyclohexylcarbamate(84)

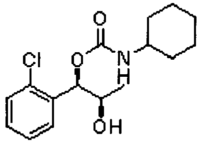

A regioisomer of monocarbamate was separated and purified by conducting the silica gel column chromatography as described in Example 26, to obtain the title compound (0.35g, yield 10%).
¹H NMR(400MHz, CDCl₃) δ1.10∼1.39(m, 7H), 1.61(s, 3H), 1.71∼1.74(m, 2H), 1.87(d, *J*=11.2Hz, 1H), 2.48(d, *J*=10.8Hz, 1H), 3.46(t, *J*=*4Hz,* 1H), 4.10∼4.11(m, 1H), 4.80(br s 1H), 5.97(d, *J*=5.6Hz, 1H), 7.23∼7.41(m, 4H)

### Example 85: Synthesis of 1-(2-chlorophenyl)-2-hydroxypropyl-1-N-cyclohexylcarbamate(85)

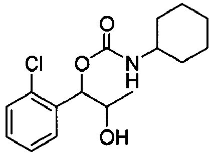

A regioisomer of monocarbamate was separated and purified by conducting the silica gel column chromatography as described in Example 33, to obtain the title compound (0.26g, yield 10%).
¹H NMR(400MHz, CDCl₃) δ 1.12∼1.19(m, 3H), 1.22(d, *J*=6Hz, 3H), 1.27∼1.37(m, 1H), 1.71(t, *J*=6Hz, 2H), 1.86∼1.88(m, 1H), 1.97∼2.00(m, 1H), 2.18(d, *J*=4Hz, 1H), 3.47(S, 1H), 4.12(t, *J*=6Hz, 1H), 4.78(S, 1H), 5.97(d, *J*=6Hz, 1H), 7.23∼7.40(m, 4H)

### Example 86: Synthesis of 1-(2-chlorophenyl)-(S)-2-hydroxypropyl-(S)-1-N-benzylcarbamate(86)

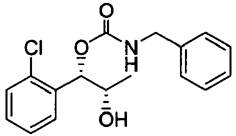

A regioisomer of monocarbamate was separated and purified by conducting the silica gel column chromatography as described in Example 20, to obtain the title compound (0.19g, yield 10∼30%).
¹H NMR(400MHz, CDCl₃) δ 1.23(d, *J*=6Hz, 3H), 2.16(d, *J*=4Hz, 1H), 4.12(t, *J*=6Hz, 1H), 4.31∼4.44(m, 2H), 5.22(br S, 1H), 6.04(d, *J*=6Hz, 1H), 7.27∼7.42(m, 9H)

### Example 87: Synthesis of 1-(2-chlorophenyl)-(R)-2-hydroxypropyl-(R)-1-N-benzylcarbamate(87)

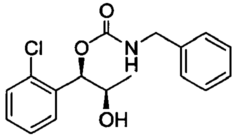

A regioisomer of monocarbamate was separated and purified by conducting the silica gel column chromatography as described in Example 27, to obtain the title compound (0.07g, yield 10∼30%).
¹H NMR(400MHz, CDCl₃) δ 1.23(d, *J*=6Hz, 3H), 2.16(d, *J*=4Hz, 1H), 4.12(t, *J*=6Hz, 1H), 4.31∼4.44(m, 2H), 5.22(br S, 1H), 6.04(d, *J*=6Hz, 1H), 7.27∼7.42(m, 9H)

### Example 88: Synthesis of 1-(2-chlorophenyl)-2-hydrozypropyl-1-N-benzylcarbamate(88)

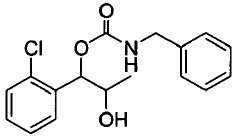

A regioisomer of monocarbamate was separated and purified by conducting the silica gel column chromatography as described in Example 34, to obtain the title compound (0.21g, yield 14%).
¹H NMR(400MHz, CDCl₃) δ 1.23(d, *J*=6Hz, 3H), 2.16(d, *J*=4Hz, 1H), 4.12(t, *J*=6Hz, 1H), 4.31∼4.44(m, 2H), 5.22(br S, 1H), 6.04(d, *J*=6Hz, 1H), 7.27∼7.42(m, 9H)

### Example 89: Synthesis of 1-(2,4-dichlorophenyl)-(S)-2-hydroxypropyl-(S)-1-carbamate(89)

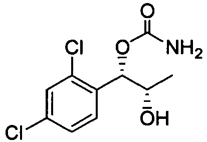

A regioisomer of monocarbamate was separated and purified by conducting the silica gel column chromatography as described in Example 36, to obtain the title compound (0.05g, yield 10∼30%).
¹H NMR(400MHz, CDCl₃) δ1.13(d, *J* = 6.8Hz, 3H), 2.49(d, *J* = 4.0Hz, 1H), 4.66∼4.74(m, 1H), 4.76(br s, 2H), 6.20(d, *J* = 8.8Hz, 1H), 7.30(d, J=8.4Hz, 1H), 7.39(d, J=2.0Hz, 2H), 7.50(dd, J=8.4Hz, 2.0Hz, 1H)

### Example 90: Synthesis of 1-(2,6-dichlorophenyl)-(S)-2-hydroxypropyl-(S)-1-carbamate(90)

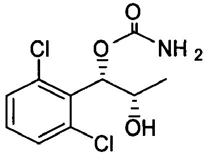

A regioisomer of monocarbamate was separated and purified by conducting the silica gel column chromatography as described in Example 37, to obtain the title compound (0.07g, yield 24%).
¹H NMR(400MHz, CDCl₃) δ1.13(d, *J* = 6.8Hz, 3H), 2.49(d, *J* = 4.0Hz, 1H), 4.66∼4.74(m, 1H), 4.76(br s, 2H), 6.20(d, *J*= 8.8Hz, 1H), 7.25∼7.40(m, 3H)

### Example 91: Synthesis of 1-(2,3-dichlorophenyl)-(S)-2-hydroxypropyl-(S)-1-carbamate(91)

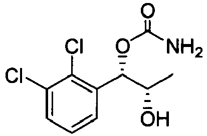

A regioisomer of monocarbamate was separated and purified by conducting the silica gel column chromatography as described in Example 38, to obtain the title compound (0.08g, yield 10∼30%).
¹H NMR(400MHz, CDCl₃) δ1.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 92: Synthesis of 1-(2,4-dichlorophenyl)-(S)-2-hydroxybutyl-(S)-1-carbamate(92)

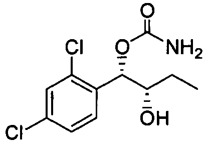

A regioisomer of monocarbamate was separated and purified by conducting the silica gel column chromatography as described in Example 39, to obtain the title compound (0.07g, yield 10∼30%).
¹H NMR(400MHz, CDCl₃) δ0.77(t, *J* = 7.4Hz, 3H), 0.92∼1.01(m, 1H), 1.18∼1.28(m, 1H), 4.06∼4.13(m, 1H), 4.96(d, *J* = 6.0Hz, 1H), 5.91(d, *J* = 8.8Hz, 1H), 6.4(br s, 2H), 7.30∼7.50(m, 3H)

### Example 93: Synthesis of 1-(2,6-dichlorophenyl)-(S)-2-hydroxybutyl-(S)-1-carbamate(93)

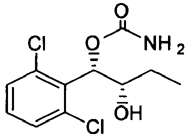

A regioisomer of monocarbamate was separated and purified by conducting the silica gel column chromatography as described in Example 40, to obtain the title compound (0.11g, yield 29%).
¹H NMR(400MHz, CDCl₃) δ0.77(t, *J* = 7.4Hz, 3H), 0.92∼1.01(m, 1H), 1.18∼1.28(m, 1H), 4.06∼4.13(m, 1H), 4.96(d, *J*=6.0Hz, 1H), 5.91(d, *J*=8.8Hz, 1H), 6.4(br s, 2H), 7.25∼7.40(m, 3H)

### Example 94: Synthesis of 1-(2,4-dichlorophenyl)-(S)-2-hydroxy-3-methylbutyl-(S)-1-carbamate(94)

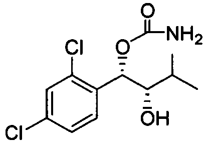

A regioisomer of monocarbamate was separated and purified by conducting the silica gel column chromatography as described in Example 41, to obtain the title compound (0.01g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.30∼7.50(m, 3H)

### Example 95: Synthesis of 1-(2,6-dichlorophenyl)-(S)-2-hydroxy-3-methylbutyl-(S)-1-carbamate(95)

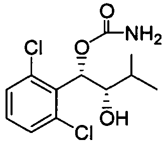

A regioisomer of monocarbamate was separated and purified by conducting the silica gel column chromatography as described in Example 42, to obtain the title compound (0.03g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.25∼7.40(m, 3H)

### Example 96: Synthesis of 1-(2,4-dichlorophenyl)-(S)-2-hydroxyhexyl-(S)-1-carbamate(96)

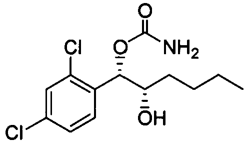

A regioisomer of monocarbamate was separated and purified by conducting the silica gel column chromatography as described in Example 43, to obtain the title compound (0.21g, yield 10∼30%).
¹H NMR(400MHz, CDCl₃) δ0.85(t, *J*=7.2Hz, 3H), 1.18∼1.33(m, 4H), 1.48∼1.55(m, 2H), 2.35(d, *J*=4.4Hz, 1H), 4.45∼4.50(m, 1H), 4.76(br s, 2H), 6.21(d, *J*=8.4Hz, 1H), 7.30∼7.50(m, 3H)

### Example 97: Synthesis of 1-(2,6-dichlorophenyl)-(S)-2-hydroxyhexyl-(S)-1-carbamate(97)

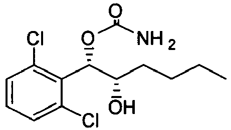

A regioisomer of monocarbamate was separated and purified by conducting the silica gel column chromatography as described in Example 44, to obtain the title compound (0.06g, yield 29%).
¹H NMR(400MHz, CDCl₃) δ0.85(t, *J* = 7.2Hz, 3H), 1.18∼1.33(m, 4H), 1.48∼1.55(m, 2H), 2.35(d, *J* = 4.4Hz, 1H), 4.45∼4.50(m, 1H), 4.76(br s, 2H), 6.21(d, *J*=8.4Hz, 1H), 7.16∼7.34(m, 3H)

### Example 98: Synthesis of 1-(2,4-dichlorophenyl)-(R)-2-hydroxypropyl-(R)-1-carbamate(98)

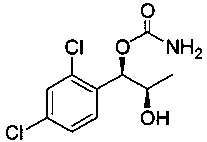

A regioisomer of monocarbamate was separated and purified by conducting the silica gel column chromatography as described in Example 45, to obtain the title compound (0.04g, yield 10∼30%).
¹H NMR(400MHz, CDCl₃) δ1.13(d, *J*=6.8Hz, 3H), 2.49(d, *J*=4.0Hz, 1H), 4.66∼4.74(m, 1H), 4.76(br s, 2H), 6.20(d, *J*=8.8Hz, 1H), 7.30∼7.50(m, 3H)

### Example 99: Synthesis of 1-(2,6-dichlorophenyl)-(R)-2-hydroxypropyl-(R)-1-carbamate(99)

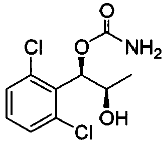

A regioisomer of monocarbamate was separated and purified by conducting the silica gel column chromatography as described in Example 46, to obtain the title compound (0.09g, yield 10∼30%).
¹H NMR(400MHz, CDCl₃) δ1.13(d, *J* = 6.8Hz, 3H), 2.49(d, *J* = 4.0Hz, 1H), 4.66∼4.74(m, 1H), 4.76(br s, 2H), 6.20(d, *J* = 8.8Hz, 1H), 7.25∼7.40(m, 3H)

### Example 100: Synthesis of 1-(2,3-dichlorophenyl)-(R)-2-hydroxypropyl-(R)-1-carbamate(100)

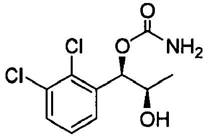

A regioisomer of monocarbamate was separated and purified by conducting the silica gel column chromatography as described in Example 47, to obtain the title compound (0.25g, yield 10∼30%).
¹H NMR(400MHz, CDCl₃) 81.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 101: Synthesis of 1-(2,4-dichlorophenyl)-(R)-2-hydroxybutyl-(R)-1-carbamate(101)

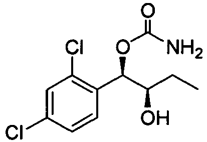

A regioisomer of monocarbamate was separated and purified by conducting the silica gel column chromatography as described in Example 48, to obtain the title compound (0.08g, yield 10∼30%).
¹H NMR(400MHz, CDCl₃) δ0.77(t, *J* = 7.4Hz, 3H), 0.92∼1.01(m, 1H), 1.18∼1.28(m, 1H), 4.06∼4.13(m, 1H), 4.96(d, *J* = 6.0Hz, 1H), 5.91(d, *J* = 8.8Hz, 1H), 6.4(br s, 2H), 7.30∼7.50(m, 3H)

### Example 102: Synthesis of 1-(2,6-dichlorophenyl)-(R)-2-hydroxybutyl-(R)-1-carbamate(102)

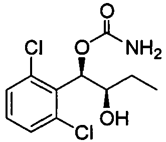

A regioisomer of monocarbamate was separated and purified by conducting the silica gel column chromatography as described in Example 49, to obtain the title compound (0.09g, yield 10∼30%).
¹H NMR(400MHz, CDCl₃) δ0.77(t, *J* = 7.4Hz, 3H), 0.92∼1.01(m, 1H), 1.18∼1.28(m, 1H), 4.06∼4.13(m, 1H), 4.96(d, *J* = 6.0Hz, 1H), 5.91(d, *J* = 8.8Hz, 1H), 6.4(br s, 2H), 7.25∼7.40(m, 3H)

### Example 103: Synthesis of 1-(2,4-dichlorophenyl)-(R)-2-hydroxy-3-methylbutyl-(R)-1-carbamate(103)

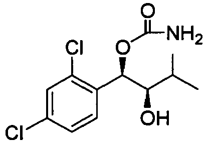

A regioisomer of monocarbamate was separated and purified by conducting the silica gel column chromatography as described in Example 50, to obtain the title compound (0.01g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.30∼7.50(m, 3H)

### Example 104: Synthesis of 1-(2,6-dichlorophenyl)-(R)-2-hydroxy-3-methylbutyl-(R)-1-carbamate(104)

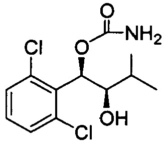

A regioisomer of monocarbamate was separated and purified by conducting the silica gel column chromatography as described in Example 51, to obtain the title compound (0.01g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.25∼7.40(m, 3H)

### Example 105: Synthesis of 1-(2,4-dichlorophenyl)-(R)-2-hydroxyhexyl-(R)-1-carbamate(105)

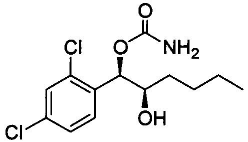

A regioisomer of monocarbamate was separated and purified by conducting the silica gel column chromatography as described in Example 52, to obtain the title compound (0.21 g, yield 10∼30%).
¹H NMR(400MHz, CDCl₃) δ0.85(t, *J*=7.2Hz, 3H), 1.18∼1.33(m, 4H), 1.48∼1.55(m, 2H), 2.35(d, *J*=4.4Hz, 1H), 4.45∼4.50(m, 1H), 4.76(br s, 2H), 6.21(d, *J*=8.4Hz, 1H), 7.30∼7.50(m, 3H)

### Example 106: Synthesis of 1-(2,6-dichlorophenyl)-(R)-2-hydroxyhexyl-(R)-1-carbamate(106)

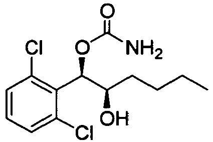

A regioisomer of monocarbamate was separated and purified by conducting the silica gel column chromatography as described in Example 53, to obtain the title compound (0.12g, yield 10∼30%).
¹H NMR(400MHz, CDCl₃) δ0.85(t, *J* = 7.2Hz, 3H), 1.18∼1.33(m, 4H), 1.48∼1.55(m, 2H), 2.35(d, *J*= 4.4Hz, 1H), 4.45∼4.50(m, 1H), 4.76(br s, 2H), 6.21(d, *J*= 8.4Hz, 1H), 7.16∼7.34(m, 3H)

### Example 107: Synthesis of 1-(2,4-dichlorophenyl)-2-hydrozypropyl-1-carbamate(107)

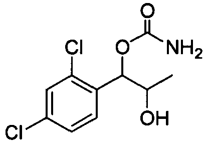

A regioisomer of monocarbamate was separated and purified by conducting the silica gel column chromatography as described in Example 54, to obtain the title compound (0.05g, yield 10∼30%).
¹H NMR(400MHz, CDCl₃) δ1.13(d, *J*=6.8Hz, 3H), 2.49(d, *J*=4.0Hz, 1H), 4.66∼4.74(m, 1H), 4.76(br s, 2H), 6.20(d, *J*=8.8Hz, 1H), 7.30∼7.50(m, 3H)

### Example 108: Synthesis of 1-(2,6-dichlorophenyl)-2-hydroarypropyl-1-carbamate(108)

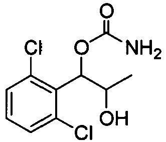

A regioisomer of monocarbamate was separated and purified by conducting the silica gel column chromatography as described in Example 55, to obtain the title compound (0.06g, yield 10∼30%).
¹H NMR(400MHz, CDCl₃) δ1.13(d, *J* = 6.8Hz, 3H), 2.49(d, *J* = 4.0Hz, 1H), 4.66∼4.74(m, 1H), 4.76(br s, 2H), 6.20(d, *J* = 8.8Hz, 1H), 7.25∼7.40(m, 3H)

### Example 109: Synthesis of 1-(2,3-dichlorophenyl)-(R)-2-hydroxypropyl-(R)-1-carbamate(109)

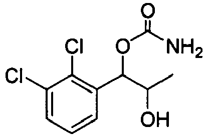

A regioisomer of monocarbamate was separated and purified by conducting the silica gel column chromatography as described in Example 56, to obtain the title compound (0.02g, yield 10∼30%).
¹H NMR(400MHz, CDCl₃) 81.15(d, *J* = 6.4Hz, 3H), 3.66(d, *J* = 9.2Hz, 1H), 4.73(br s, 2H), 5.43(t, *J* = 9.0Hz, 1H), 5.62∼5.69(m, 1H), 7.18∼7.22(m, 3H),

### Example 110: Synthesis of 1-(2,4-dichlorophenyl)-2-hydroxybutyl-1-carbamate(110)

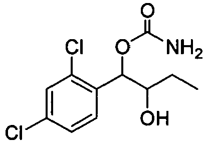

A regioisomer of monocarbamate was separated and purified by conducting the silica gel column chromatography as described in Example 57, to obtain the title compound (0.07g, yield 10∼30%).
¹H NMR(400MHz, CDCl₃) δ0.77(t, *J*=7.4Hz, 3H), 0.92∼1.01(m, 1H), 1.18∼1.28(m, 1H), 4.06∼4.13(m, 1H), 4.96(d, *J*=6.0Hz, 1H), 5.91(d, J=8.8Hz, 1H), 6.4(br s, 2H), 7.30∼7.50(m, 3H)

### Example 111: Synthesis of 1-(2,6-dichlorophenyl)-2-hydroxybutyl-1-carbamate(111)

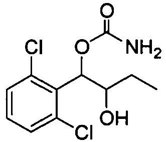

A regioisomer of monocarbamate was separated and purified by conducting the silica gel column chromatography as described in Example 58, to obtain the title compound (0.10g, yield 10∼30%).
¹H NMR(400MHz, CDCl₃) δ0.77(t, *J* = 7.4Hz, 3H), 0.92∼1.01(m, 1H), 1.18∼1.28(m, 1H), 4.06∼4.13(m, 1H), 4.96(d, *J* = 6.0Hz, 1H), 5.91(d, *J* = 8.8Hz, 1H), 6.4(br s, 2H), 7.25∼7.40(m, 3H)

### Example 112: Synthesis of 1-(2,4-dichlorophenyl)-2-hydroxy-3-methylbutyl-1-carbamate(112)

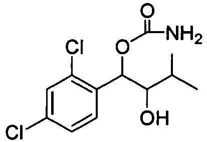

A regioisomer of monocarbamate was separated and purified by conducting the silica gel column chromatography as described in Example 59, to obtain the title compound (0.04g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.30∼7.50(m, 3H)

### Example 113: Synthesis of 1-(2,6-dichlorophenyl)-2-hydroxy-3-methylbutyl-1-carbamate(113)

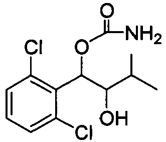

A regioisomer of monocarbamate was separated and purified by conducting the silica gel column chromatography as described in Example 60, to obtain the title compound (0.01g, yield 10∼30%).
¹H NMR(400MHz, CDCl₃) δ1.00(t, *J* = 7.2Hz, 6H), 1.73∼1.79(m, 1H), 3.67∼3.69(m, 1H), 4.96(d, *J*=6.0Hz, 1H), 5.91(d, *J*=8.8Hz, 1H), 6.42(br s, 2H), 7.25∼7.40(m, 3H)

### Example 114: Synthesis of 1-(2,4-dichlorophenyl)-2-hydroxyhexyl-1-carbamate(114)

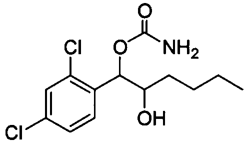

A regioisomer of monocarbamate was separated and purified by conducting the silica gel column chromatography as described in Example 61, to obtain the title compound (0.21g, yield 10∼30%).
¹H NMR(400MHz, CDCl₃) δ0.85(t, *J*=7.2Hz, 3H), 1.18∼1.33(m, 4H), 1.48∼1.55(m, 2H), 2.35(d, *J*=4.4Hz, 1H), 4.45∼4.50(m, 1H), 4.76(br s, 2H), 6.21(d, *J*=8.4Hz, 1H), 7.30∼7.50(m, 3H)

### Example 115: Synthesis of 1-(2,6-dichlorophenyl)-2-hydroxyhezyl-1-carbamate(115)

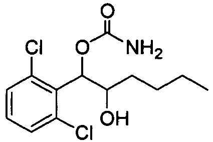

A regioisomer of monocarbamate was separated and purified by conducting the silica gel column chromatography as described in Example 62, to obtain the title compound (0.12g, yield 10∼30%).
¹H NMR(400MHz, CDCl₃) δ0.85(t, *J* = 7.2Hz, 3H), 1.18∼1.33(m, 4H), 1.48∼1.55(m, 2H), 2.35(d, *J*= 4.4Hz, 1H), 4.45∼4.50(m, 1H), 4.76(br s, 2H), 6.21(d, *J* = 8.4Hz, 1H), 7.16∼7.34(m, 3H)

Compounds 1 to 115 produced in Examples 1 to 115 were summarized in following Tables 1 and 2.

**[Table 1] Compounds 1 to 67 having the structure of Chemical Formula 1 where 'A' is a carbamoyl derivative and 'B' is H**

| No. | X | n (position) | 1^{st} Chiral | 2^{nd} Chiral | R¹ | A | B |
|---|---|---|---|---|---|---|---|
| | | | | | | A = carbamoyl derivative R² = | B = H |
| 1 | Cl | 1(2-) | S | S | Me | H | H |
| 2 | Cl | 1(2-) | R | R | Me | H | H |
| 3 | Cl | 1(2-) | Rac. | Rac. | Me | H | H |
| 4 | Cl | 1(2-) | S | R | Me | H | H |
| 5 | Cl | 1(2-) | R | S | Me | H | H |
| 6 | Cl | 1(2-) | S | S | Et | H | H |
| 7 | Cl | 1(2-) | R | R | Et | H | H |
| 8 | Cl | 1(2-) | Rac. | Rac. | Et | H | H |
| 9 | Cl | 1(2-) | S | S | Isopropyl | H | H |
| 10 | Cl | 1(2-) | R | R | Isopropyl | H | H |
| 11 | Cl | 1(2-) | Rac. | Rac. | Isopropyl | H | H |
| 12 | Cl | 1(2-) | S | S | butyl | H | H |
| 13 | Cl | 1(2-) | R | R | butyl | H | H |
| 14 | Cl | 1(2-) | Rac. | Rac. | butyl | H | H |
| 15 | Cl | 1(2-) | S | S | Me | Me | H |
| 16 | Cl | 1(2-) | S | S | Me | Propyl | H |
| 17 | Cl | 1(2-) | S | S | Me | Isopropyl | H |
| 18 | Cl | 1(2-) | S | S | Me | Cyclopropyl | H |
| 19 | Cl | 1(2-) | S | S | Me | Cvclohexvl | H |
| 20 | Cl | 1(2-) | S | S | Me | Benzyl | H |
| 21 | Cl | 1(2-) | S | s | Me | Bicyclo[2.2.1]heptane | H |
| 22 | Cl | 1(2-) | R | R | Me | Me | H |
| 23 | Cl | 1(2-) | R | R | Me | Propyl | H |
| 24 | Cl | 1(2-) | R | R | Me | Isopropyl | H |
| 25 | Cl | 1(2-) | R | R | Me | Cyclopropyl | H |
| 26 | Cl | 1(2-) | R | R | Me | Cyclohexyl | H |
| 27 | Cl | 1(2-) | R | R | Me | Benzyl | H |
| 28 | Cl | 1(2-) | R | R | Me | Bicyclo[2.2.1]heptane | H |
| 29 | Cl | 1(2-) | Rac. | Rac. | Me | Me | H |
| 30 | Cl | 1(2-) | Rac. | Rac. | Me | Propyl | H |
| 31 | Cl | 1(2-) | Rac. | Rac. | Me | Isopropyl | H |
| 32 | Cl | 1(2-) | Rac. | Rac. | Me | Cyclopropyl | H |
| 33 | Cl | 1(2-) | Rac. | Rac. | Me | Cyclohexyl | H |
| 34 | Cl | 1(2-) | Rac. | Rac. | Me | Benzyl | H |
| 35 | Cl | 1(2-) | Rac, | Rac. | Me | Bicyclo[2.2.1]heptane | H |
| 36 | Cl | 2(2,4-) | S | S | Me | H | H |
| 37 | Cl | 2(2,6-) | S | S | Me | H | H |
| 38 | Cl | 2(2,3-) | S | S | Me | H | H |
| 39 | Cl | 2(2,4-) | S | S | Et | H | H |
| 40 | Cl | 2(2,6-) | S | S | Et | H | H |
| 41 | Cl | 2(2,4-) | S | S | Isopropyl | H | H |
| 42 | Cl | 2(2,6-) | S | S | Isopropyl | H | H |
| 43 | Cl | 2(2,4-) | S | S | butyl | H | H |
| 44 | Cl | 2(2,6-) | S | S | butyl | H | H |
| 45 | Cl | 2(2,4-) | R | R | Me | H | H |
| 46 | Cl | 2(2,6-) | R | R | Me | H | H |
| 47 | Cl | 2(2,3-) | R | R | Me | H | H |
| 48 | Cl | 2(2,4-) | R | R | Et | H | H |
| 49 | Cl | 2(2,6-) | R | R | Et | H | H |
| 50 | Cl | 2(2,4-) | R | R | Isopropyl | H | H |
| 51 | Cl | 2(2,6-) | R | R | Isopropyl | H | H |
| 52 | Cl | 2(2,4-) | R | R | butyl | H | H |
| 53 | Cl | 2(2,6-) | R | R | butyl | H | H |
| 54 | Cl | 2(2,4-) | Rac, | Rac. | Me | H | H |
| 55 | Cl | 2(2,6-) | Rac, | Rac. | Me | H | H |
| 56 | Cl | 2(2,3-) | Rac, | Rac. | Me | H | H |
| 57 | Cl | 2(2,4-) | Rac, | Rac. | Et | H | H |
| 58 | Cl | 2(2,6-) | Rac, | Rac. | Et | H | H |
| 59 | Cl | 2(2,4-) | Rac, | Rac. | Isopropyl | H | H |
| 60 | Cl | 2(2,6-) | Rac, | Rac. | Isopropyl | H | H |
| 61 | Cl | 2(2,4-) | Rac, | Rac. | butyl | H | H |
| 62 | Cl | 2(2,6-) | Rac, | Rac. | butyl | H | H |
| 63 | F | 1(2-) | S | S | Me | H | H |
| 64 | F | 1(2-) | R | R | Me | H | H |
| 65 | I | 1(2-) | S | S | Me | H | H |
| 66 | I | 1(2-) | R | R | Me | H | H |
| 67 | I | 1(2-) | S | S | Et | H | H |

**[Table 2] Compounds 68 to 115 having the structure of Chemical Formula 1 where 'A' is H and 'B' is a carbamoyl derivative**

| No. | X | n (position) | 1^{st} Chiral | 2^{nd} Chiral | R¹ | A | B |
|---|---|---|---|---|---|---|---|
| | | | | | | A=H | B= carbamoyl derivative R³= |
| 68 | Cl | 1(2-) | S | S | Me | H | H |
| 69 | Cl | 1(2-) | R | R | Me | H | H |
| 70 | Cl | 1(2-) | Rac. | Rac. | Me | H | H |
| 71 | Cl | 1(2-) | S | S | Me | H | Me |
| 72 | Cl | 1(2-) | R | R | Me | H | Me |
| 73 | Cl | 1(2-) | Rac. | Rac. | Me | H | Me |
| 74 | Cl | 1(2-) | S | S | Me | H | Propyl |
| 75 | Cl | 1(2-) | R | R | Me | H | Propyl |
| 76 | Cl | 1(2-) | Rac. | Rac. | Me | H | Propyl |
| 77 | Cl | 1(2-) | S | S | Me | H | Isopropyl |
| 78 | Cl | 1(2-) | R | R | Me | H | Isopropyl |
| 79 | Cl | 1(2-) | Rac. | Rac. | Me | H | Isopropyl |
| 80 | Cl | 1(2-) | S | S | Me | H | Cyclopropyl |
| 81 | Cl | 1(2-) | R | R | Me | H | Cyclopropyl |
| 82 | Cl | 1(2-) | Rac. | Rac. | Me | H | Cyclopropyl |
| 83 | Cl | 1(2-) | S | S | Me | H | Cyclohexyl |
| 84 | Cl | 1(2-) | R | R | Me | H | Cyclohexyl |
| 85 | Cl | 1(2-) | Rac. | Rac. | Me | H | Cyclohexyl |
| 86 | Cl | 1(2-) | S | S | Me | H | Benzyl |
| 87 | Cl | 1(2-) | R | R | Me | H | Benzyl |
| 88 | Cl | 1(2-) | Rac. | Rac. | Me | H | Benzyl |
| 89 | Cl | 2(2,4-) | S | S | Me | H | H |
| 90 | Cl | 2(2,6-) | S | S | Me | H | H |
| 91 | Cl | 2(2,3-) | S | S | Me | H | H |
| 92 | Cl | 2(2,4-) | S | S | Et | H | H |
| 93 | Cl | 2(2,6-) | S | S | Et | H | H |
| 94 | Cl | 2(2,4-) | S | S | Isopropyl | H | H |
| 95 | Cl | 2(2,6-) | S | S | Isopropyl | H | H |
| 96 | Cl | 2(2,4-) | S | S | Butyl | H | H |
| 97 | Cl | 2(2,6-) | S | S | Butyl | H | H |
| 98 | Cl | 2(2,4-) | R | R | Me | H | H |
| 99 | Cl | 2(2,6-) | R | R | Me | H | H |
| 100 | Cl | 2(2,3-) | R | R | Me | H | H |
| 101 | Cl | 2(2,4-) | R | R | Et | H | H |
| 102 | Cl | 2(2,6-) | R | R | Et | H | H |
| 103 | Cl | 2(2,4-) | R | R | Isopropyl | H | H |
| 104 | Cl | 2(2,6-) | R | R | Isopropyl | H | H |
| 105 | Cl | 2(2,4-) | R | R | Butyl | H | H |
| 106 | Cl | 2(2,6-) | R | R | Butyl | H | H |
| 107 | Cl | 2(2,4-) | Rac. | Rac. | Me | H | H |
| 108 | Cl | 2(2,6-) | Rac. | Rac. | Me | H | H |
| 109 | Cl | 2(2,3-) | Rac. | Rac. | Me | H | H |
| 110 | Cl | 2(2,4-) | Rac. | Rac. | Et | H | H |
| 111 | Cl | 2(2,6-) | Rac. | Rac. | Et | H | H |
| 112 | Cl | 2(2,4-) | Rac. | Rac. | Isopropyl | H | H |
| 113 | Cl | 2(2,6-) | Rac. | Rac. | Isopropyl | H | H |
| 114 | Cl | 2(2,4-) | Rac. | Rac. | Butyl | H | H |
| 115 | Cl | 2(2,6-) | Rac. | Rac. | Butyl | H | H |

### [Animal Testing Examples]

For testing, male mice (ICR) were purchased from ORIENT BIO INC. (Korea), divided into several groups with 6 mice in each group, and were adapted for 4-5 days. The mice having the weight ranging from 19g to 26g were employed for the test. The pharmacological effect of the test compounds on muscle relaxation was evaluated by Rotarod test, grip strength test, and muscular force (wire hang) test. All mice were adapted to the test environment at one hour before starting the tests. The pharmacological effects of all the test compounds were evaluated by administration through peritoneal cavity of the mice (10 ul/g, bw).

### Experimental Example 1: Measurement of muscle relaxation activity by endurance time on a rotarod rotating at accelerated speed

At 24 hours before testing, the mice to be tested were preliminarily trained for 5 minutes on a rod rotating at the rate of 6 revolutions per a minute. The pharmacological effect on muscle relaxation of the test compounds were evaluated by observing the mice on a rod for 5 minutes, where the rod was accelerated from 4 to 40 revolutions per a minute during the test time. The endurance time that each mouse endures on the acceleratedly rotated rod without falling off from the rod was recorded. As test time for evaluation, a maximum of 5 minutes was applied. In case the mouse does not fall off from the rod for testing time, the endurance time was recorded as 5 minutes. All the test compounds were intraperitoneally administered (10 ul/g, bw) to the mice at 15 minutes, 30 minutes, 1 hour, and 2 hours prior to the testing, and the median effective concentration (ED50) was determined at the time that the drug exhibits its maximum pharmacological effect. The obtained results were shown in following Table 3. This experimentation was conducted according to the method described in the reference, 'Monville et al. (2006) Comparison of incremental and accelerating protocol of the rotarod test for the assessment of motor deficits in the 6-OHDA model. J. Neurosci. Meth. 158: 219-223'.

### Experimental Example 2: Measurement of muscle relaxation activity by residence time on a rotarod rotating at a fixed speed

All the mice to be tested were preliminarily trained for 5 minutes on a rod rotating at the rate of 15 revolutions per a minute. The mice that could not remain on the rod without falling off therefrom for a minimum of 2 minutes were excluded from this testing. After the training, all the mice were allowed to rest for 45-60 minutes. Before the administration of the test compounds, the mice were subjected to a further training for one minute on the rod rotating under the same condition, where the mice falling off from the rod were excluded from this experimentation. All the test compounds were intraperitoneally administered (10 ul/g, bw) to the mice at 15 minutes, 30 minutes, 1 hour, and 2 hours prior to the testing, and the median effective concentration (ED50) was determined at the time(generally 15 min, 30min or 60min) that the comounds exhibit their maximum pharmacological effect. In case a mouse stays on the rod until the test is finished, the time was recorded as 10 minutes. As test time for evaluation, a maximum of 10 minutes was applied. The obtained results were shown in following Table 3. This experimentation was conducted according to the method described in the reference, 'Yasuda et al. (2005) Antipyretic, analgesic and muscle relaxant activities of Pueraria isoflavonoids and their metabolites from Pueraria lobata Ohwi - a traditional Chinese drug. Biol. Pharm. Bull. 28: 1224-1228'.

### Experimental Example 3: Measurement of muscle relaxation activity by grip strenth

A grip strength test using the test animals' forelimbs was performed using an instrument equipped with triangle ring and designed so as to easily grip with the forelimbs of experimental animals, manufactured from Ugo Basile Inc.(Ugo Basile, Model47106, Italy). The test was conducted before and after administration of the compounds to evaluate the effects thereof. All the test compounds were intraperitoneally administered (10 ul/g, bw) at 15 minutes, 30 minutes, 1 hour, and 2 hours before test, and the median effective concentration (ED50) was determined at the time(generally 15 min, 30min or 60min) that the compounds exhibits therir maximum pharmacological effect. The mouse was made to grip the rod with its forelimbs, and its tail was pulled, where the force at which the mouse detached from the rod was recorded. The instrument indicated the force in grams. All of the mice were given 3 opportunities for test, and the 3 highest values among the test opportunities were selected and the mean value was used as the test result. The obtained results are shown in Table 3. This experimentation was conducted according to the method described in the reference, 'Nevins et al. (1993) Quantitative grip strength assessment as a means of evaluating muscle relaxation in mice. Psychopharmacol. 110: 92-96'.

### Experimental Example 4: Measurement of muscle relaxation activity by wire hang

This experimentation was conducted using a metal wire of 30cm in length, which was suspended between two pillars at a height of about 40cm from the bottom covered with a soft pad. All the test compounds were administered to the mice through peritoneal cavity (10 ul/g, bw) at 15 minutes, 30 minutes, 1 hour, and 2 hours prior to the testing, and the median effective concentration (ED50) was determined at the time that the compound exhibits their maximum pharmacological effect. Each mouse was made to grip the wire using two forelimbs, and the elapse time before the mouse fell off from the wire to the pad on the bottom was recorded in seconds. Each mouse was given 5 opportunities for this test at an interval of 2 minutes period. The highest 3 records among the test opportunities were selected and the mean value was used as the test result. The obtained results are shown in Table 3. This experimentation was was conducted according to the method described in the reference, 'Jacqueline N. Crawley (1999) Behavioral phenotyping of transgenic and knockout mice: experimental design and evaluation of general health, sensory functions, motor abilities, and specific behavioral tests. Brain Res. 835: 18-26'.

### [Statistical Analysis]

The obtained results are shown as mean±sem. The difference between the groups was statistically analyzed by ANOVA, and then, further examined by Dunnett's test or Bonferroni test. If p is less than 0.05, it was determined that the difference between the groups had statistical significance.

### [Results]

The results of muscle relaxation activity of the phenyl carbamate compounds measured in above Experimental Examples 1 to 4 are shown in following Table 3. In the Table 3, the ED50 was represented by the concentration where the compound shows the 50% of muscle relaxation activity compared to the vehicle only (100%).

**[Table 3] Results of the measurements of muscle relaxation activity of the phenyl carbamate compounds**

| No. | MR test (ED50; mg/kg, bw) | | | | control |
|---|---|---|---|---|---|
| | I | II | III | IV | |
| 1 | 39.7 | 23.3 | 40.9 | 13.3 | 1 |
| 2 | 66.3 | 76.5 | 110.0 | 43.3 | 1 |
| 3 | 57.1 | 47.7 | 72.6 | 34.0 | 1 |
| 4 | 69.3 | 65.0 | 124.2 | 40.0 | 1 |
| 5 | 66.9 | 65.5 | 95.0 | 52.5 | 1 |
| 6 | - | - | 70.7 | - | 1 |
| 7 | - | - | 248.4 | - | 1 |
| 8 | 50.6 | - | 69.5 | - | 1 |
| 9 | - | - | 103.9 | - | 1 |
| 11 | 102.5 | - | 126.1 | - | 1 |
| 15 | 51.4 | 42.8 | 83.6 | 25.4 | 1 |
| 16 | 48.7 | 61.6 | 67.8 | 16.1 | 2 |
| 17 | 73.1 | 66.4 | 91.5 | 41.4 | 2 |
| 18 | 59.2 | 61.2 | 87.4 | 29.5 | 2 |
| 19 | 95.3 | - | 109.8 | 28.5 | 2 |
| 22 | 25.7 | 25.1 | 28.3 | 22.4 | 1 |
| 23 | - | - | 73.8 | 46.0 | 2 |
| 24 | 38.6 | 44.3 | 48.8 | 17.8 | 2 |
| 25 | 30.0 | 18.3 | 46.1 | 32.9 | 1 |
| 26 | - | - | - | 63.8 | 2 |
| 29 | 30.2 | 41.0 | 46.0 | 38.0 | 2 |
| 30 | - | - | 65.4 | 31.7 | 2 |
| 31 | - | - | 50.4 | 52.1 | 1 |
| 32 | - | - | 45.2 | 36.6 | 2 |
| 33 | - | - | - | 74.6 | 2 |
| 63 | | | 118.3 | 100^{a}(84.2%) | 1 |
| 64 | | | 120^{a}(35.4%) | 100^{a}(30.8%) | 1 |
| 65 | | 28.0 | 42.9 | 23.4 | 2 |
| 66 | | 67.9 | 46.0 | 76.3 | 2 |
| 67 | | 30.2 | 69.1 | 26.2 | 2 |

| | | | | | |
|---|---|---|---|---|---|
| I = Acc. Rotatod (accelerated rotating ratarod test; Experimental Example 1), II= Fixed 15 r.p.m. Rotarod (constantly rotating ratarod test; Experimental Example 2), III = Grip strength (Experimental Example 3), IV = Wire hang (Experimental Example 4) a = the concentration administered and effect (%) compared to that of control treated with vehicle only Control 1: administered with vehicle only (Vehicle 1: 30% PEG400(Polyethylene Glycol 400)) Control 2: administered with vehicle only (Vehicle 2: 20% Tween 80) | | | | | |

## Claims

1. A compound or the pharmaceutically acceptable salt thereof selected from the group consisting of:
1-(2-chlorophenyl)-1-hydroxypropyl-2-carbamate,
1 -(2-chlorophenyl)-1 -hydroxy-3 -methyl-butyl-2-carbamate,
1-(2-chlorophenyl)-1-hydroxyhexyl-2-carbamate,
1-(2-chlorophenyl)-1-hydroxypropyl-2-N-methylcarbamate,
1-(2-chlorophenyl)-1-hydroxypropyl-2-N-propylcarbamate,
1-(2-chlorophenyl)-1-hydroxypropyl-2-N-isopropylcarbamate,
1-(2-chlorophenyl)-1-hydroxypropyl-2-N-cyclopropylcarbamate,
1-(2-chlorophenyl)-1-hydroxypropyl-2-N-cyclohexylcarbamate,
1-(2-chlorophenyl)-1-hydroxypropyl-2-N-benzylcarbamate,
1-(2-chlorophenyl)-1-hydroxypropyl-2-N-bicyclo[2,2,1]heptanecarbamate,
1-(2,4-dichlorophenyl)-1-hydroxypropyl-2-carbamate,
1-(2,6-dichlorophenyl)-1-hydroxypropyl-2-carbamate,
1-(2,4-dichlorophenyl)-1-hydroxy-3-methyl-butyl-2-carbamate,
1-(2,6-dichlorophenyl)-1-hydroxy-3-methyl-butyl-2-carbamate,
1-(2,4-dichlorophenyl)-1-hydroxyhexyl-2-carbamate,
1-(2,6-dichlorophenyl)-1-hydroxyhexyl-2-carbamate,
1-(2-chlorophenyl)-2-hydroxypropyl-1-carbamate,
1-(2-chlorophenyl)-2-hydroxypropyl-1-N-methylcarbamate,
1-(2-chlorophenyl)-2-hydroxypropyl-1-N-propylcarbamate,
1-(2-chlorophenyl)-2-hydroxypropyl-1-N-isopropylcarbamate,
1-(2-chlorophenyl)-2-hydroxypropyl-1-N-cyclopropylcarbamate,
1-(2-chlorophenyl)-2-hydroxypropyl-1-N-cyclohexylcarbamate,
1-(2-chlorophenyl)-2-hydroxypropyl-1-N-benzylcarbamate,
1-(2,4-dichlorophenyl)-2-hydroxypropyl-1-carbamate,
1-(2,6-dichlorophenyl)-2-hydroxypropyl-1-carbamate,
1-(2, 4-dichlorophenyl)-2-hydroxybutyl-1-carbamate
1-(2,4-dichlorophenyl)-2-hydroxy-3-methyl-butyl-1-carbamate,
1-(2,6-dichlorophenyl)-2-hydroxy-3-methyl-butyl-1-carbamate,
1-(2,4-dichlorophenyl)-2-hydroxyhexyl-1-carbamate,
1-(2,6-dichlorophenyl)-2-hydroxyhexyl-1-carbamate,
1-(2-fluorophenyl)-1-hydroxypropyl-2-carbamate,
1-(2-iodophenyl)-1-hydroxypropyl-2-carbamate,
1-(2-iodophenyl)-1-hydroxybutyl-2-carbamate, 1-(2,3-dichlorophenyl)-1-hydroxypropyl-2-carbamate, and
1-(2,3-dichlorophenyl)-2-hydroxypropyl-1-carbamate
wherein the compound is in the form of racemate, enantiomer, diastereomer, a mixture of enantiomer, or a mixture of diastereomer.

2. A compound or a pharmaceutically acceptable salt thereof according to Claim 1 selected from the group consisting of:
1-(2-chlorophenyl)-(S)-1-hydroxypropyl-(S)-2-carbamate,
1-(2-chlorophenyl)-(R)-1-hydroxypropyl-(R)-2-carbamate,
racemate of 1-(2-chlorophenyl)-(S)-1-hydroxypropyl-(S)-2-carbamate and 1-(2-chlorophenyl)-(R)-1-hydroxypropyl-(R)-2-carbamate,
1-(2-chlorophenyl)-(R)-1-hydroxypropyl-(S)-2-carbamate,
1-(2-chlorophenyl)-(S)-1-hydroxypropyl-(R)-2-carbamate,
1-(2-chlorophenyl)-(S)-1-hydroxy-3-methyl-butyl-(S)-2-carbamate,
racemate of 1-(2-chlorophenyl)-(S)-1-hydroxy-3-methyl-butyl-(S)-2-carbamate and 1-(2-chlorophenyl)-(R)-1 -hydroxy-3 -methyl-butyl-(R)-2-carbamate,
1-(2-chlorophenyl)-(S)-1-hydroxypropyl-(S)-2-N-methylcarbamate,
1-(2-chlorophenyl)-(S)-1-hydroxypropyl-(S)-2-N-propylcarbamate,
1-(2-chlorophenyl)-(S)-1-hydroxypropyl-(S)-2-N-isopropylcarbamate,
1-(2-chlorophenyl)-(S)-1-hydroxypropyl-(S)-2-N-cyclopropylcarbamate,
1-(2-chlorophenyl)-(S)-1-hydroxypropyl-(S)-2-N-cyclohexyl carbamate,
1-(2-chlorophenyl)-(R)-1-hydroxypropyl-(R)-2-N-methylcarbamate,
1-(2-chlorophenyl)-(R)-1-hydroxypropyl-(R)-2-N-propylcarbamate,
1-(2-chlorophenyl)-(R)-1-hydroxypropyl-(R)-2-N-isopropylcarbamate,
1-(2-chlorophenyl)-(R)-1-hydroxypropyl-(R)-2-N-cyclopropylcarbamate,
1-(2-chlorophenyl)-(R)-1-hydroxypropyl-(R)-2-N-cyclohexyl carbamate,
racemate of 1-(2-chlorophenyl)-(S)-1-hydroxypropyl-(S)-2-N-methylcarbamate and 1-(2-chlorophenyl)-(R)-1-hydroxypropyl-(R)-2-N-methylcarbamate
racemate of 1-(2-chlorophenyl)-(S)-1-hydroxypropyl-(S)-2-N-propylcarbamate and 1-(2-chlorophenyl)-(R)-1-hydroxypropyl-(R)-2-N-propylcarbamate,
racemate of 1-(2-chlorophenyl)-(S)-1-hydroxypropyl-(S)-2-N-isopropylcarbamate and 1-(2-chlorophenyl)-(R)-1-hydroxypropyl-(R)-2-N-isopropylcarbamate,
racemate of 1-(2-chlorophenyl)-(S)-1-hydroxypropyl-(S)-2-N-cyclopropylcarbamate and 1-(2-chlorophenyl)-(R)-1-hydroxypropyl-(R)-2-N-cyclopropylcarbamate,
racemate of 1-(2-chlorophenyl)-(S)-1-hydroxypropyl-(S)-2-N-cyclohexylcarbamate and 1-(2-chlorophenyl)-(R)-1-hydroxypropyl-(R)-2-N-cyclohexylcarbamate,
1-(2-fluorophenyl)-(S)-1-hydroxypropyl-(S)-2-carbamate,
1-(2-fluorophenyl)-(R)-1-hydroxypropyl-(R)-2-carbamate,
1-(2-iodophenyl)-(S)-1-hydroxypropyl-(S)-2-carbamate,
1-(2-iodophenyl)-(R)-1-hydroxypropyl-(R)-2-carbamate, and
1-(2-iodophenyl)-(S)-1-hydroxybutyl-(S)-2-carbamate.

3. A compound of Claim 1, wherein the compound is 1-(2-chlorophenyl)-1-hydroxypropyl-2-carbamate, or a pharmaceutically acceptable salt thereof.

4. A compound or a pharmaceutically acceptable salt thereof according to any proceeding claim for use in a method of muscle relaxation, wherein the compound is selected from the group consisting of:
1-(2-chlorophenyl)-(S)-1-hydroxypropyl-(S)-2-carbamate,
1-(2-chlorophenyl)-(R)-1-hydroxypropyl-(R)-2-carbamate,
Racemate of 1-(2-chlorophenyl)-(S)-1-hydroxypropyl-(S)-2-carbamate and 1-(2-chlorophenyl)-(R)-1-hydroxypropyl-(R)-2-carbamate,
1-(2-chlorophenyl)-(R)-1-hydroxypropyl-(S)-2-carbamate,
1-(2-chlorophenyl)-(S)-1-hydroxypropyl-(R)-2-carbamate,
1-(2-chlorophenyl)-(S)-1-hydroxy-3-methyl-butyl-(S)-2-carbamate,
Racemate of 1-(2-chlorophenyl)-(S)-1-hydroxy-3-methyl-butyl-(S)-2-carbamate and 1-(2-chlorophenyl)-(R)-1 -hydroxy-3 -methyl-butyl-(R)-2-carbamate,
1-(2-chlorophenyl)-(S)-1-hydroxypropyl-(S)-2-N-methylcarbamate,
1-(2-chlorophenyl)-(S)-1-hydroxypropyl-(S)-2-N-propylcarbamate,
1-(2-chlorophenyl)-(S)-1-hydroxypropyl-(S)-2-N-isopropylcarbamate,
1-(2-chlorophenyl)-(S)-1-hydroxypropyl-(S)-2-N-cyclopropylcarbamate,
1-(2-chlorophenyl)-(S)-1-hydroxypropyl-(S)-2-N-cyclohexyl carbamate,
1-(2-chlorophenyl)-(R)-1-hydroxypropyl-(R)-2-N-methylcarbamate,
1-(2-chlorophenyl)-(R)-1-hydroxypropyl-(R)-2-N-propylcarbamate,
1-(2-chlorophenyl)-(R)-1-hydroxypropyl-(R)-2-N-isopropylcarbamate,
1-(2-chlorophenyl)-(R)-1-hydroxypropyl-(R)-2-N-cyclopropylcarbamate,
1-(2-chlorophenyl)-(R)-1-hydroxypropyl-(R)-2-N-cyclohexyl carbamate,
Racemate of 1-(2-chlorophenyl)-(S)-1-hydroxypropyl-(S)-2-N-methylcarbamate and 1-(2-chlorophenyl)-(R)-1-hydroxypropyl-(R)-2-N-methylcarbamate,
Racemate of 1-(2-chlorophenyl)-(S)-1-hydroxypropyl-(S)-2-N-propylcarbamate and 1-(2-chlorophenyl)-(R)-1-hydroxypropyl-(R)-2-N-propylcarbamate,
Racemate of 1-(2-chlorophenyl)-(S)-1-hydroxypropyl-(S)-2-N-isopropylcarbamate and 1-(2-chlorophenyl)-(R)-1-hydroxypropyl-(R)-2-N-isopropylcarbamate,
Racemate of 1-(2-chlorophenyl)-(S)-1-hydroxypropyl-(S)-2-N-cyclopropylcarbamate and 1-(2-chlorophenyl)-(R)-1-hydroxypropyl-(R)-2-N-cyclopropylcarbamate,
Racemate of 1-(2-chlorophenyl)-(S)-1-hydroxypropyl-(S)-2-N-cyclohexylcarbamate and 1-(2-chlorophenyl)-(R)-1-hydroxypropyl-(R)-2-N-cyclohexylcarbamate,
1-(2-fluorophenyl)-(S)-1-hydroxypropyl-(S)-2-carbamate,
1-(2-fluorophenyl)-(R)-1-hydroxypropyl-(R)-2-carbamate,
1-(2-iodophenyl)-(S)-1-hydroxypropyl-(S)-2-carbamate,
1-(2-iodophenyl)-(R)-1-hydroxypropyl-(R)-2-carbamate, and
1-(2-iodophenyl)-(S)-1-hydroxybutyl-(S)-2-carbamate.

5. A compound or a pharmaceutically acceptable salt thereof according to Claim 4 for use in a method of muscle relaxation, wherein the compound is 1-(2-chlorophenyl)-(S)-1-hydroxypropyl-(S)-2-carbamate, or a pharmaceutically acceptable salt thereof.

6. A compound or a pharmaceutically acceptable salt thereof according to any of Claims 1 to 3 for use in a method of treating a disease associated with muscle spasms selected from the group consisting of herniation of the vertebral disc, vascular disorders of the spinal cord, spastic spinal paralysis, cervical spondylosiscerebral palsy, sequelae of injuries, and spinocerebellar degeneration and
wherein the compound is selected from the group consisting of:
1-(2-chlorophenyl)-(S)-1-hydroxypropyl-(S)-2-carbamate,
1-(2-chlorophenyl)-(R)-1-hydroxypropyl-(R)-2-carbamate,
Racemate of 1-(2-chlorophenyl)-(S)-1-hydroxypropyl-(S)-2-carbamate and 1-(2-chlorophenyl)-(R)-1-hydroxypropyl-(R)-2-carbamate,
1-(2-chlorophenyl)-(R)-1-hydroxypropyl-(S)-2-carbamate,
1-(2-chlorophenyl)-(S)-1-hydroxypropyl-(R)-2-carbamate,
1-(2-chlorophenyl)-(S)-1-hydroxy-3-methyl-butyl-(S)-2-carbamate,
Racemate of 1-(2-chlorophenyl)-(S)-1-hydroxy-3-methyl-butyl-(S)-2-carbamate and 1-(2-chlorophenyl)-(R)-1 -hydroxy-3 -methyl-butyl-(R)-2-carbamate,
1-(2-chlorophenyl)-(S)-1-hydroxypropyl-(S)-2-N-methylcarbamate,
1-(2-chlorophenyl)-(S)-1-hydroxypropyl-(S)-2-N-propylcarbamate,
1-(2-chlorophenyl)-(S)-1-hydroxypropyl-(S)-2-N-isopropylcarbamate,
1-(2-chlorophenyl)-(S)-1-hydroxypropyl-(S)-2-N-cyclopropylcarbamate,
1-(2-chlorophenyl)-(S)-1-hydroxypropyl-(S)-2-N-cyclohexyl carbamate,
1-(2-chlorophenyl)-(R)-1-hydroxypropyl-(R)-2-N-methylcarbamate,
1-(2-chlorophenyl)-(R)-1-hydroxypropyl-(R)-2-N-propylcarbamate,
1-(2-chlorophenyl)-(R)-1-hydroxypropyl-(R)-2-N-isopropylcarbamate,
1-(2-chlorophenyl)-(R)-1-hydroxypropyl-(R)-2-N-cyclopropylcarbamate,
1-(2-chlorophenyl)-(R)-1-hydroxypropyl-(R)-2-N-cyclohexyl carbamate,
Racemate of 1-(2-chlorophenyl)-(S)-1-hydroxypropyl-(S)-2-N-methylcarbamate and 1-(2-chlorophenyl)-(R)-1-hydroxypropyl-(R)-2-N-methylcarbamate,
Racemate of 1-(2-chlorophenyl)-(S)-1-hydroxypropyl-(S)-2-N-propylcarbamate and 1-(2-chlorophenyl)-(R)-1-hydroxypropyl-(R)-2-N-propylcarbamate,
Racemate of 1-(2-chlorophenyl)-(S)-1-hydroxypropyl-(S)-2-N-isopropylcarbamate and 1-(2-chlorophenyl)-(R)-1-hydroxypropyl-(R)-2-N-isopropylcarbamate,
Racemate of 1-(2-chlorophenyl)-(S)-1-hydroxypropyl-(S)-2-N-cyclopropylcarbamate and 1-(2-chlorophenyl)-(R)-1-hydroxypropyl-(R)-2-N-cyclopropylcarbamate,
Racemate of 1-(2-chlorophenyl)-(S)-1-hydroxypropyl-(S)-2-N-cyclohexylcarbamate and 1-(2-chlorophenyl)-(R)-1-hydroxypropyl-(R)-2-N-cyclohexylcarbamate,
1-(2-fluorophenyl)-(S)-1-hydroxypropyl-(S)-2-carbamate,
1-(2-fluorophenyl)-(R)-1-hydroxypropyl-(R)-2-carbamate,
1-(2-iodophenyl)-(S)-1-hydroxypropyl-(S)-2-carbamate,
1-(2-iodophenyl)-(R)-1-hydroxypropyl-(R)-2-carbamate, and
1-(2-iodophenyl)-(S)-1-hydroxybutyl-(S)-2-carbamate.

7. A compound or a pharmaceutically acceptable salt thereof according to Claim 6 for use in a method of treatment according to Claim 6, wherein the compound is 1-(2-chlorophenyl)-(S)-1-hydroxypropyl-(S)-2-carbamate, or a pharmaceutically acceptable salt thereof.

8. A pharmaceutical composition comprising a compound or pharmaceutically acceptable salt thereof according to any of Claims 1 to 3 and one or more pharmaceutically acceptable components.

## Patentansprüche

1. Verbindung, oder das pharmazeutisch verträgliche Salz davon, die aus der Gruppe ausgewählt ist, die aus Folgenden besteht:
1-(2-Chlorphenyl)-1-hydroxypropyl-2-carbamat,
1-(2-Chlorphenyl)-1-hydroxy-3-methylbutyl-2-carbamat,
1-(2-Chlorphenyl)-1-hydroxyhexyl-2-carbamat,
1-(2-Chlorphenyl)-1-hydroxypropyl-2-N-methylcarbamat,
1-(2-Chlorphenyl)-1-hydroxypropyl-2-N-propylcarbamat,
1-(2-Chlorphenyl)-1-hydroxypropyl-2-N-isopropylcarbamat,
1-(2-Chlorphenyl)-1-hydroxypropyl-2-N-cyclopropylcarbamat,
1-(2-Chlorphenyl)-1-hydroxypropyl-2-N-cyclohexylcarbamat,
1-(2-Chlorphenyl)-1-hydroxypropyl-2-N-benzylcarbamat,
1-(2-Chlorphenyl)-1-hydroxypropyl-2-N-bicyclo[2.2.1]heptancarbamat,
1-(2,4-Dichlorphenyl)-1-hydroxypropyl-2-carbamat,
1-(2,6-Dichlorphenyl)-1-hydroxypropyl-2-carbamat,
1-(2,4-Dichlorphenyl)-1-hydroxy-3-methylbutyl-2-carbamat,
1-(2,6-Dichlorphenyl)-1-hydroxy-3-methylbutyl-2-carbamat,
1-(2,4-Dichlorphenyl)-1-hydroxyhexyl-2-carbamat,
1-(2,6-Dichlorphenyl)-1-hydroxyhexyl-2-carbamat,
1-(2-Chlorphenyl)-2-hydroxypropyl-1-carbamat,
1-(2-Chlorphenyl)-2-hydroxypropyl-1-N-methylcarbamat,
1-(2-Chlorphenyl)-2-hydroxypropyl-1-N-propylcarbamat,
1-(2-Chlorphenyl)-2-hydroxypropyl-1-N-isopropylcarbamat,
1-(2-Chlorphenyl)-2-hydroxypropyl-1-N-cyclopropylcarbamat,
1-(2-Chlorphenyl)-2-hydroxypropyl-1-N-cyclohexylcarbamat,
1-(2-Chlorphenyl)-2-hydroxypropyl-1-N-benzylcarbamat,
1-(2,4-Dichlorphenyl)-2-hydroxypropyl-1-carbamat,
1-(2,6-Dichlorphenyl)-2-hydroxypropyl-1-carbamat,
1-(2,4-Dichlorphenyl)-2-hydroxybutyl-1-carbamat,
1-(2,4-Dichlorphenyl)-2-hydroxy-3-methylbutyl-1-carbamat,
1-(2,6-Dichlorphenyl)-2-hydroxy-3-methylbutyl-1-carbamat,
1-(2,4-Dichlorphenyl)-2-hydroxyhexyl-1-carbamat,
1-(2,6-Dichlorphenyl)-2-hydroxyhexyl-1-carbamat,
1-(2-Fluorphenyl)-1-hydroxypropyl-2-carbamat,
1-(2-Iodphenyl)-1-hydroxypropyl-2-carbamat,
1-(2-Iodphenyl)-1-hydroxybutyl-2-carbamat,
1-(2,3-Dichlorphenyl)-1-hydroxypropyl-2-carbamat und
1-(2,3-Dichlorphenyl)-2-hydroxypropyl-1-carbamat,
wobei die Verbindung in Form von Racemat, Enantiomer, Diastereomer, einer Mischung von Enantiomeren oder einer Mischung von Diastereomeren vorliegt.

2. Verbindung, oder ein pharmazeutisch verträgliches Salz davon, nach Anspruch 1, die aus der Gruppe ausgewählt ist, die aus Folgenden besteht:
1-(2-Chlorphenyl)-(S)-1-hydroxypropyl-(S)-2-carbamat,
1-(2-Chlorphenyl)-(R)-1-hydroxypropyl-(R)-2-carbamat,
Racemat von 1-(2-Chlorphenyl)-(S)-1-hydroxypropyl-(S)-2-carbamat und 1-(2-Chlorphenyl)-(R)-1-hydroxypropyl-(R)-2-carbamat,
1-(2-Chlorphenyl)-(R)-1-hydroxypropyl-(S)-2-carbamat,
1-(2-Chlorphenyl)-(S)-1-hydroxypropyl-(R)-2-carbamat,
1-(2-Chlorphenyl)-(S)-1-hydroxy-3-methylbutyl-(S)-2-carbamat,
Racemat von 1-(2-Chlorphenyl)-(S)-1-hydroxy-3-methylbutyl-(S)-2-carbamat und 1-(2-Chlorphenyl)-(R)-1-hydroxy-3-methylbutyl-(R)-2-carbamat,
1-(2-Chlorphenyl)-(S)-1-hydroxypropyl-(S)-2-N-methylcarbamat,
1-(2-Chlorphenyl)-(S)-1-hydroxypropyl-(S)-2-N-propylcarbamat,
1-(2-Chlorphenyl)-(S)-1-hydroxypropyl-(S)-2-N-isopropylcarbamat,
1-(2-Chlorphenyl)-(S)-1-hydroxypropyl-(S)-2-N-cyclopropylcarbamat,
1-(2-Chlorphenyl)-(S)-1-hydroxypropyl-(S)-2-N-cyclohexylcarbamat,
1-(2-Chlorphenyl)-(R)-1-hydroxypropyl-(R)-2-N-methylcarbamat,
1-(2-Chlorphenyl)-(R)-1-hydroxypropyl-(R)-2-N-propylcarbamat,
1-(2-Chlorphenyl)-(R)-1-hydroxypropyl-(R)-2-N-isopropylcarbamat,
1-(2-Chlorphenyl)-(R)-1-hydroxypropyl-(R)-2-N-cyclopropylcarbamat,
1-(2-Chlorphenyl)-(R)-1-hydroxypropyl-(R)-2-N-cyclohexylcarbamat,
Racemat von 1-(2-Chlorphenyl)-(S)-1-hydroxypropyl-(S)-2-N-methylcarbamat und 1-(2-Chlorphenyl)-(R)-1-hydroxypropyl-(R)-2-N-methylcarbamat,
Racemat von 1-(2-Chlorphenyl)-(S)-l-hydroxypropyl-(S)-2-N-propylcarbamat und 1-(2-Chlorphenyl)-(R)-1-hydroxypropyl-(R)-2-N-propylcarbamat,
Racemat von 1-(2-Chlorphenyl)-(S)-1-hydroxypropyl-(S)-2-N-isopropylcarbamat und 1-(2-Chlorphenyl)-(R)-1-hydroxypropyl-(R)-2-N-isopropylcarbamat,
Racemat von 1-(2-Chlorphenyl)-(S)-1-hydroxypropyl-(S)-2-N-cyclopropylcarbamat und 1-(2-Chlorphenyl)-(R)-1-hydroxypropyl-(R)-2-N-cyclopropylcarbamat,
Racemat von 1-(2-Chlorphenyl)-(S)-1-hydroxypropyl-(S)-2-N-cyclohexylcarbamat und 1-(2-Chlorphenyl)-(R)-1-hydroxypropyl-(R)-2-N-cyclohexylcarbamat,
1-(2-Fluorphenyl)-(S)-1-hydroxypropyl-(S)-2-carbamat,
1-(2-Fluorphenyl)-(R)-1-hydroxypropyl-(R)-2-carbamat,
1-(2-Iodphenyl)-(S)-1-hydroxypropyl-(S)-2-carbamat,
1-(2-Iodphenyl)-(R)-1-hydroxypropyl-(R)-2-carbamat und
1-(2-Iodphenyl)-(S)-1-hydroxybutyl-(S)-2-carbamat.

3. Verbindung nach Anspruch 1, wobei die Verbindung 1-(2-Chlorphenyl)-1-hydroxypropyl-2-carbamat oder ein pharmazeutisch verträgliches Salz davon ist.

4. Verbindung oder ein pharmazeutisch verträgliches Salz davon nach einem vorstehenden Anspruch für die Verwendung in einem Verfahren zur Muskelrelaxation, wobei die Verbindung aus der Gruppe ausgewählt ist, die aus Folgenden besteht:
1-(2-Chlorphenyl)-(S)-1-hydroxypropyl-(S)-2-carbamat,
1-(2 -Chlorphenyl)-(R)-1-hydroxypropyl-(R)-2-carbamat,
Racemat von 1-(2-Chlorphenyl)-(S)-1-hydroxypropyl-(S)-2-carbamat und 1-(2-Chlorphenyl)-(R)-1-hydroxypropyl-(R)-2-carbamat,
1-(2-Chlorphenyl)-(R)-1-hydroxypropyl-(S)-2-carbamat,
1-(2-Chlorphenyl)-(S)-1-hydroxypropyl-(R)-2-carbamat,
1-(2-Chlorphenyl)-(S)-1-hydroxy-3-methylbutyl-(S)-2-carbamat,
Racemat von 1-(2-Chlorphenyl)-(S)-1-hydroxy-3-methylbutyl-(S)-2-carbamat und 1-(2-Chlorphenyl)-(R)-1-hydroxy-3-methylbutyl-(R)-2-carbamat,
1-(2-Chlorphenyl)-(S)-1-hydroxypropyl-(S)-2-N-methylcarbamat,
1-(2-Chlorphenyl)-(S)-1-hydroxypropyl-(S)-2-N-propylcarbamat,
1-(2-Chlorphenyl)-(S)-1-hydroxypropyl-(S)-2-N-isopropylcarbamat,
1-(2-Chlorphenyl)-(S)-1-hydroxypropyl-(S)-2-N-cyclopropylcarbamat,
1-(2-Chlorphenyl)-(S)-1-hydroxypropyl-(S)-2-N-cyclohexylcarbamat,
1-(2-Chlorphenyl)-(R)-1-hydroxypropyl-(R)-2-N-methylcarbamat,
1-(2-Chlorphenyl)-(R)-1-hydroxypropyl-(R)-2-N-propylcarbamat,
1-(2-Chlorphenyl)-(R)-1-hydroxypropyl-(R)-2-N-isopropylcarbamat,
1-(2-Chlorphenyl)-(R)-1 -hydroxypropyl-(R)-2-N-cyclopropylcarbamat,
1-(2-Chlorphenyl)-(R)-1-hydroxypropyl-(R)-2-N-cyclohexyl carbamat,
Racemat von 1-(2-Chlorphenyl)-(S)-1-hydroxypropyl-(S)-2-N-methylcarbamat und 1-(2-Chlorphenyl)-(R)-1-hydroxypropyl-(R)-2-N-methylcarbamat,
Racemat von 1-(2-Chlorphenyl)-(S)-1-hydroxypropyl-(S)-2-N-propylcarbamat und 1-(2-Chlorphenyl)-(R)-1-hydroxypropyl-(R)-2-N-propylcarbamat,
Racemat von 1-(2-Chlorphenyl)-(S)-1 -hydroxypropyl-(S)-2-N-isopropylcarbamat und 1-(2-Chlorphenyl)-(R)-1-hydroxypropyl-(R)-2-N-isopropylcarbamat,
Racemat von 1-(2-Chlorphenyl)-(S)-1-hydroxypropyl-(S)-2-N-cyclopropylcarbamat und 1-(2-Chlorphenyl)-(R)-1-hydroxypropyl-(R)-2-N-cyclopropylcarbamat,
Racemat von 1-(2-Chlorphenyl)-(S)-1-hydroxypropyl-(S)-2-N-cyclohexylcarbamat und 1-(2-Chlorphenyl)-(R)-1-hydroxypropyl-(R)-2-N-cyclohexylcarbamat,
1-(2-Fluorphenyl)-(S)-1-hydroxypropyl-(S)-2-carbamat,
1-(2-Fluorphenyl)-(R)-1-hydroxypropyl-(R)-2-carbamat,
1 -(2-Iodphenyl)-(S)-1-hydroxypropyl-(S)-2-carbamat,
1-(2-Iodphenyl)-(R)-1-hydroxypropyl-(R)-2-carbamat und
1-(2-Iodphenyl)-(S)-1-hydroxybutyl-(S)-2-carbamat.

5. Verbindung oder ein pharmazeutisch verträgliches Salz davon nach Anspruch 4 für die Verwendung in einem Verfahren zur Muskelrelaxation, wobei die Verbindung 1-(2-Chlorphenyl)-(S)-1-hydroxypropyl-(S)-2-carbamat oder ein pharmazeutisch verträgliches Salz davon ist.

6. Verbindung oder ein pharmazeutisch verträgliches Salz davon nach einem der Ansprüche 1 bis 3 für die Verwendung in einem Verfahren zur Behandlung einer mit Muskelkrampf verbundenen Erkrankung, die aus der Gruppe ausgewählt ist, die aus Folgenden besteht: Herniation der Bandscheibe, Gefäßerkrankungen des Rückenmarks, spastischer Spinalparalyse, zervikaler Spondylose, Zerebralparese, Folgen von Verletzungen und spinozerebellärer Degeneration, und
wobei die Verbindung aus der Gruppe ausgewählt ist, die aus Folgenden besteht:
1-(2-Chlorphenyl)-(S)-1-hydroxypropyl-(S)-2-carbamat,
1-(2-Chlorphenyl)-(R)-1-hydroxypropyl-(R)-2-carbamat,
Racemat von 1-(2-Chlorphenyl)-(S)-1-hydroxypropyl-(S)-2-carbamat und 1-(2-Chlorphenyl)-(R)-1-hydroxypropyl-(R)-2-carbamat,
1-(2-Chlorphenyl)-(R)-1-hydroxypropyl-(S)-2-carbamat,
1-(2-Chlorphenyl)-(S)-1-hydroxypropyl-(R)-2-carbamat,
1-(2-Chlorphenyl)-(S)-1-hydroxy-3-methylbutyl-(S)-2-carbamat,
Racemat von 1-(2-Chlorphenyl)-(S)-1-hydroxy-3-methylbutyl-(S)-2-carbamat und 1-(2-Chlorphenyl)-(R)-1-hydroxy-3-methylbutyl-(R)-2-carbamat,
1-(2-Chlorphenyl)-(S)-1 -hydroxypropyl-(S)-2-N-methylcarbamat,
1-(2-Chlorphenyl)-(S)-1-hydroxypropyl-(S)-2-N-propylcarbamat,
1-(2-Chlorphenyl)-(S)-1-hydroxypropyl-(S)-2-N-isopropylcarbamat,
1-(2-Chlorphenyl)-(S)-1-hydroxypropyl-(S)-2-N-cyclopropylcarbamat,
1-(2-Chlorphenyl)-(S)-1-hydroxypropyl-(S)-2-N-cyclohexylcarbamat,
1-(2-Chlorphenyl)-(R)-1-hydroxypropyl-(R)-2-N-methylcarbamat,
1-(2-Chlorphenyl)-(R)-1-hydroxypropyl-(R)-2-N-propylcarbamat,
1-(2-Chlorphenyl)-(R)-1-hydroxypropyl-(R)-2-N-isopropylcarbamat,
1-(2-Chlorphenyl)-(R)-1-hydroxypropyl-(R)-2-N-cyclopropylcarbamat,
1-(2-Chlorphenyl)-(R)-1-hydroxypropyl-(R)-2-N-cyclohexylcarbamat,
Racemat von 1-(2-Chlorphenyl)-(S)-1 -hydroxypropyl-(S)-2-N-methylcarbamat und 1-(2-Chlorphenyl)-(R)-1-hydroxypropyl-(R)-2-N-methylcarbamat,
Racemat von 1-(2-Chlorphenyl)-(S)-1 -hydroxypropyl-(S)-2-N-propylcarbamat und 1-(2-Chlorphenyl)-(R)-1-hydroxypropyl-(R)-2-N-propylcarbamat,
Racemat von 1-(2-Chlorphenyl)-(S)-1-hydroxypropyl-(S)-2-N-isopropylcarbamat und 1-(2-Chlorphenyl)-(R)-1-hydroxypropyl-(R)-2-N-isopropylcarbamat,
Racemat von 1-(2-Chlorphenyl)-(S)-1-hydroxypropyl-(S)-2-N-cyclopropylcarbamat und 1-(2-Chlorphenyl)-(R)-1-hydroxypropyl-(R)-2-N-cyclopropylcarbamat,
Racemat von 1-(2-Chlorphenyl)-(S)-1-hydroxypropyl-(S)-2-N-cyclohexylcarbamat und 1-(2-Chlorphenyl)-(R)-1-hydroxypropyl-(R)-2-N-cyclohexylcarbamat,
1-(2-Fluorphenyl)-(S)-1-hydroxypropyl-(S)-2-carbamat,
1-(2-Fluorphenyl)-(R)-1-hydroxypropyl-(R)-2-carbamat,
1-(2-Iodphenyl)-(S)-1-hydroxypropyl-(S)-2-carbamat,
1-(2-Iodphenyl)-(R)-1-hydroxypropyl-(R)-2-carbamat und
1-(2-Iodphenyl)-(S)-1-hydroxybutyl-(S)-2-carbamat.

7. Verbindung oder ein pharmazeutisch verträgliches Salz davon nach Anspruch 6 für die Verwendung in einem Verfahren zur Behandlung nach Anspruch 6, wobei die Verbindung 1-(2-Chlorphenyl)-(S)-1-hydroxypropyl-(S)-2-carbamat oder ein pharmazeutisch verträgliches Salz davon ist.

8. Pharmazeutische Zusammensetzung, die eine Verbindung oder ein pharmazeutisch verträgliches Salz davon nach einem der Ansprüche 1 bis 3 und eine oder mehrere pharmazeutisch verträgliche Komponenten umfasst.

## Revendications

1. Composé ou le sel pharmaceutiquement acceptable de celui-ci, choisi dans le groupe constitué par :
le 1-(2-chlorophényl)-1-hydroxypropyl-2-carbamate,
le 1-(2-chlorophényl)-1-hydroxy-3-méthylbutyl-2-carbamate,
le 1-(2-chlorophényl)-1-hydroxyhexyl-2-carbamate,
le 1-(2-chlorophényl)-1-hydroxypropyl-2-N-méthylcarbamate,
le 1-(2-chlorophényl)-1-hydroxypropyl-2-N-propylcarbamate,
le 1-(2-chlorophényl)-1-hydroxypropyl-2-N-isopropylcarbamate,
le 1-(2-chlorophényl)-1-hydroxypropyl-2-N-cyclopropylcarbamate,
le 1-(2-chlorophényl)-1-hydroxypropyl-2-N-cyclohexylcarbamate,
le 1-(2-chlorophényl)-1-hydroxypropyl-2-N-benzylcarbamate,
le 1-(2-chlorophényl)-1-hydroxypropyl-2-N-bicyclo[2,2,1]heptanecarbamate,
le 1-(2,4-dichlorophényl)-1-hydroxypropyl-2-carbamate,
le 1-(2,6-dichlorophényl)-1-hydroxypropyl-2-carbamate,
le 1-(2,4-dichlorophényl)-1-hydroxy-3-méthylbutyl-2-carbamate,
le 1-(2,6-dichlorophényl)-1-hydroxy-3-méthylbutyl-2-carbamate,
le 1-(2,4-dichlorophényl)-1-hydroxyhexyl-2-carbamate,
le 1-(2,6-dichlorophényl)-1-hydroxyhexyl-2-carbamate,
le 1-(2-chlorophényl)-2-hydroxypropyl-1-carbamate,
le 1-(2-chlorophényl)-2-hydroxypropyl-1-N-méthylcarbamate,
le 1-(2-chlorophényl)-2-hydroxypropyl-1-N-propylcarbamate,
le 1-(2-chlorophényl)-2-hydroxypropyl-1-N-isopropylcarbamate,
le 1-(2-chlorophényl)-2-hydroxypropyl-1-N-cyclopropylcarbamate,
le 1-(2-chlorophényl)-2-hydroxypropyl-1-N-cyclohexylcarbamate,
le 1-(2-chlorophényl)-2-hydroxypropyl-1-N-benzylcarbamate,
le 1-(2,4-dichlorophényl)-2-hydroxypropyl-1-carbamate,
le 1-(2,6-dichlorophényl)-2-hydroxypropyl-1-carbamate,
le 1-(2,4-dichlorophényl)-2-hydroxybutyl-1-carbamate,
le 1-(2,4-dichlorophényl)-2-hydroxy-3-méthylbutyl-1-carbamate,
le 1-(2,6-dichlorophényl)-2-hydroxy-3-méthylbutyl-1-carbamate,
le 1-(2,4-dichlorophényl)-2-hydroxyhexyl-1-carbamate,
le 1-(2,6-dichlorophényl)-2-hydroxyhexyl-1-carbamate,
le 1-(2-fluorophényl)-1-hydroxypropyl-2-carbamate,
le 1-(2-iodophényl)-1-hydroxypropyl-2-carbamate,
le 1-(2-iodophényl)-1-hydroxybutyl-2-carbamate,
le 1-(2,3-dichlorophényl)-1-hydroxypropyl-2-carbamate, et
le 1-(2,3-dichlorophényl)-2-hydroxypropyl-1-carbamate
où le composé se trouve sous la forme d'un racémate, d'un énantiomère, d'un diastéréoisomère, d'un mélange d'énantiomères, ou d'un mélange de diastéréoisomères.

2. Composé ou un sel pharmaceutiquement acceptable de celui-ci selon la revendication 1, choisi dans le groupe constitué par :
le 1-(2-chlorophényl)-(S)-1-hydroxypropyl-(S)-2-carbamate,
le 1-(2-chlorophényl)-(R)-1-hydroxypropyl-(R)-2-carbamate,
le racémate de 1-(2-chlorophényl)-(S)-1-hydroxypropyl-(S)-2-carbamate et de 1-(2-chlorophényl)-(R)-1-hydroxypropyl-(R)-2-carbamate,
le 1-(2-chlorophényl)-(R)-1-hydroxypropyl-(S)-2-carbamate,
le 1-(2-chlorophényl)-(S)-1-hydroxypropyl-(R)-2-carbamate,
le 1-(2-chlorophényl)-(S)-1-hydroxy-3-méthylbutyl-(S)-2-carbamate,
le racémate de 1-(2-chlorophényl)-(S)-1-hydroxy-3-méthylbutyl-(S)-2-carbamate et de 1-(2-chlorophényl)-(R)-1-hydroxy-3-méthylbutyl-(R)-2-carbamate,
le 1-(2-chlorophényl)-(S)-1-hydroxypropyl-(S)-2-N-méthylcarbamate,
le 1-(2-chlorophényl)-(S)-1-hydroxypropyl-(S)-2-N-propylcarbamate,
le 1-(2-chlorophényl)-(S)-1-hydroxypropyl-(S)-2-N-isopropylcarbamate,
le 1-(2-chlorophényl)-(S)-1-hydroxypropyl-(S)-2-N-cyclopropylcarbamate,
le 1-(2-chlorophényl)-(S)-1-hydroxypropyl-(S)-2-N-cyclohexylcarbamate,
le 1-(2-chlorophényl)-(R)-1-hydroxypropyl-(R)-2-N-méthylcarbamate,
le 1-(2-chlorophényl)-(R)-1-hydroxypropyl-(R)-2-N-propylcarbamate,
le 1-(2-chlorophényl)-(R)-1-hydroxypropyl-(R)-2-N-isopropylcarbamate,
le 1-(2-chlorophényl)-(R)-1-hydroxypropyl-(R)-2-N-cyclopropylcarbamate,
le 1-(2-chlorophényl)-(R)-1-hydroxypropyl-(R)-2-N-cyclohexylcarbamate,
le racémate de 1-(2-chlorophényl)-(S)-1-hydroxypropyl-(S)-2-N-méthylcarbamate et de 1-(2-chlorophényl)-(R)-1-hydroxypropyl-(R)-2-N-méthylcarbamate,
le racémate de 1-(2-chlorophényl)-(S)-1-hydroxypropyl-(S)-2-N-propylcarbamate et de 1-(2-chlorophényl)-(R)-1-hydroxypropyl-(R)-2-N-propylcarbamate,
le racémate de 1-(2-chlorophényl)-(S)-1-hydroxypropyl-(S)-2-N-isopropylcarbamate et
de 1-(2-chlorophényl)-(R)-1-hydroxypropyl-(R)-2-N-isopropylcarbamate,
le racémate de 1-(2-chlorophényl)-(S)-1-hydroxypropyl-(S)-2-N-cyclopropylcarbamate et de 1-(2-chlorophényl)-(R)-1-hydroxypropyl-(R)-2-N-cyclopropylcarbamate,
le racémate de 1-(2-chlorophényl)-(S)-1-hydroxypropyl-(S)-2-N-cyclohexylcarbamate et de 1-(2-chlorophényl)-(R)-1-hydroxypropyl-(R)-2-N-cyclohexylcarbamate,
le 1-(2-fluorophényl)-(S)-1-hydroxypropyl-(S)-2-carbamate,
le 1-(2-fluorophényl)-(R)-1-hydroxypropyl-(R)-2-carbamate,
le 1-(2-iodophényl)-(S)-1-hydroxypropyl-(S)-2-carbamate,
le 1-(2-iodophényl)-(R)-1-hydroxypropyl-(R)-2-carbamate, et
le 1-(2-iodophényl)-(S)-1-hydroxybutyl-(S)-2-carbamate.

3. Composé selon la revendication 1, où le composé est le 1-(2-chlorophényl)-1-hydroxypropyl-2-carbamate, ou un sel pharmaceutiquement acceptable de celui-ci.

4. Composé ou un sel pharmaceutiquement acceptable de celui-ci selon l'une quelconque des revendications précédentes, pour une utilisation dans une méthode de myorelaxation, où le composé est choisi dans le groupe constitué par :
le 1-(2-chlorophényl)-(S)-1-hydroxypropyl-(S)-2-carbamate,
le 1-(2-chlorophényl)-(R)-1-hydroxypropyl-(R)-2-carbamate,
le racémate de 1-(2-chlorophényl)-(S)-1-hydroxypropyl-(S)-2-carbamate et de 1-(2-chlorophényl)-(R)-1-hydroxypropyl-(R)-2-carbamate,
le 1-(2-chlorophényl)-(R)-1-hydroxypropyl-(S)-2-carbamate,
le 1-(2-chlorophényl)-(S)-1-hydroxypropyl-(R)-2-carbamate,
le 1-(2-chlorophényl)-(S)-1-hydroxy-3-méthylbutyl-(S)-2-carbamate,
le racémate de 1-(2-chlorophényl)-(S)-1-hydroxy-3-méthylbutyl-(S)-2-carbamate et de 1-(2-chlorophényl)-(R)-1-hydroxy-3-méthylbutyl-(R)-2-carbamate,
le 1-(2-chlorophényl)-(S)-1-hydroxypropyl-(S)-2-N-méthylcarbamate,
le 1-(2-chlorophényl)-(S)-1-hydroxypropyl-(S)-2-N-propylcarbamate,
le 1-(2-chlorophényl)-(S)-1-hydroxypropyl-(S)-2-N-isopropylcarbamate,
le 1-(2-chlorophényl)-(S)-1-hydroxypropyl-(S)-2-N-cyclopropylcarbamate,
le 1-(2-chlorophényl)-(S)-1-hydroxypropyl-(S)-2-N-cyclohexylcarbamate,
le 1-(2-chlorophényl)-(R)-1-hydroxypropyl-(R)-2-N-méthylcarbamate,
le 1-(2-chlorophényl)-(R)-1-hydroxypropyl-(R)-2-N-propylcarbamate,
le 1-(2-chlorophényl)-(R)-1-hydroxypropyl-(R)-2-N-isopropylcarbamate,
le 1-(2-chlorophényl)-(R)-1-hydroxypropyl-(R)-2-N-cyclopropylcarbamate,
le 1-(2-chlorophényl)-(R)-1-hydroxypropyl-(R)-2-N-cyclohexylcarbamate,
le racémate de 1-(2-chlorophényl)-(S)-1-hydroxypropyl-(S)-2-N-méthylcarbamate et de 1-(2-chlorophényl)-(R)-1-hydroxypropyl-(R)-2-N-méthylcarbamate,
le racémate de 1-(2-chlorophényl)-(S)-1-hydroxypropyl-(S)-2-N-propylcarbamate et de 1-(2-chlorophényl)-(R)-1-hydroxypropyl-(R)-2-N-propylcarbamate,
le racémate de 1-(2-chlorophényl)-(S)-1-hydroxypropyl-(S)-2-N-isopropylcarbamate et
de 1-(2-chlorophényl)-(R)-1-hydroxypropyl-(R)-2-N-isopropylcarbamate,
le racémate de 1-(2-chlorophényl)-(S)-1-hydroxypropyl-(S)-2-N-cyclopropylcarbamate et de 1-(2-chlorophényl)-(R)-1-hydroxypropyl-(R)-2-N-cyclopropylcarbamate,
le racémate de 1-(2-chlorophényl)-(S)-1-hydroxypropyl-(S)-2-N-cyclohexylcarbamate et de 1-(2-chlorophényl)-(R)-1-hydroxypropyl-(R)-2-N-cyclohexylcarbamate,
le 1-(2-fluorophényl)-(S)-1-hydroxypropyl-(S)-2-carbamate,
le 1-(2-fluorophényl)-(R)-1-hydroxypropyl-(R)-2-carbamate,
le 1-(2-iodophényl)-(S)-1-hydroxypropyl-(S)-2-carbamate,
le 1-(2-iodophényl)-(R)-1-hydroxypropyl-(R)-2-carbamate, et
le 1-(2-iodophényl)-(S)-1-hydroxybutyl-(S)-2-carbamate.

5. Composé ou un sel pharmaceutiquement acceptable de celui-ci selon la revendication 4, pour une utilisation dans une méthode de myorelaxation, où le composé est le 1-(2-chlorophényl)-(S)-1-hydroxypropyl-(S)-2-carbamate, ou un sel pharmaceutiquement acceptable de celui-ci.

6. Composé ou un sel pharmaceutiquement acceptable de celui-ci selon l'une quelconque des revendications 1 à 3, pour une utilisation dans une méthode de traitement d'une maladie associée aux spasmes musculaires choisie dans le groupe constitué par les hernies des disques intervertébraux, les troubles vasculaires de la moelle épinière, la maladie de Little, la spondylose cervicale, l'infirmité motrice cérébrale, les séquelles de lésions, et la dégénérescence spinocérébelleuse, et
où le composé est choisi dans le groupe constitué par :
le 1-(2-chlorophényl)-(S)-1-hydroxypropyl-(S)-2-carbamate,
le 1-(2-chlorophényl)-(R)-1-hydroxypropyl-(R)-2-carbamate,
le racémate de 1-(2-chlorophényl)-(S)-1-hydroxypropyl-(S)-2-carbamate et de 1-(2-chlorophényl)-(R)-1-hydroxypropyl-(R)-2-carbamate,
le 1-(2-chlorophényl)-(R)-1-hydroxypropyl-(S)-2-carbamate,
le 1-(2-chlorophényl)-(S)-1-hydroxypropyl-(R)-2 -carbamate,
le 1-(2-chlorophényl)-(S)-1-hydroxy-3-méthylbutyl-(S)-2-carbamate,
le racémate de 1-(2-chlorophényl)-(S)-1-hydroxy-3-méthylbutyl-(S)-2-carbamate et de 1-(2-chlorophényl)-(R)-1-hydroxy-3-méthylbutyl-(R)-2-carbamate,
le 1-(2-chlorophényl)-(S)-1-hydroxypropyl-(S)-2-N-méthylcarbamate,
le 1-(2-chlorophényl)-(S)-1-hydroxypropyl-(S)-2-N-propylcarbamate,
le 1-(2-chlorophényl)-(S)-1-hydroxypropyl-(S)-2-N-isopropylcarbamate,
le 1-(2-chlorophényl)-(S)-1-hydroxypropyl-(S)-2-N-cyclopropylcarbamate,
le 1-(2-chlorophényl)-(S)-1-hydroxypropyl-(S)-2-N-cyclohexylcarbamate,
le 1-(2-chlorophényl)-(R)-1-hydroxypropyl-(R)-2-N-méthylcarbamate,
le 1-(2-chlorophényl)-(R)-1-hydroxypropyl-(R)-2-N-propylcarbamate,
le 1-(2-chlorophényl)-(R)-1-hydroxypropyl-(R)-2-N-isopropylcarbamate,
le 1-(2-chlorophényl)-(R)-1-hydroxypropyl-(R)-2-N-cyclopropylcarbamate,
le 1-(2-chlorophényl)-(R)-1-hydroxypropyl-(R)-2-N-cyclohexylcarbamate,
le racémate de 1-(2-chlorophényl)-(S)-1-hydroxypropyl-(S)-2-N-méthylcarbamate et de 1-(2-chlorophényl)-(R)-1-hydroxypropyl-(R)-2-N-méthylcarbamate,
le racémate de 1-(2-chlorophényl)-(S)-1-hydroxypropyl-(S)-2-N-propylcarbamate et de 1-(2-chlorophényl)-(R)-1-hydroxypropyl-(R)-2-N-propylcarbamate,
le racémate de 1-(2-chlorophényl)-(S)-1-hydroxypropyl-(S)-2-N-isopropylcarbamate et
de 1-(2-chlorophényl)-(R)-1-hydroxypropyl-(R)-2-N-isopropylcarbamate,
le racémate de 1-(2-chlorophényl)-(S)-1-hydroxypropyl-(S)-2-N-cyclopropylcarbamate et de 1-(2-chlorophényl)-(R)-1-hydroxypropyl-(R)-2-N-cyclopropylcarbamate,
le racémate de 1-(2-chlorophényl)-(S)-1-hydroxypropyl-(S)-2-N-cyclohexylcarbamate et de 1-(2-chlorophényl)-(R)-1-hydroxypropyl-(R)-2-N-cyclohexylcarbamate,
le 1-(2-fluorophényl)-(S)-1-hydroxypropyl-(S)-2-carbamate,
le 1-(2-fluorophényl)-(R)-1-hydroxypropyl-(R)-2-carbamate,
le 1-(2-iodophényl)-(S)-1-hydroxypropyl-(S)-2-carbamate,
le 1-(2-iodophényl)-(R)-1-hydroxypropyl-(R)-2-carbamate, et
le 1-(2-iodophényl)-(S)-1-hydroxybutyl-(S)-2-carbamate.

7. Composé ou un sel pharmaceutiquement acceptable de celui-ci selon la revendication 6, pour une utilisation dans une méthode de traitement selon la revendication 6, où le composé est le 1-(2-chlorophényl)-(S)-1-hydroxypropyl-(S)-2-carbamate, ou un sel pharmaceutiquement acceptable de celui-ci.

8. Composition pharmaceutique comprenant un composé ou un sel pharmaceutiquement acceptable de celui-ci selon l'une quelconque des revendications 1 à 3, et un ou plusieurs composants pharmaceutiquement acceptables.
